# EUROPEAN PATENT SPECIFICATION

(11) **EP 3 997 095 B1**
(45) Date of publication and mention of the grant of the patent: **12.02.2025**
(21) Application number: 20841410.2
(22) Date of filing: 10.07.2020
(51) Int. Cl.: C07D 493/04, A61K 31/366, A61P 9/00, A61P 25/00

(54) **CANNABINOID DERIVATIVES**
CANNABINOID-DERIVATE
DÉRIVÉS CANNABINOÏDES

(30) Priority: 12.07.2019 US 201962873686 P
(43) Date of publication of application: 18.05.2022
(73) Proprietor: Canopy Growth Corporation, Smiths Falls, Ontario K7A 0A8 (CA)
(72) Inventor: OMEARA, Jeffrey Alan, Halton Hills, Ontario L7G 4S6 (CA); TO, Quang Huy, Smiths Falls, Ontario K7A 0A8 (CA); GUNNING, Patrick Thomas, Mississauga, Ontario L5M 0T1 (CA)
(74) Representative: Marks & Clerk LLP
(86) International application number: PCT/CA2020/050966
(87) International publication number: WO 2021/007660

(56) References cited:
- WO-A1-2014/039042
- WO-A1-2014/134127
- WO-A1-2019/234728
- WO-A1-2020/041321
- WO-A2-00/56303
- US-A1- 2005 009 903
- US-A1- 2007 179 135
- BARRETT, T.N. ET AL.: "Cascade Polyketide and Polyene Cyclizations: Biomimetic Total Synthesis of Hongoquercin B", J.A.C.S., vol. 136, no. 49, 25 November 2014 (2014-11-25), pages 17013 - 17015, XP055784229, ISSN: 0002-7863

## Description

### BACKGROUND OF THE INVENTION

### Field of the Invention

This invention relates generally to cannabinoid derivatives, pharmaceutical compositions comprising them, and medical uses of the cannabinoid derivatives.

### Technical Background

Every individual has an endocannabinoid system comprised of chemical receptors in the brain, immune system, and central nervous system, for example including the cannabinoid 1 (CB1) receptor and cannabinoid 2 (CB2) receptor. The endocannabinoid system regulates many important physiological processes and several components of the endocannabinoid system, such as receptors, transporters, endocannabinoids and enzymes involved in the synthesis and degradation of endocannabinoids, are under active investigation as targets to treat a diverse array of indications.

CB1 and CB2 receptors are a class of cell membrane receptors belonging to the G protein-coupled receptor (GPCR) superfamily. The CB1 and CB2 receptors are distinguished from each other by their amino acid sequence, tissue distribution, signaling mechanisms, and ability to bind sub-type specific ligands. The CB1 receptor is mainly expressed in the central nervous system (CNS), lungs, liver, adipose tissue, and kidneys, and the CB2 receptor is mainly localized in immune cells (e.g. macrophages and T-cells), on cells that are involved in bone formation and bone loss, and in the gastrointestinal system. These receptors have been associated with many human diseases including obesity, diabetes, fibrosis, liver diseases, cardiovascular disease, cancer, pain, inflammation, MS spasticity, and glaucoma, among others.

Cannabinoids are compounds active on cannabinoid receptors in humans and have been implicated in many of the pharmacological benefits on the diseases noted above. Cannabinoids of plant origin, also known as phytocannabinoids, are abundant in *Cannabis.* Medical use of cannabis and associated phytocannabinoids is becoming widely accepted in many countries, including the United States, as an alternative form of medicine. Many states have legalized its use for qualified medical conditions such as chronic pain, epilepsy, sleep disorders, anxiety, cancer, glaucoma, nausea, amyotrophic lateral sclerosis (ALS), Alzheimer's disease, Crohn's disease, Post-traumatic Stress Disorder (PTSD), arthritis, fibromyalgia, and others.

In addition to the CB1 and CB2 receptors, other receptors have also been implicated in modulating the activity of cannabinoids in the human and/or animal body. For example, the serotonin receptors, such as 5HT1A and 5HT2A, are likewise GPCRs that have been identified as cannabinoid targets. The serotonin receptors modulate the release of many neurotransmitters, including glutamate, GABA, dopamine, epinephrine/norepinephrine and acetylcholine, as well as many hormones. Like the CB1 and CB2 receptors, the serotonin receptors influence various biological and neurological processes, such as anxiety, appetite, cognition, and mood, among others. Other receptors identified as being influenced by cannabinoid or cannabinoid-like compounds include GPR18, GPR55, GPR119, TRPV1, TPRV2, PPARs (e.g. PPARγ), and the µ-opioid receptors. Indeed, binding of these receptors may be responsible for off-target effects of cannabinoids.

One of the most common ways that cannabinoids are used for medicinal use in many countries is through smoking of cannabis. Although proven to be beneficial in certain indications, smoking medical cannabis has disadvantages. For example, the smoke from the plant matter comprises carcinogens and other toxins in addition to the desired cannabinoids. Heavy cannabis use through smoking has also been associated with accelerated pulmonary decline, lung damage, and emphysema. Another disadvantage of smoking medical cannabis is difficulty in maintaining control over the proper dosing of medicinal cannabis due to active ingredients fluctuations (e.g., the amounts of active ingredients may differ depending on the differences present in plant varietals as well as changing growing conditions which result in intravarietal variations). Finally, consumption through smoking has a relatively low bioavailability of target compounds compared to other delivery methods.

A less common way to utilize cannabis for medical use is to extract beneficial cannabinoids from cannabis. Many extraction processes have been developed for isolating and purifying natural cannabinoids, but there has been difficulty in isolating individual cannabinoids at high levels of purity, both for active ingredients for use in medicine and product manufacturing and/or as standards for use in research and development.

Therefore, there exists a need for novel ligands of cannabinoid receptors that have the potential for therapeutic benefit.

WO 2014/039042 A1 relates to novel cannabinergic compounds and uses thereof.

WO 2014/134127 A1 relates to cannabinergic nitrate esters and related analogues.

US 2005/009903 A1 relates to CB2-selective cannabinoid analogues.

### SUMMARY OF THE INVENTION

The invention is set out in the appended set of claims. In addition, any reference to methods of treatment in the subsequent paragraphs of this description is to be interpreted as references to the compounds, pharmaceutical compositions and medicaments of the present invention for use in a method of treatment of the human or animal body by therapy.

One aspect of the present invention relates to compounds of formula (I): 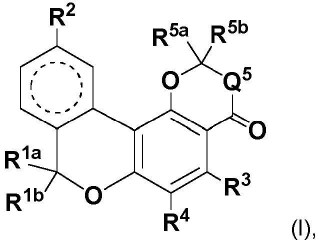 or an enantiomer, diastereomer, racemate, or tautomer thereof, or a pharmaceutically acceptable salt, solvate or hydrate of the compound, enantiomer, diastereomer, racemate, or tautomer, wherein the 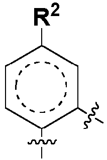 moiety is 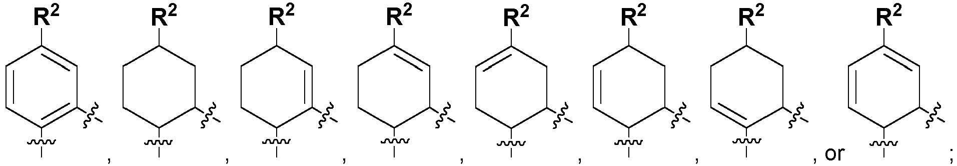
R^{1a} and R^{1b} are independently hydrogen, C₁-C₈ alkyl, C₂-C₃ alkenyl, C₂-C₈ alkynyl, hydroxy, halo, -O(C₁-C₄ alkyl), -C(O)(C₁-C₄ alkyl), -CO₂H, -CO₂(C₁-C₄ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{1c}R^{1d}, wherein
R^{1c} and R^{1d} are independently hydrogen, C₁-C₄ alkyl, or -C(O)(C₁-C₄ alkyl),
or R^{1a} and R^{1b} together with a carbon atom to which they are attached form a cycloalkyl or heterocycloalkyl ring;
R² is hydrogen, C₁-C₈ alkyl, hydroxy, -CO₂H, -CO₂(C₁-C₈ alkyl), or oxo when attached to a ring of appropriate saturation;
R³ is hydrogen, C₁-C₁₂ alkyl, C₂-C₁₂ alkenyl, C₂-C₁₂ alkynyl, -(OCH₂CH₂)₀₋₆O(C₁-C₈ alkyl), -(C₀-C₄ alkyl)-NR^{3a}R^{3b}, -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl, wherein R^{3a} and R^{3b} are each independently hydrogen or C₁-C₆ alkyl;
R⁴ is hydrogen, -CO₂H or -CO₂(C₁-C₆ alkyl); and
**R^{5a}** is:
   hydrogen, C₁-C₈ alkyl, C₂-C₃ alkenyl, C₂-C₈ alkynyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₈ alkyl), -CO₂H, -CO₂(C₁-C₈ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, and
R^{5b} is:
   hydrogen, C₂-C₃ alkenyl, C₂-C₈ alkynyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₈ alkyl), -CO₂H, -CO₂(C₁-C₈ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, wherein
R^{5c} and R^{5d} are independently hydrogen, C₁-C₆ alkyl, C₂-C₆ alkenyl, C₂-C₆ alkynyl or -(C₀-C₄ alkyl)C(O)₁₋₂(C₁-C₆ alkyl);
or R^{5a} and R^{5b} together with a carbon atom to which they are attached form a cycloalkyl or heterocycloalkyl ring; and
Q⁵ is O, S, or NR^{5e}, wherein
R^{5e} is hydrogen, C₁-C₈ alkyl, C₂-C₈ alkenyl, C₂-C₈ alkynyl, -(CH₂CH₂O)₁₋₈(C₁-C₄ alkyl), -C(O)R^{5f}, -CO₂R^{5f}, -(C₁-C₄ alkyl)-aryl, -(C₁-C₄ alkyl)-heteroaryl, -(C₁-C₄ alkyl)-cycloalkyl or -(C₁-C₄ alkyl)-heterocycloalkyl, wherein each R^{5f} is hydrogen, C₁-C₈ alkyl, C₂-C₈ alkenyl, C₂-C₈ alkynyl, or -(CH₂CH₂O)₁₋₆(C₀-C₄ alkyl);
wherein
   each alkyl, alkenyl and alkynyl is unsubstituted, fluorinated, substituted with one or two hydroxyl or C₁-C₆ alkoxy groups, or substituted with one or two oxo groups;
   each cycloalkyl has 3-10 ring carbons and is unsaturated or partially unsaturated, and optionally includes one or two fused cycloalkyl rings, each fused ring having 3-8 ring members, and is substituted with 0-6 R⁷;
   each heterocycloalkyl has 3-10 ring members and 1-3 heteroatoms where each is independently nitrogen, oxygen or sulfur and is unsaturated or partially unsaturated, and optionally includes one or two fused cycloalkyl rings, each having 3-8 ring members, and is substituted with 0-6 R⁷;
   each aryl is a phenyl or a naphthyl, and optionally includes one or two fused cycloalkyl or heterocycloalkyl rings, each fused cycloalkyl or heterocycloalkyl ring having 4-8 ring members, and is substituted with 0-5 R⁸;
   each heteroaryl is a 5-6 membered monocyclic heteroaryl ring having 1-4 heteroatoms, where each is independently nitrogen, oxygen or sulfur or a 8-10 membered bicyclic heteroaryl having 1-5 heteroatoms where each is independently nitrogen, oxygen or sulfur, and optionally includes one or two fused cycloalkyl or heterocycloalkyl rings, each fused cycloalkyl or heterocycloalkyl ring having 4-8 ring members, and is substituted with 0-5 R⁸,
   in which
      each R⁷ is independently oxo, C₁-C₄ alkyl, -Cl, -F, -Br, -CN, -SF₅, -N₃, nitro, -SR^{A}, -S(O)₁₋₂R^{A,} -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} or -NR^{B}S(O)₁₋₂NR^{B}R^{A}; and
      each R⁸ is independently optionally-substituted C₁-C₄ alkyl, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} or -NR^{B}S(O)₁₋₂NR^{B}R^{A};
         wherein each R^{A} is independently H or C₁-C₃ alkyl, and each R^{B} is independently H, C₁-C₃ alkyl, C₁-C₃ fluoroalkyl, C₁-C₃ hydroxyalkyl, -S(O)₁-₂(C₁-C₃ alkyl), -C(O)(C₁-C₃ alkyl) or -CO₂(C₁-C₃ alkyl), or R^{A} and R^{B} together with the nitrogen atom to which they are attached come together to form an unsubstituted heterocycloalkyl ring comprised of 3-6 ring members.

In another aspect, the present invention relates to pharmaceutical compositions comprising a compound (e.g., a compound of formula (I)) as described herein, or an enantiomer, diastereomer, racemate, or tautomer thereof, or a pharmaceutically acceptable salt, solvate or hydrate of the compound, enantiomer, diastereomer, racemate, or tautomer. In an embodiment, the pharmaceutical compositions comprise a pharmaceutically acceptable excipient, diluent, or carrier.

In another aspect, the present invention relates to a compound (e.g., a compound of formula (I)) or a composition as described herein for use in treating or preventing a disease associated with a cannabinoid receptor (e.g. CB1, CB2, 5HT1A, 5HT2A, GPR18, GPR55, GPR119, TRPV1, TPRV2, PPARγ, or a µ-opioid receptor).

In certain embodiments, the diseases that may be treated or prevented with the compounds or compositions as described herein include, but are not limited to attention-deficit/hyperactivity disorder (ADHD)/attention-deficit disorder (ADD), alcohol use disorder, allergic asthma, amyotrophic lateral sclerosis (ALS), Alzheimer's, anorexia (e.g. human immunodeficiency virus (HIV)-related cachexia), anxiety disorders (e.g., social anxiety disorder, specific phobia, test anxiety, generalized anxiety disorder), arthritis, atherosclerosis, autism, bipolar disorder, burns, cancer, cancer pain, Charcot-Marie-Tooth disease, chronic inflammatory demyelinating polyneuropathies, chronic allograft nephropathy, cocaine use disorder, complex regional pain syndrome, chronic allograft nephropathy, depression, fibromyalgia, fragile X syndrome/fragile X-associated tremor and ataxia syndrome (FXTAS), frontotemporal dementias (behavioural variant), gingivitis pyrexia, glaucoma, glioblastoma, glomerulonephropathy, Huntington's disease, hypertrophic scars, inflammatory bowel disease (IBD)/ irritable bowel syndrome (IBS), inflammation, Inflammatory myopathies, ischemia, kidney fibrosis, keloids, leukodystrophies, liver fibrosis, liver cirrhosis, lung fibrosis, migraine, multiple sclerosis, myocardial infarction, nausea (e.g. chemotherapy-induced nausea and vomiting (CINV), motion sickness), neuropathic pain (e.g., postherpetic neuralgia, painful diabetic neuropathy), nightmare disorder, non-alcoholic fatty liver disease, obesity, obsessive-compulsive disorder, opioid sparing, opioid use disorder, osteoarthritis, osteoporosis, pain (e.g., acute or chronic pain), Parkinson's, post-concussion syndrome/traumatic brain injury, psychosis/schizophrenia, post-traumatic stress disorder (PTSD), regulation of bone mass, rapid eye movement (REM) sleep behaviour disorder, reperfusion injury, Rett syndrome, rheumatoid arthritis, skin conditions (e.g. acne, psoriatic arthritis), sleep disorders (e.g., insomnia, restless legs syndrome (RLS)), spinocerebellar ataxias, systemic fibrosis, systemic sclerosis, thermal injury, tobacco use disorder/nicotine dependence, Tourette's, tumors, and trigeminal neuralgia.

In another aspect, the present invention relates to use of a compound as described herein, or an enantiomer, diastereomer, racemate, or tautomer thereof, or a pharmaceutically acceptable salt, solvate or hydrate of the compound, enantiomer, diastereomer, racemate, or tautomer, for the treatment or prevention of a disease associated with cannabinoid receptor (e.g. CB1, CB2, 5HT1A, 5HT2A, GPR18, GPR55, GPR119, TRPV1, TPRV2, PPARγ, or a µ-opioid receptor).

In another aspect, the present invention relates to use of a pharmaceutical composition as described herein for the treatment or prevention of a disease associated with cannabinoid receptor (e.g. CB1, CB2, 5HT1A, 5HT2A, GPR18, GPR55, GPR119, TRPV1, TPRV2, PPARγ, or a µ-opioid receptor).

Also disclosed herein, but not forming part of the claimed invention, is the use of a compound as described herein, or an enantiomer, diastereomer, racemate, or tautomer, or a pharmaceutically acceptable salt, solvate or hydrate of the compound, enantiomer, diastereomer, racemate, or tautomer, for selectively modulating the activity of a CB1 or CB2 receptor.

Also disclosed herein, but not forming part of the claimed invention, is the use of a pharmaceutical composition as described herein, for selectively modulating the activity of a CB1 or CB2 receptor.

Other aspects and embodiments of the invention are evident in view of the detailed description provided herein.

### DETAILED DESCRIPTION

The present invention provides compounds capable of acting as ligands to one or more cannabinoid receptors and/or prodrugs thereof. As used herein, "cannabinoid receptor" refers to a broad class of receptors that bind, interact with and/or are influenced functionally by cannabinoids or cannabinoid-like compounds. Without limitation, cannabinoid receptors may include CB1, CB2, 5HT1A, 5HT2A, GPR18, GPR55, GPR119, TRPV1, TPRV2, PPARs (e.g. PPARγ) or µ-opioid receptors. In an embodiment, the present invention further relates to compounds and pharmaceutical compositions, and to compounds and pharmaceutical compositions for use in treating or preventing one or more diseases associated with a cannabinoid receptor. Also disclosed herein, but not part of the claimed invention, are methods and uses of the compounds and pharmaceutical compositions for treating or preventing one or more diseases associated with a cannabinoid receptor.

The compounds of the present invention can be defined generically as with respect to formula (I) as appropriate, 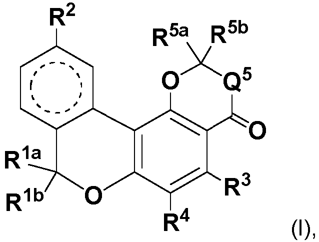 or in various subgenera compounds in which within the structural formula (I), the 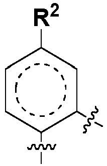 moiety, R^{1a} and R^{1b}, R², R³, R⁴, R^{5a} and R^{5b}, and Q⁵ are optionally independently selected from the groups (Ia) to (If), (1a) to (1e), (2a) to (2j), (3a) to (3k), (4a) to (4p), (5a) to (5e), (6a) to (6x), and (7a) to (7e), defined herein below (e.g., wherein the compound is of a structural formula as defined in any combination of the embodiments below).

In certain embodiments of the compounds as otherwise described herein, the 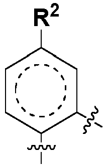 moiety is selected from the following groups (1a) - (1e):

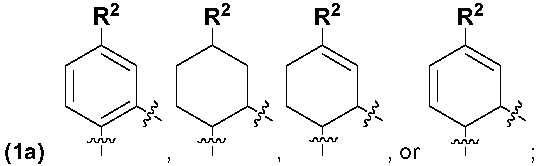

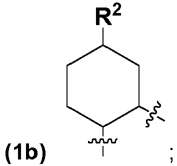

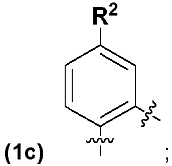

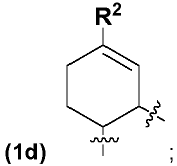

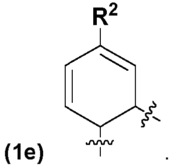

In certain embodiments of the compounds as otherwise described herein, R^{1a} and R^{1b} are independently selected from one of the following groups (2a) - (2j):
**(2a)** hydrogen, C₁-C₄ alkyl, C₂-C₄ alkenyl, hydroxy, halo, -O(C₁-C₄ alkyl), -C(O)(C₁-C₄ alkyl), -CO₂(C₁-C₄ alkyl), -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl;
**(2b)** hydrogen, C₁-C₄ alkyl, hydroxy, or -CO₂(C₁-C₄ alkyl);
**(2c)** hydrogen, hydroxy, or C₁-C₄ alkyl;
**(2d)** C₁-C₄ alkyl;
**(2e)** R^{1a} and R^{1b} are both methyl
**(2f)** R^{1a} is methyl and R^{1b} is hydrogen;
**(2g)** R^{1a} and R^{1b} are both hydrogen;
**(2h)** R^{1a} is hydrogen and R^{1b} is -NR^{1c}R^{1d};
**(2i)** R^{1a} and R^{1b} together with a carbon atom form a cycloalkyl ring (e.g. a saturated cycloalkyl ring) comprising 3 to 6 carbo members;
**(2j)** R^{1a} and R^{1b} together with a carbon atom form a cyclopentyl.

In certain embodiments of the compounds as otherwise described herein, R² is selected from one of the following groups (3a) - (3k):
**(3a)** hydrogen, C₁-C₄ alkyl, hydroxy, -CO₂H, -CO₂(C₁-C₄ alkyl), or oxo;
**(3b)** hydrogen, C₁-C₆ alkyl, hydroxy, -CO₂H, or oxo;
**(3c)** hydrogen or C₁-C₆ alkyl;
**(3d)** hydrogen;
**(3e)** C₁-C₄ alkyl (e.g., methyl);
**(3f)** methyl, ethyl, or propyl (e.g., isopropyl or n-propyl);
**(3g)** hydroxy, oxo or -CO₂H;
**(3h)** hydroxy;
**(3i)** oxo;
**(3j)** -CH₂OH;
**(3k)** -CO₂H.

In certain embodiments of the compounds as otherwise described herein, R³ is selected from one of the following groups (4a) - (4p):
**(4a)** hydrogen, C₁-C₁₂ alkyl, C₂-C₁₂ alkenyl, C₂-C₁₂ alkynyl, -(OCH₂CH₂)₀₋₆OCH₃, -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl;
**(4b)** hydrogen, C₁-C₁₂ alkyl, -(C₀-C₄ alkyl)-heteroaryl, -(C₀-C₄ alkyl)-cycloalkyl or -(C₀-C₄ alkyl)-heterocycloalkyl;
**(4c)** hydrogen, C₁-C₈ alkyl, -(C₁-C₄alkyl)-heteroaryl or -(C₁-C₄ alkyl)-heterocycloalkyl;
**(4d)** hydrogen, C₄-C₉ alkyl, -(C₁-C₂ alkyl)-heteroaryl or -(C₁-C₂ alkyl)-heterocycloalkyl;
**(4e)** hydrogen or C₁-C₁₀ alkyl;
**(4f)** hydrogen;
**(4g)** C₂-C₉ alkyl, e.g., unsubstituted n-C₂-C₉ alkyl;
**(4h)** C₄-C₆ alkyl, e.g., unsubstituted n-C₄-C₆ alkyl;
**(4i)** n-pentyl;
**(4j)** 1,1-dimethylheptyl;
**(4k)** n-propyl;
**(4l)** -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl;
**(4m)** -(C₀-C₄ alkyl)-heterocycloalkyl, wherein the heterocycloalkyl has 3-8 ring members and 1-3 heteroatoms that are nitrogen, oxygen or sulfur and is substituted with 0-4 R⁷;
**(4n)** -(C₀-C₄ alkyl)-heterocycloalkyl, wherein the heterocycloalkyl has 3-8 ring members and 1-3 heteroatoms that are nitrogen, oxygen or sulfur and is substituted with 0-4 R⁷, wherein the heterocycloalkyl is piperidinyl, pyrrolidinyl, azetidinyl, or aziridinyl;
**(4o)** -(C₀-C₄ alkyl)-heterocycloalkyl, wherein the heterocycloalkyl has 3-8 ring members and 1-3 heteroatoms that are nitrogen, oxygen or sulfur and is substituted with 0-4 R⁷, wherein the heterocycloalkyl is azetidinyl and is substituted with 1-2 R⁷ which are independently oxo, C₁-C₄ alkyl, -Cl, -F, -Br, -C(O)R^{A}, or -CO₂R^{A}, wherein R^{A} is H or C₁-C₃ alkyl;
**(4p)** -(C₀-C₄ alkyl)-heterocycloalkyl, wherein the heterocycloalkyl has 3-8 ring members and 1-3 heteroatoms that are nitrogen, oxygen or sulfur and is substituted with 0-4 R⁷, wherein R⁷ is -C(O)CH₃.

In certain embodiments of the compounds as otherwise described herein, R⁴ is selected from one of the following groups (5a) - (5e):
**(5a)** hydrogen, -CO₂H or -CO₂(C₁-C₆ alkyl);
**(5b)** hydrogen, -CO₂H, -CO₂CH₃ or -CO₂CH₂CH₃;
**(5c)** hydrogen;
**(5d)** -CO₂H;
**(5e)** -CO₂(C₁-C₆ alkyl).

In certain embodiments of the compounds as otherwise described herein, the compound has one of the following structural formulae (Ia) - (If):

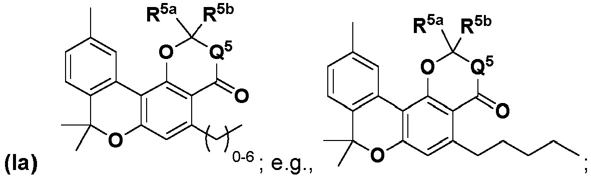

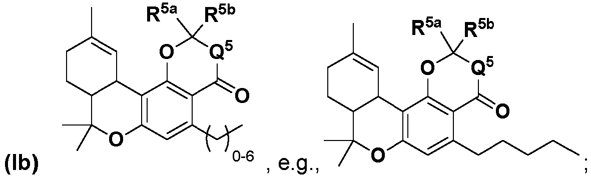

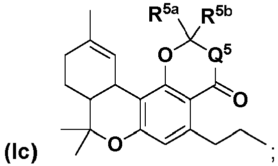

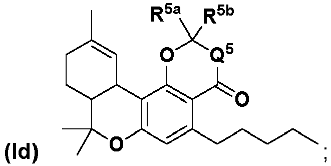

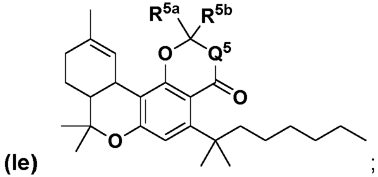

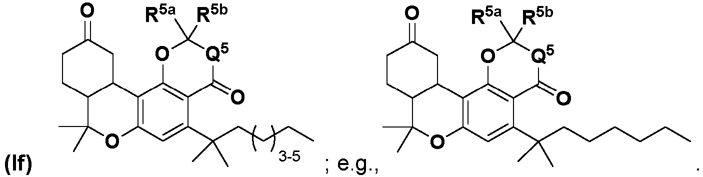

In compounds described herein, R^{5a} and R^{5b} are selected from one of the following groups (6a) - (6x). In compounds of the invention, R^{5b} is not C₁-C₈ alkyl.
**(6a)** hydrogen, C₁-C₆ alkyl, C₂-C₆ alkenyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₆ alkyl), -CO₂H, -CO₂(C₁-C₆ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, or R^{5a} and R^{5b} together with a carbon atom form a cycloalkyl or heterocycloalkyl ring;
**(6b)** hydrogen, C₁-C₆ alkyl, C₂-C₆ alkenyl, -(C₀-C₄ alkyl)O(C₁-C₆ alkyl), -(C₀-C₄ alkyl)C(O)(C₁-C₆ alkyl), -CO₂H, -CO₂(C₁-C₆ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or R^{5a} and R^{5b} together with a carbon atom form a cycloalkyl or heterocycloalkyl ring;
**(6c)** C₁-C₆ alkyl, C₂-C₆ alkenyl, -(C₀-C₄ alkyl)O(C₁-C₆ alkyl), -(C₀-C₄ alkyl)C(O)(C₁-C₆ alkyl), -CO₂H, -CO₂(C₁-C₆ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or R^{5a} and R^{5b} together with a carbon atom form a cycloalkyl or heterocycloalkyl ring;
**(6d)** hydrogen, C₁-C₆ alkyl, C₂-C₆ alkenyl, -(C₀-C₄ alkyl)O(C₁-C₆ alkyl), -(C₀-C₄ alkyl)C(O)(C₁-C₆ alkyl), -CO₂H, or -CO₂(C₁-C₆ alkyl);
**(6e)** C₁-C₆ alkyl, C₂-C₆ alkenyl, -(C₀-C₄ alkyl)O(C₁-C₆ alkyl), -(C₀-C₄ alkyl)C(O)(C₁-C₆ alkyl), -CO₂H, or -CO₂(C₁-C₆ alkyl);
**(6f)** hydrogen, C₁-C₆ alkyl or C₁-C₆ alkenyl;
**(6g)** hydrogen or C₁-C₄ alkyl;
**(6h)** C₁-C₄ alkyl (e.g., methyl);
**(6i)** hydrogen or methyl;
**(6j)** hydrogen, C₁-C₄ alkyl, -CH₂O(C₁-C₆ alkyl), -CH₂C(O)(C₁-C₆ alkyl), -CO₂H, or -CO₂(C₁-C₆ alkyl);
(6k) R^{5a} is -NHR^{5c}R^{5d}, wherein R^{5c} and R^{5d} are independently hydrogen, C₁-C₆ alkyl, C₂-C₆ alkenyl, or -(C₀-C₄ alkyl)C(O)(C₁-C₆ alkyl), and R^{5b} is hydrogen;
**(61)** R^{5a} is -(C₀-C₄ alkyl)-aryl, -(C₀-C₄ alkyl)-heteroaryl, -(C₀-C₄ alkyl)-cycloalkyl, or -(C₀-C₄ alkyl)-heterocycloalkyl;
**(6m)** R^{5a} is an unsubstituted or substituted phenyl;
**(6n)** R^{5a} is an unsubstituted phenyl;
**(6o)** R^{5a} is a substituted phenyl, e.g. 4-fluorophenyl or 4-trifluoromethylphenyl;
**(6p)** as defined in (6k)-(6o), wherein R^{5b} is hydrogen, C₁-C₄ alkyl, -CH₂OCH₃, -CH₂C(O)CH₃, -CO₂H, or -CO₂(C₁-C₂ alkyl);
**(6q)** as defined in (6k)-(6o), wherein R^{5b} is hydrogen or methyl;
**(6r)** as defined in (6k)-(6o), wherein R^{5b} is hydrogen or methyl;
**(6s)** as defined in (6k)-(6o), wherein R^{5b} is hydrogen;
**(6t)** R^{5a} is methyl, and R^{5b} is methyl, -CH₂O(C₁-C₆ alkyl), -CH₂C(O)(C₁-C₆ alkyl), -CO₂H, or -CO₂(C₁-C₆ alkyl);
**(6u)** R^{5a} and R^{5b} together with a carbon atom form a C₃-C₆ cycloalkyl (e.g. a cyclopentyl) or heterocycloalkyl ring comprised of 3-6 carbon members;
**(6v)** R^{5a} is C₁-C₄ alkyl (e.g. methyl or chloropropyl), -CH₂OCH₃, -CH₂C(O)CH₃, -CO₂H, or -CO₂(C₁-C₂ alkyl) and R^{5b} is hydrogen or methyl;
**(6w)** R^{5a} is -CH₂OCH₃, -CO₂H, or -CO₂CH₂CH₃ and R^{5b} is methyl;
**(6x)** R^{5a} -CH_{2C}(O)CH₃ and R^{5b} is hydrogen or methyl.

In certain embodiments of the compounds as otherwise described herein, Q⁵ is selected from one of the following groups (7a) - (7e):
**(7a)** oxygen or sulfur;
**(7b)** oxygen
**(7c)** sulfur
**(7d)** -NR^{5e};
**(7e)** -NR^{5e}, wherein R^{5e} is hydrogen, C₁-C₈ alkyl, -C(O)R^{5f}, or -CO₂R^{5f}, wherein each R^{5f} is hydrogen or C₁-C₆ alkyl.

Various compounds of formulae 1-272 disclosed herein include compounds of formula (I), each as defined in each of the following rows (or enantiomers, diastereomers, racemates, or tautomers thereof, or pharmaceutically acceptable salts, solvates or hydrates of the compounds, enantiomers, diastereomers, racemates, or tautomers), wherein each entry is a group number as defined above. As defined above, compounds in which R^{5b} is C₁-C₈ alkyl are not compounds according to the present invention.

| **Formula** | 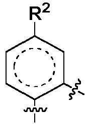 | **R^{1a} and R^{1b}** | **R²** | **R³** | **R⁴** | **R^{5a} and R^{5b}** | **Q⁵** |
|---|---|---|---|---|---|---|---|
| 1 | (1a)-(1e) | (2a) | (3a) | (4a) | (5c) | (6b) | (7b) |
| 2 | (1a)-(1e) | (2a) | (3a) | (4a) | (5c) | (6c) | (7b) |
| 3 | (1a)-(1e) | (2a) | (3a) | (4a) | (5c) | (6m) | (7b) |
| 4 | (1a)-(1e) | (2a) | (3a) | (4a) | (5c) | (6q) | (7b) |
| 5 | (1a)-(1e) | (2a) | (3a) | (4a) | (5c) | (6r) | (7b) |
| 6 | (1a)-(1e) | (2a) | (3a) | (4a) | (5c) | (6t) | (7b) |
| 7 | (1a)-(1e) | (2a) | (3a) | (4d) | (5c) | (6b) | (7b) |
| 8 | (1a)-(1e) | (2a) | (3a) | (4d) | (5c) | (6c) | (7b) |
| 9 | (1a)-(1e) | (2a) | (3a) | (4d) | (5c) | (6m) | (7b) |
| 10 | (1a)-(1e) | (2a) | (3a) | (4d) | (5c) | (6q) | (7b) |
| 11 | (1a)-(1e) | (2a) | (3a) | (4d) | (5c) | (6r) | (7b) |
| 12 | (1a)-(1e) | (2a) | (3a) | (4d) | (5c) | (6t) | (7b) |
| 13 | (1a)-(1e) | (2a) | (3a) | (4g),(4i),(4j),(4k) | (5c) | (6b) | (7b) |
| 14 | (1a)-(1e) | (2a) | (3a) | (4g),(4i),(4j),(4k) | (5c) | (6c) | (7b) |
| 15 | (1a)-(1e) | (2a) | (3a) | (4g),(4i),(4j),(4k) | (5c) | (6m) | (7b) |
| 16 | (1a)-(1e) | (2a) | (3a) | (4g),(4i),(4j),(4k) | (5c) | (6q) | (7b) |
| 17 | (1a)-(1e) | (2a) | (3a) | (4g),(4i),(4j),(4k) | (5c) | (6r) | (7b) |
| 18 | (1a)-(1e) | (2a) | (3a) | (4g),(4i),(4j),(4k) | (5c) | (6t) | (7b) |
| 19 | (1a)-(1e) | (2a) | (3e) | (4a) | (5c) | (6b) | (7b) |
| 20 | (1a)-(1e) | (2a) | (3e) | (4a) | (5c) | (6c) | (7b) |
| 21 | (1a)-(1e) | (2a) | (3e) | (4a) | (5c) | (6m) | (7b) |
| 22 | (1a)-(1e) | (2a) | (3e) | (4a) | (5c) | (6q) | (7b) |
| 23 | (1a)-(1e) | (2a) | (3e) | (4a) | (5c) | (6r) | (7b) |
| 24 | (1a)-(1e) | (2a) | (3e) | (4a) | (5c) | (6t) | (7b) |
| 25 | (1a)-(1e) | (2a) | (3e) | (4d) | (5c) | (6b) | (7b) |
| 26 | (1a)-(1e) | (2a) | (3e) | (4d) | (5c) | (6c) | (7b) |
| 27 | (1a)-(1e) | (2a) | (3e) | (4d) | (5c) | (6m) | (7b) |
| 28 | (1a)-(1e) | (2a) | (3e) | (4d) | (5c) | (6q) | (7b) |
| 29 | (1a)-(1e) | (2a) | (3e) | (4d) | (5c) | (6r) | (7b) |
| 30 | (1a)-(1e) | (2a) | (3e) | (4d) | (5c) | (6t) | (7b) |
| 31 | (1a)-(1e) | (2a) | (3e) | (4g),(4i),(4j),(4k) | (5c) | (6b) | (7b) |
| 32 | (1a)-(1e) | (2a) | (3e) | (4g),(4i),(4j),(4k) | (5c) | (6c) | (7b) |
| 33 | (1a)-(1e) | (2a) | (3e) | (4g),(4i),(4j),(4k) | (5c) | (6m) | (7b) |
| 34 | (1a)-(1e) | (2a) | (3e) | (4g),(4i),(4j),(4k) | (5c) | (6q) | (7b) |
| 35 | (1a)-(1e) | (2a) | (3e) | (4g),(4i),(4j),(4k) | (5c) | (6r) | (7b) |
| 36 | (1a)-(1e) | (2a) | (3e) | (4g),(4i),(4j),(4k) | (5c) | (6t) | (7b) |
| 37 | (1a)-(1e) | (2e) | (3a) | (4a) | (5c) | (6b) | (7b) |
| 38 | (1a)-(1e) | (2e) | (3a) | (4a) | (5c) | (6c) | (7b) |
| 39 | (1a)-(1e) | (2e) | (3a) | (4a) | (5c) | (6m) | (7b) |
| 40 | (1a)-(1e) | (2e) | (3a) | (4a) | (5c) | (6q) | (7b) |
| 41 | (1a)-(1e) | (2e) | (3a) | (4a) | (5c) | (6r) | (7b) |
| 42 | (1a)-(1e) | (2e) | (3a) | (4a) | (5c) | (6t) | (7b) |
| 43 | (1a)-(1e) | (2e) | (3a) | (4d) | (5c) | (6b) | (7b) |
| 44 | (1a)-(1e) | (2e) | (3a) | (4d) | (5c) | (6c) | (7b) |
| 45 | (1a)-(1e) | (2e) | (3a) | (4d) | (5c) | (6m) | (7b) |
| 46 | (1a)-(1e) | (2e) | (3a) | (4d) | (5c) | (6q) | (7b) |
| 47 | (1a)-(1e) | (2e) | (3a) | (4d) | (5c) | (6r) | (7b) |
| 48 | (1a)-(1e) | (2e) | (3a) | (4d) | (5c) | (6t) | (7b) |
| 49 | (1a)-(1e) | (2e) | (3a) | (4g),(4i),(4j),(4k) | (5c) | (6b) | (7b) |
| 50 | (1a)-(1e) | (2e) | (3a) | (4g), (4i), (4j), (4k) | (5c) | (6c) | (7b) |
| 51 | (1a)-(1e) | (2e) | (3a) | (4g),(4i),(4j),(4k) | (5c) | (6m) | (7b) |
| 52 | (1a)-(1e) | (2e) | (3a) | (4g),(4i),(4j),(4k) | (5c) | (6q) | (7b) |
| 53 | (1a)-(1e) | (2e) | (3a) | (4g),(4i),(4j),(4k) | (5c) | (6r) | (7b) |
| 54 | (1a)-(1e) | (2e) | (3a) | (4g),(4i),(4j),(4k) | (5c) | (6t) | (7b) |
| 55 | (1a)-(1e) | (2e) | (3e) | (4a) | (5c) | (6b) | (7b) |
| 56 | (1a)-(1e) | (2e) | (3e) | (4a) | (5c) | (6c) | (7b) |
| 57 | (1a)-(1e) | (2e) | (3e) | (4a) | (5c) | (6m) | (7b) |
| 58 | (1a)-(1e) | (2e) | (3e) | (4a) | (5c) | (6q) | (7b) |
| 59 | (1a)-(1e) | (2e) | (3e) | (4a) | (5c) | (6r) | (7b) |
| 60 | (1a)-(1e) | (2e) | (3e) | (4a) | (5c) | (6t) | (7b) |
| 61 | (1a)-(1e) | (2e) | (3e) | (4d) | (5c) | (6b) | (7b) |
| 62 | (1a)-(1e) | (2e) | (3e) | (4d) | (5c) | (6c) | (7b) |
| 63 | (1a)-(1e) | (2e) | (3e) | (4d) | (5c) | (6m) | (7b) |
| 64 | (1a)-(1e) | (2e) | (3e) | (4d) | (5c) | (6q) | (7b) |
| 65 | (1a)-(1e) | (2e) | (3e) | (4d) | (5c) | (6r) | (7b) |
| 66 | (1a)-(1e) | (2e) | (3e) | (4d) | (5c) | (6t) | (7b) |
| 67 | (1a)-(1e) | (2e) | (3e) | (4g),(4i),(4j),(4k) | (5c) | (6b) | (7b) |
| 68 | (1a)-(1e) | (2e) | (3e) | (4g),(4i),(4j),(4k) | (5c) | (6c) | (7b) |
| 69 | (1a)-(1e) | (2e) | (3e) | (4g),(4i),(4j),(4k) | (5c) | (6m) | (7b) |
| 70 | (1a)-(1e) | (2e) | (3e) | (4g),(4i),(4j),(4k) | (5c) | (6q) | (7b) |
| 71 | (1a)-(1e) | (2e) | (3e) | (4g),(4i),(4j),(4k) | (5c) | (6r) | (7b) |
| 72 | (1a)-(1e) | (2e) | (3e) | (4g),(4i),(4j),(4k) | (5c) | (6t) | (7b) |
| 73 | (1a)-(1e) | (2i) | (3a) | (4a) | (5c) | (6b) | (7b) |
| 74 | (1a)-(1e) | (2i) | (3a) | (4a) | (5c) | (6c) | (7b) |
| 75 | (1a)-(1e) | (2i) | (3a) | (4a) | (5c) | (6m) | (7b) |
| 76 | (1a)-(1e) | (2i) | (3a) | (4a) | (5c) | (6q) | (7b) |
| 77 | (1a)-(1e) | (2i) | (3a) | (4a) | (5c) | (6r) | (7b) |
| 78 | (1a)-(1e) | (2i) | (3a) | (4a) | (5c) | (6t) | (7b) |
| 79 | (1a)-(1e) | (2i) | (3a) | (4d) | (5c) | (6b) | (7b) |
| 80 | (1a)-(1e) | (2i) | (3a) | (4d) | (5c) | (6c) | (7b) |
| 81 | (1a)-(1e) | (2i) | (3a) | (4d) | (5c) | (6m) | (7b) |
| 82 | (1a)-(1e) | (2i) | (3a) | (4d) | (5c) | (6q) | (7b) |
| 83 | (1a)-(1e) | (2i) | (3a) | (4d) | (5c) | (6r) | (7b) |
| 84 | (1a)-(1e) | (2i) | (3a) | (4d) | (5c) | (6t) | (7b) |
| 85 | (1a)-(1e) | (2i) | (3a) | (4g),(4i),(4j),(4k) | (5c) | (6b) | (7b) |
| 86 | (1a)-(1e) | (2i) | (3a) | (4g),(4i),(4j),(4k) | (5c) | (6c) | (7b) |
| 87 | (1a)-(1e) | (2i) | (3a) | (4g),(4i),(4j),(4k) | (5c) | (6m) | (7b) |
| 88 | (1a)-(1e) | (2i) | (3a) | (4g),(4i),(4j),(4k) | (5c) | (6q) | (7b) |
| 89 | (1a)-(1e) | (2i) | (3a) | (4g),(4i),(4j),(4k) | (5c) | (6r) | (7b) |
| 90 | (1a)-(1e) | (2i) | (3a) | (4g),(4i),(4j),(4k) | (5c) | (6t) | (7b) |
| 91 | (1a)-(1e) | (2i) | (3e) | (4a) | (5c) | (6b) | (7b) |
| 92 | (1a)-(1e) | (2i) | (3e) | (4a) | (5c) | (6c) | (7b) |
| 93 | (1a)-(1e) | (2i) | (3e) | (4a) | (5c) | (6m) | (7b) |
| 94 | (1a)-(1e) | (2i) | (3e) | (4a) | (5c) | (6q) | (7b) |
| 95 | (1a)-(1e) | (2i) | (3e) | (4a) | (5c) | (6r) | (7b) |
| 96 | (1a)-(1e) | (2i) | (3e) | (4a) | (5c) | (6t) | (7b) |
| 97 | (1a)-(1e) | (2i) | (3e) | (4d) | (5c) | (6b) | (7b) |
| 98 | (1a)-(1e) | (2i) | (3e) | (4d) | (5c) | (6c) | (7b) |
| 99 | (1a)-(1e) | (2i) | (3e) | (4d) | (5c) | (6m) | (7b) |
| 100 | (1a)-(1e) | (2i) | (3e) | (4d) | (5c) | (6q) | (7b) |
| 101 | (1a)-(1e) | (2i) | (3e) | (4d) | (5c) | (6r) | (7b) |
| 102 | (1a)-(1e) | (2i) | (3e) | (4d) | (5c) | (6t) | (7b) |
| 103 | (1a)-(1e) | (2i) | (3e) | (4g),(4i),(4j),(4k) | (5c) | (6b) | (7b) |
| 104 | (1a)-(1e) | (2i) | (3e) | (4g),(4i),(4j),(4k) | (5c) | (6c) | (7b) |
| 105 | (1a)-(1e) | (2i) | (3e) | (4g),(4i),(4j),(4k) | (5c) | (6m) | (7b) |
| 106 | (1a)-(1e) | (2i) | (3e) | (4g),(4i),(4j),(4k) | (5c) | (6q) | (7b) |
| 107 | (1a)-(1e) | (2i) | (3e) | (4g),(4i),(4j),(4k) | (5c) | (6r) | (7b) |
| 108 | (1a)-(1e) | (2i) | (3e) | (4g),(4i),(4j),(4k) | (5c) | (6t) | (7b) |
| 109 | (1d) | (2a) | (3a) | (4a) | (5c) | (6b) | (7b) |
| 110 | (1d) | (2a) | (3a) | (4a) | (5c) | (6c) | (7b) |
| 111 | (1d) | (2a) | (3a) | (4a) | (5c) | (6m) | (7b) |
| 112 | (1d) | (2a) | (3a) | (4a) | (5c) | (6q) | (7b) |
| 113 | (1d) | (2a) | (3a) | (4a) | (5c) | (6r) | (7b) |
| 114 | (1d) | (2a) | (3a) | (4a) | (5c) | (6t) | (7b) |
| 115 | (1d) | (2a) | (3a) | (4d) | (5c) | (6b) | (7b) |
| 116 | (1d) | (2a) | (3a) | (4d) | (5c) | (6c) | (7b) |
| 117 | (1d) | (2a) | (3a) | (4d) | (5c) | (6m) | (7b) |
| 118 | (1d) | (2a) | (3a) | (4d) | (5c) | (6q) | (7b) |
| 119 | (1d) | (2a) | (3a) | (4d) | (5c) | (6r) | (7b) |
| 120 | (1d) | (2a) | (3a) | (4d) | (5c) | (6t) | (7b) |
| 121 | (1d) | (2a) | (3a) | (4g),(4i),(4j),(4k) | (5c) | (6b) | (7b) |
| 122 | (1d) | (2a) | (3a) | (4g),(4i),(4j),(4k) | (5c) | (6c) | (7b) |
| 123 | (1d) | (2a) | (3a) | (4g),(4i),(4j),(4k) | (5c) | (6m) | (7b) |
| 124 | (1d) | (2a) | (3a) | (4g),(4i),(4j),(4k) | (5c) | (6q) | (7b) |
| 125 | (1d) | (2a) | (3a) | (4g),(4i),(4j),(4k) | (5c) | (6r) | (7b) |
| 126 | (1d) | (2a) | (3a) | (4g),(4i),(4j),(4k) | (5c) | (6t) | (7b) |
| 127 | (1d) | (2a) | (3e) | (4a) | (5c) | (6b) | (7b) |
| 128 | (1d) | (2a) | (3e) | (4a) | (5c) | (6c) | (7b) |
| 129 | (1d) | (2a) | (3e) | (4a) | (5c) | (6m) | (7b) |
| 130 | (1d) | (2a) | (3e) | (4a) | (5c) | (6q) | (7b) |
| 131 | (1d) | (2a) | (3e) | (4a) | (5c) | (6r) | (7b) |
| 132 | (1d) | (2a) | (3e) | (4a) | (5c) | (6t) | (7b) |
| 133 | (1d) | (2a) | (3e) | (4d) | (5c) | (6b) | (7b) |
| 134 | (1d) | (2a) | (3e) | (4d) | (5c) | (6c) | (7b) |
| 135 | (1d) | (2a) | (3e) | (4d) | (5c) | (6m) | (7b) |
| 136 | (1d) | (2a) | (3e) | (4d) | (5c) | (6q) | (7b) |
| 137 | (1d) | (2a) | (3e) | (4d) | (5c) | (6r) | (7b) |
| 138 | (1d) | (2a) | (3e) | (4d) | (5c) | (6t) | (7b) |
| 139 | (1d) | (2a) | (3e) | (4g),(4i),(4j),(4k) | (5c) | (6b) | (7b) |
| 140 | (1d) | (2a) | (3e) | (4g),(4i),(4j),(4k) | (5c) | (6c) | (7b) |
| 141 | (1d) | (2a) | (3e) | (4g),(4i),(4j),(4k) | (5c) | (6m) | (7b) |
| 142 | (1d) | (2a) | (3e) | (4g),(4i),(4j),(4k) | (5c) | (6q) | (7b) |
| 143 | (1d) | (2a) | (3e) | (4g),(4i),(4j),(4k) | (5c) | (6r) | (7b) |
| 144 | (1d) | (2a) | (3e) | (4g),(4i),(4j),(4k) | (5c) | (6t) | (7b) |
| 145 | (1d) | (2e) | (3a) | (4a) | (5c) | (6b) | (7b) |
| 146 | (1d) | (2e) | (3a) | (4a) | (5c) | (6c) | (7b) |
| 147 | (1d) | (2e) | (3a) | (4a) | (5c) | (6m) | (7b) |
| 148 | (1d) | (2e) | (3a) | (4a) | (5c) | (6q) | (7b) |
| 149 | (1d) | (2e) | (3a) | (4a) | (5c) | (6r) | (7b) |
| 150 | (1d) | (2e) | (3a) | (4a) | (5c) | (6t) | (7b) |
| 151 | (1d) | (2e) | (3a) | (4d) | (5c) | (6b) | (7b) |
| 152 | (1d) | (2e) | (3a) | (4d) | (5c) | (6c) | (7b) |
| 153 | (1d) | (2e) | (3a) | (4d) | (5c) | (6m) | (7b) |
| 154 | (1d) | (2e) | (3a) | (4d) | (5c) | (6q) | (7b) |
| 155 | (1d) | (2e) | (3a) | (4d) | (5c) | (6r) | (7b) |
| 156 | (1d) | (2e) | (3a) | (4d) | (5c) | (6t) | (7b) |
| 157 | (1d) | (2e) | (3a) | (4g),(4i),(4j),(4k) | (5c) | (6b) | (7b) |
| 158 | (1d) | (2e) | (3a) | (4g),(4i),(4j),(4k) | (5c) | (6c) | (7b) |
| 159 | (1d) | (2e) | (3a) | (4g),(4i),(4j),(4k) | (5c) | (6m) | (7b) |
| 160 | (1d) | (2e) | (3a) | (4g),(4i),(4j),(4k) | (5c) | (6q) | (7b) |
| 161 | (1d) | (2e) | (3a) | (4g),(4i),(4j),(4k) | (5c) | (6r) | (7b) |
| 162 | (1d) | (2e) | (3a) | (4g),(4i),(4j),(4k) | (5c) | (6t) | (7b) |
| 163 | (1d) | (2e) | (3e) | (4a) | (5c) | (6b) | (7b) |
| 164 | (1d) | (2e) | (3e) | (4a) | (5c) | (6c) | (7b) |
| 165 | (1d) | (2e) | (3e) | (4a) | (5c) | (6m) | (7b) |
| 166 | (1d) | (2e) | (3e) | (4a) | (5c) | (6q) | (7b) |
| 167 | (1d) | (2e) | (3e) | (4a) | (5c) | (6r) | (7b) |
| 168 | (1d) | (2e) | (3e) | (4a) | (5c) | (6t) | (7b) |
| 169 | (1d) | (2e) | (3e) | (4d) | (5c) | (6b) | (7b) |
| 170 | (1d) | (2e) | (3e) | (4d) | (5c) | (6c) | (7b) |
| 171 | (1d) | (2e) | (3e) | (4d) | (5c) | (6m) | (7b) |
| 172 | (1d) | (2e) | (3e) | (4d) | (5c) | (6q) | (7b) |
| 173 | (1d) | (2e) | (3e) | (4d) | (5c) | (6r) | (7b) |
| 174 | (1d) | (2e) | (3e) | (4d) | (5c) | (6t) | (7b) |
| 175 | (1d) | (2e) | (3e) | (4g),(4i),(4j),(4k) | (5c) | (6b) | (7b) |
| 176 | (1d) | (2e) | (3e) | (4g),(4i),(4j),(4k) | (5c) | (6c) | (7b) |
| 177 | (1d) | (2e) | (3e) | (4g),(4i),(4j),(4k) | (5c) | (6m) | (7b) |
| 178 | (1d) | (2e) | (3e) | (4g),(4i),(4j),(4k) | (5c) | (6q) | (7b) |
| 179 | (1d) | (2e) | (3e) | (4g),(4i),(4j),(4k) | (5c) | (6r) | (7b) |
| 180 | (1d) | (2e) | (3e) | (4g),(4i),(4j),(4k) | (5c) | (6t) | (7b) |
| 181 | (1d) | (2i) | (3a) | (4a) | (5c) | (6b) | (7b) |
| 182 | (1d) | (2i) | (3a) | (4a) | (5c) | (6c) | (7b) |
| 183 | (1d) | (2i) | (3a) | (4a) | (5c) | (6m) | (7b) |
| 184 | (1d) | (2i) | (3a) | (4a) | (5c) | (6q) | (7b) |
| 185 | (1d) | (2i) | (3a) | (4a) | (5c) | (6r) | (7b) |
| 186 | (1d) | (2i) | (3a) | (4a) | (5c) | (6t) | (7b) |
| 187 | (1d) | (2i) | (3a) | (4d) | (5c) | (6b) | (7b) |
| 188 | (1d) | (2i) | (3a) | (4d) | (5c) | (6c) | (7b) |
| 189 | (1d) | (2i) | (3a) | (4d) | (5c) | (6m) | (7b) |
| 190 | (1d) | (2i) | (3a) | (4d) | (5c) | (6q) | (7b) |
| 191 | (1d) | (2i) | (3a) | (4d) | (5c) | (6r) | (7b) |
| 192 | (1d) | (2i) | (3a) | (4d) | (5c) | (6t) | (7b) |
| 193 | (1d) | (2i) | (3a) | (4g),(4i),(4j),(4k) | (5c) | (6b) | (7b) |
| 194 | (1d) | (2i) | (3a) | (4g),(4i),(4j),(4k) | (5c) | (6c) | (7b) |
| 195 | (1d) | (2i) | (3a) | (4g),(4i),(4j),(4k) | (5c) | (6m) | (7b) |
| 196 | (1d) | (2i) | (3a) | (4g),(4i),(4j),(4k) | (5c) | (6q) | (7b) |
| 197 | (1d) | (2i) | (3a) | (4g),(4i),(4j),(4k) | (5c) | (6r) | (7b) |
| 198 | (1d) | (2i) | (3a) | (4g),(4i),(4j),(4k) | (5c) | (6t) | (7b) |
| 199 | (1d) | (2i) | (3e) | (4a) | (5c) | (6b) | (7b) |
| 200 | (1d) | (2i) | (3e) | (4a) | (5c) | (6c) | (7b) |
| 201 | (1d) | (2i) | (3e) | (4a) | (5c) | (6m) | (7b) |
| 202 | (1d) | (2i) | (3e) | (4a) | (5c) | (6q) | (7b) |
| 203 | (1d) | (2i) | (3e) | (4a) | (5c) | (6r) | (7b) |
| 204 | (1d) | (2i) | (3e) | (4a) | (5c) | (6t) | (7b) |
| 205 | (1d) | (2i) | (3e) | (4d) | (5c) | (6b) | (7b) |
| 206 | (1d) | (2i) | (3e) | (4d) | (5c) | (6c) | (7b) |
| 207 | (1d) | (2i) | (3e) | (4d) | (5c) | (6m) | (7b) |
| 208 | (1d) | (2i) | (3e) | (4d) | (5c) | (6q) | (7b) |
| 209 | (1d) | (2i) | (3e) | (4d) | (5c) | (6r) | (7b) |
| 210 | (1d) | (2i) | (3e) | (4d) | (5c) | (6t) | (7b) |
| 211 | (1d) | (2i) | (3e) | (4g),(4i),(4j),(4k) | (5c) | (6b) | (7b) |
| 212 | (1d) | (2i) | (3e) | (4g),(4i),(4j),(4k) | (5c) | (6c) | (7b) |
| 213 | (1d) | (2i) | (3e) | (4g),(4i),(4j),(4k) | (5c) | (6m) | (7b) |
| 214 | (1d) | (2i) | (3e) | (4g),(4i),(4j),(4k) | (5c) | (6q) | (7b) |
| 215 | (1d) | (2i) | (3e) | (4g),(4i),(4j),(4k) | (5c) | (6r) | (7b) |
| 216 | (1d) | (2i) | (3e) | (4g),(4i),(4j),(4k) | (5c) | (6t) | (7b) |

| **Formula** | **Structural Formula** | **R^{5a} and R^{5b}** | **Q⁵** |
|---|---|---|---|
| 217 | (Ia) | (6b) | (7b) |
| 218 | (Ia) | (6c) | (7b) |
| 219 | (Ia) | (6m) | (7b) |
| 220 | (Ia) | (6q) | (7b) |
| 221 | (Ia) | (6r) | (7b) |
| 222 | (Ia) | (6t) | (7b) |
| 223 | (Ib) | (6b) | (7b) |
| 224 | (Ib) | (6c) | (7b) |
| 225 | (Ib) | (6m) | (7b) |
| 226 | (Ib) | (6q) | (7b) |
| 227 | (Ib) | (6r) | (7b) |
| 228 | (Ib) | (6t) | (7b) |
| 229 | (Ic) | (6b) | (7b) |
| 230 | (Ic) | (6c) | (7b) |
| 231 | (Ic) | (6m) | (7b) |
| 232 | (Ic) | (6q) | (7b) |
| 233 | (Ic) | (6r) | (7b) |
| 234 | (Ic) | (6t) | (7b) |
| 235 | (Id) | (6b) | (7b) |
| 236 | (Id) | (6c) | (7b) |
| 237 | (Id) | (6m) | (7b) |
| 238 | (Id) | (6q) | (7b) |
| 239 | (Id) | (6r) | (7b) |
| 240 | (Id) | (6t) | (7b) |
| 241 | (Ie) | (6b) | (7b) |
| 242 | (Ie) | (6c) | (7b) |
| 243 | (Ie) | (6m) | (7b) |
| 244 | (Ie) | (6q) | (7b) |
| 245 | (Ie) | (6r) | (7b) |
| 246 | (Ie) | (6t) | (7b) |
| 247 | (If) | (6b) | (7b) |
| 248 | (If) | (6c) | (7b) |
| 249 | (If) | (6m) | (7b) |
| 250 | (If) | (6q) | (7b) |
| 251 | (If) | (6r) | (7b) |
| 252 | (If) | (6t) | (7b) |

| **Formula** | **Structural Formula** | **R^{5a} and R^{5b}** | **Q⁵** |
|---|---|---|---|
| 253 | (Ia) | (6g) | (7b) |
| 254 | (Ia) | (6i) | (7b) |
| 255 | (Ia) | (6p) | (7b) |
| 256 | (Ia) | (6x) | (7b) |
| 257 | (Ib) | (6g) | (7b) |
| 258 | (Ib) | (6i) | (7b) |
| 259 | (Ib) | (6p) | (7b) |
| 260 | (Ib) | (6x) | (7b) |
| 261 | (Ic) | (6g) | (7b) |
| 262 | (Ic) | (6i) | (7b) |
| 263 | (Ic) | (6p) | (7b) |
| 264 | (Ic) | (6x) | (7b) |
| 265 | (Id) | (6g) | (7b) |
| 266 | (Id) | (6i) | (7b) |
| 267 | (Id) | (6p) | (7b) |
| 268 | (Id) | (6x) | (7b) |
| 269 | (Ie) | (6g) | (7b) |
| 270 | (Ie) | (6i) | (7b) |
| 271 | (Ie) | (6p) | (7b) |
| 272 | (Ie) | (6x) | (7b) |

In certain additional compounds, including any of the compounds described with reference to formulae (I) and (Ia)-(If) and formulae 1-272 above, each optionally substituted alkyl, alkenyl, and alkynyl recited in any one of preceding compounds is unsubstituted. In alternative additional compounds, including any of the compounds described with reference to formulae (I) and (Ia)-(If) and formulae 1-272 above, each optionally substituted alkyl, alkenyl, and alkynyl recited in any one of preceding compounds is independently substituted or unsubstituted. In further alternative additional compounds, including any of the compounds described with reference to formulae (I) and (Ia)-(If) and formulae 1-272 above, each optionally substituted alkyl, alkenyl, and alkynyl recited in any one of preceding compounds is substituted.

In certain additional compounds, including any of the compounds described with reference to formulae (I) and (Ia)-(If) and formulae 1-272 above and the compound described in the paragraph immediately above, each cycloalkyl recited in any one of the preceding compounds is a 3-7 membered monocyclic cycloalkyl. For example, in certain particular compounds, including any of the compounds described with reference to formulae (I) and (Ia)-(If) and formulae 1-272 above and the compound described in the paragraph immediately above, each cycloalkyl recited in any one of the preceding compounds is a cyclopropyl, a cyclobutyl, a cyclopentyl, a cyclopentenyl, a cyclohexyl or a cyclohexenyl.

In certain additional compounds, including any of the compounds described with reference to formulae (I) and (Ia)-(If) and formulae 1-272 above and any compound described in the two paragraphs immediately above, each heterocycloalkyl recited in any one of the preceding compounds is a 4-7 membered monocyclic heterocycloalkyl having 1-2 heteroatoms selected from O, S and N. For example, in certain particular compounds, including any of the compounds described with reference to formulae (I) and (Ia)-(If) and formulae 1-272 above and any compound described in the two paragraphs immediately above, each heterocycloalkyl recited in any one of the preceding compounds is a pyrrolidinyl, a tetrahydrofuranyl, a tetrahydrothienyl, a piperidinyl, a piperazinyl, a morpholinyl, a thiomorpholinyl, a tetrahydro-2H-pyranyl, or a tetrahydro-2H-thiopyranyl. In certain particular compounds, including any of the compounds described with reference to formulae (I) and (Ia)-(If) and formulae 1-272 above and any compound described in the two paragraphs immediately above, each heterocycloalkyl recited in any one of the preceding compounds is a pyrrolidine, a piperidine, a piperazine, a tetrahydrofuran, a (1H)dihydropyran, or a morpholine (e.g., each unsubstituted).

In certain additional compounds, including any of the compounds described with reference to formulae (I) and (Ia)-(If) and formulae 1-272 above and any compound described in the three paragraphs immediately above, each aryl is phenyl.

In certain additional compounds, including any of the compounds described with reference to formulae (I) and (Ia)-(If) and formulae 1-272 above and any compound described in the four paragraphs immediately above, each heteroaryl is a 5-6 membered monocyclic heteroaryl having 1-3 heteroatoms selected from O, S and N. For example, in certain particular compounds, including any of the compounds described with reference to formulae (I) and (Ia)-(If) and formulae 1-272 above and any compound described in the four paragraphs immediately above, each heteroaryl is a monocyclic heteroaryl is substituted with 0-3 R⁸, e.g., is unsubstituted, substituted with one R⁸ or substituted with two R⁸. In certain particular compounds, including any of the compounds described with reference to formulae (I)) and (la)-(If) and formulae 1-272 above and any compound described in the four paragraphs immediately above, each heteroaryl is a furanyl, a thienyl, a pyrrolyl, a pyrazolyl, an imidazolyl, an oxazolyl or a thiazolyl.

In certain additional compounds, including any of the compounds described with reference to formulae (I) and (Ia)-(If) and formulae 1-272 above and any compound described in the paragraphs immediately above, each R⁷ is independently oxo, C₁-C₄ alkyl, Cl, F, Br, -CN, SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, or -NR^{B}S(O)₁₋₂R^{A}. For example, in certain additional compounds, including any of the compounds described with reference to formulae (I) and (Ia)-(If) and formuale 1-272 above and any compound described in the paragraphs immediately above, each R⁷ is independently oxo, C₁-C₄ alkyl, CI, F, Br, -CN, SF₅, -N₃, nitro, -SR^{A}, -S(O)₁₋₂R^{A}, -OR^{A}, -NR^{B}R^{A}, or -C(O)R^{A}.

In certain additional compounds, including any of the compounds described with reference to formulae (I) and (Ia)-(If) and compounds 1-272 above and any compound described in the paragraphs immediately above, each R⁸ is independently C₁-C₄ alkyl, CI, F, Br, -CN, SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -Co₂R^{A}, -OC(O)R^{A}, -C(O)SR^{A},-SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, or -NR^{B}S(O)₁₋₂R^{A}. In certain additional compounds, including any of the compounds described with reference to formulae (I) and (Ia)-(If) and formulae 1-272 above and any compound described in the paragraphs immediately above, each R⁸ is independently C₁-C₄ alkyl, Cl, F, Br, -CN, SF₅, -N₃, nitro, -SR^{A}, -S(O)₁₋₂R^{A}, -OR^{A}, -NR^{B}R^{A}, or -C(O)R^{A}.

In some embodiments, the present invention relates in particular to a compound selected from:
8,8,11-trimethyl-5-pentyl-4H,8H-benzo[c][1,3]dioxino[4,5-f]chromen-4-one;8,8,11-trimethyl-5-pentyl-2-phenyl-4H,8H-benzo[c][1,3]dioxino[4,5-f]chromen-4-one;
2,8,8,11-tetramethyl-5-pentyl-2-(4-(trifluoromethyl)phenyl)-4H,8H-benzo[c][1,3]dioxino[4,5-f]chromen-4-one;
2-acetyl-2,8,8,11-tetramethyl-5-pentyl-4H,8H-benzo[c][1,3]dioxino[4,5-f]chromen-4-one;
ethyl 2,8,8,11-tetramethyl-4-oxo-5-pentyl-4H,8H-benzo[c][1,3]dioxino[4,5-f]chromene-2-carboxylate;
2,8,8,11-tetramethyl-2-(2-oxopropyl)-5-pentyl-4H,8H-benzo[c][1,3]dioxino[4,5-f]chromen-4-one;
2,8,8,11-tetramethyl-2-(2-methylprop-1-en-1-yl)-5-pentyl-4H,8H-benzo[c][1,3]dioxino[4,5-flchromen-4-one;
8,8,11-trimethyl-5-pentyl-4H,8H-spiro[benzo[c][1,3]dioxino[4,5-f]chromene-2,1'-cyclopentan]-4-one;2-(methoxymethyl)-2,8,8,11-tetramethyl-5-pentyl-4H,8H-benzo[c][1,3]dioxino[4,5-f]chromen-4-one;
8,8,11-trimethyl-2-(2-oxopropyl)-5-pentyl-2-phenyl-4H,8H-benzo[c][1,3]dioxino[4,5-flchromen-4-one;
2-(4-fluorophenyl)-8,8,11-trimethyl-2-(2-oxopropyl)-5-pentyl-4H,8H-benzo[c][1,3]dioxino[4,5-f]chromen-4-one;
8,8,11-trimethyl-2-(2-oxopropyl)-5-pentyl-4H,8H-benzo[c][1,3]dioxino[4,5-f]chromen-4-one;
2-butyl-8,8,11-trimethyl-2-(2-oxopropyl)-5-pentyl-4H,8H-benzo[c][1,3]dioxino[4,5-flchromen-4-one;
2,8,8,11-tetramethyl-5-pentyl-4H,8H-benzo[c][1,3]dioxino[4,5-f]chromen-4-one;
2-ethyl-8,8,11-trimethyl-5-pentyl-4H,8H-benzo[c][1,3]dioxino[4,5-f]chromen-4-one;
8,8,11-trimethyl-5-pentyl-2,2-diphenyl-4H,8H-benzo[c][1,3]dioxino[4,5-f]chromen-4-one;
8,8,11-trimethyl-2-(2-oxopropyl)-5-pentyl-2-(p-tolyl)-4H,8H-benzo[c][1,3]dioxino[4,5-flchromen-4-one;
8,8,11-trimethyl-2-(4-nitrophenyl)-2-(2-oxopropyl)-5-pentyl-4H,8H-benzo[c][1,3]dioxino[4,5-f]chromen-4-one;
2-(4-methoxyphenyl)-8,8,11-trimethyl-2-(2-oxopropyl)-5-pentyl-4H,8H-benzo[c][1,3]dioxino[4,5-f]chromen-4-one;
methyl 4-(8,8,11-trimethyl-4-oxo-2-(2-oxopropyl)-5-pentyl-4H,8H-benzo[c][1,3]dioxino[4,5-f]chromen-2-yl)benzoate;
2-(4-chlorophenyl)-8,8,11-trimethyl-2-(2-oxopropyl)-5-pentyl-4H,8H-benzo[c][1,3]dioxino[4,5-f]chromen-4-one;
8,8,11-trimethyl-5-pentyl-8a,9,10,12a-tetrahydro-4H,8H-benzo[c][1,3]dioxino[4,5-f]chromen-4-one;8,8,11-trimethyl-5-pentyl-2-phenyl-8a,9,10,12a-tetrahydro-4H,8H-benzo[c][1,3]dioxino[4,5-f]chromen-4-one;
2,8,8,11-tetramethyl-5-pentyl-2-(4-(trifluoromethyl)phenyl)-8a,9,10,12a-tetrahydro-4H,8H-benzo[c][1,3]dioxino[4,5-f]chromen-4-one;
2-acetyl-2,8,8,11-tetramethyl-5-pentyl-8a,9,10,12a-tetrahydro-4H,8H-benzo[c][1,3]dioxino[4,5-f]chromen-4-one;
ethyl 2,8,8,11-tetramethyl-4-oxo-5-pentyl-8a,9,10,12a-tetrahydro-4H,8H-benzo[c][1,3]dioxino[4,5-f]chromene-2-carboxylate;
2,8,8,11-tetramethyl-2-(2-oxopropyl)-5-pentyl-8a,9,10,12a-tetrahydro-4H,8H-benzo[c][1,3]dioxino[4,5-f]chromen-4-one;
2,8,8, 11-tetramethyl-2-(2-methylprop-1-en-1-yl)-5-pentyl-8a, 9,10, 12a-tetrahydro-4H ,8H-benzo[c][1,3]dioxino[4,5-f]chromen-4-one;
8,8,11-trimethyl-5-pentyl-8a,9,10,12a-tetrahydro-4H,8H-spiro[benzo[c][1,3]dioxino[4,5-f]chromene-2,1'-cyclopentan]-4-one;2-(methoxymethyl)-2,8,8,11-tetramethyl-5-pentyl-8a,9,10,12a-tetrahydro-4H,8H-benzo[c][1,3]dioxino[4,5-f]chromen-4-one;
8,8,11-trimethyl-2-(2-oxopropyl)-5-pentyl-2-phenyl-8a,9,10,12a-tetrahydro-4H,8H-benzo[c][1,3]dioxino[4,5-f]chromen-4-one;
2-(4-fluorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one;
8,8,11-trimethyl-2-(2-oxopropyl)-5-pentyl-8a,9,10,12a-tetrahydro-4H,8H-benzo[c][1,3]dioxino[4,5-f]chromen-4-one;
2-butyl-8,8,11-trimethyl-2-(2-oxopropyl)-5-pentyl-8a,9,10,12a-tetrahydro-4H,8H-benzo[c][1,3]dioxino[4,5-f]chromen-4-one;
2,8,8,11-tetramethyl-5-pentyl-8a,9,10,12a-tetrahydro-4H,8H-benzo[c][1,3]dioxino[4,5-flchromen-4-one;
2-ethyl-8,8,11-trimethyl-5-pentyl-8a,9,10,12a-tetrahydro-4H,8H-benzo[c][1,3]dioxino[4,5-flchromen-4-one;
8,8,11-trimethyl-5-pentyl-2,2-diphenyl-8a,9,10,12a-tetrahydro-4H,8H-benzo[c][1,3]dioxino[4,5-flchromen-4-one;
8,8,11-trimethyl-2-(2-oxopropyl)-5-pentyl-2-(p-tolyl)-8a,9,10,12a-tetrahydro-4H,8H-benzo[c][1,3]dioxino[4,5-f]chromen-4-one;
8,8,11-trimethyl-2-(4-nitrophenyl)-2-(2-oxopropyl)-5-pentyl-8a,9,10,12a-tetrahydro-4H,8H-benzo[c][1,3]dioxino[4,5-f]chromen-4-one;
2-(4-methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-one;
4-(8,8,11-trimethyl-4-oxo-2-(2-oxopropyl)-5-pentyl-8a,9,10,12a-tetrahydro-4H,8H-benzo[c][1,3]dioxino[4,5-f]chromen-2-yl)benzoate; and
2-(4-chlorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one.

Compounds disclosed herein, but not forming part of the claimed invention, may be selected from:
2,2,8,8,11-pentamethyl-5-pentyl-4H,8H-benzo[c][1,3]dioxino[4,5-f]chromen-4-one;
2-(3-chloropropyl)-2,8,8,11-tetramethyl-5-pentyl-4H,8H-benzo[c][1,3]dioxino[4,5-flchromen-4-one;
2,2,8,8,11-pentamethyl-5-pentyl-8a,9,10,12a-tetrahydro-4H,8H-benzo[c][1,3]dioxino[4,5-flchromen-4-one; and
2-(3-chloropropyl)-2,8,8,11-tetramethyl-5-pentyl-8a,9,10,12a-tetrahydro-4H,8H-benzo[c][1,3]dioxino[4,5-f]chromen-4-one.

In some embodiments, the present invention further relates in particular to a compound selected from:
8,8,11-trimethyl-5-pentyl-4H,8H-benzo[c][1,3]dioxino[4,5-f]chromen-4-one; 8,8,11-trimethyl-5-pentyl-2-phenyl-4H,8H-benzo[c][1,3]dioxino[4,5-f]chromen-4-one;
8,8,11-trimethyl-2-(2-oxopropyl)-5-pentyl-2-phenyl-4H,8H-benzo[c][1,3]dioxino[4,5-flchromen-4-one;
2-(4-fluorophenyl)-8,8,11-trimethyl-2-(2-oxopropyl)-5-pentyl-4H,8H-benzo[c][1,3]dioxino[4,5-f]chromen-4-one;
8,8,11-trimethyl-2-(2-oxopropyl)-5-pentyl-4H,8H-benzo[c][1,3]dioxino[4,5-f]chromen-4-one;
2,8,8,11-tetramethyl-5-pentyl-4H,8H-benzo[c][1,3]dioxino[4,5-f]chromen-4-one;
2-ethyl-8,8,11-trimethyl-5-pentyl-4H,8H-benzo[c][1,3]dioxino[4,5-f]chromen-4-one;
2-(4-methoxyphenyl)-8,8,11-trimethyl-2-(2-oxopropyl)-5-pentyl-4H,8H-benzo[c][1,3]dioxino[4,5-f]chromen-4-one;
2-(4-chlorophenyl)-8,8,11-trimethyl-2-(2-oxopropyl)-5-pentyl-4H,8H-benzo[c][1,3]dioxino[4,5-f]chromen-4-one;
8,8,11-trimethyl-5-pentyl-8a,9,10,12a-tetrahydro-4H,8H-benzo[c][1,3]dioxino[4,5-flchromen-4-one;
8,8,11-trimethyl-5-pentyl-2-phenyl-8a,9,10,12a-tetrahydro-4H,8H-benzo[c][1,3]dioxino[4,5-f]chromen-4-one;
8,8,11-trimethyl-2-(2-oxopropyl)-5-pentyl-2-phenyl-8a,9,10,12a-tetrahydro-4H,8H-benzo[c][1,3]dioxino[4,5-f]chromen-4-one;
2-(4-fluorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one;
8,8,11-trimethyl-2-(2-oxopropyl)-5-pentyl-8a,9,10,12a-tetrahydro-4H,8H-benzo[c][1,3]dioxino[4,5-f]chromen-4-one;
2,8,8,11-tetramethyl-5-pentyl-8a,9,10,12a-tetrahydro-4H,8H-benzo[c][1,3]dioxino[4,5-flchromen-4-one;
2-ethyl-8,8,11-trimethyl-5-pentyl-8a,9,10,12a-tetrahydro-4H,8H-benzo[c][1,3]dioxino[4,5-flchromen-4-one;
2-(4-methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-one; and
2-(4-chlorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one.

In some embodiments, the present invention further relates in particular to a compound selected from:
8,8,11-trimethyl-5-pentyl-8a,9,10,12a-tetrahydro-4H,8H-benzo[c][1,3]dioxino[4,5-flchromen-4-one;
8,8,11-trimethyl-5-pentyl-2-phenyl-8a,9,10,12a-tetrahydro-4H,8H-benzo[c][1,3]dioxino[4,5-f]chromen-4-one;
8,8,11-trimethyl-2-(2-oxopropyl)-5-pentyl-2-phenyl-8a,9,10,12a-tetrahydro-4H,8H-benzo[c][1,3]dioxino[4,5-f]chromen-4-one;
2-(4-fluorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one;
8,8,11-trimethyl-2-(2-oxopropyl)-5-pentyl-8a,9,10,12a-tetrahydro-4H,8H-benzo[c][1,3]dioxino[4,5-f]chromen-4-one;
2,8,8,11-tetramethyl-5-pentyl-8a,9,10,12a-tetrahydro-4H,8H-benzo[c][1,3]dioxino[4,5-flchromen-4-one;
2-ethyl-8,8,11-trimethyl-5-pentyl-8a,9,10, 12a-tetrahydro-4H,8H-benzo[c][1,3]dioxino[4,5-flchromen-4-one;
2-(4-methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-one; and
2-(4-chlorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one.

In certain embodiments of the compounds as otherwise described herein, the compound is in the form of a pharmaceutically acceptable salt of the compound as described herein or an enantiomer, diastereomer, racemate, or tautomer thereof. The person of ordinary skill in the art will appreciate that a variety of pharmaceutically acceptable salts may be provided, such as for example described in additional detail below.

In certain embodiments of the compounds as otherwise described herein, a compound is in the form of a solvate (e.g., a hydrate) of a compound as described herein or an enantiomer, diastereomer, racemate, or tautomer thereof. The person of ordinary skill in the art will appreciate that a variety of solvates and/or hydrates may be formed.

The person of ordinary skill in the art will appreciate that the phrase "or an enantiomer, diastereomer, racemate, or tautomer thereof, or a pharmaceutically acceptable salt, solvate or hydrate of the compound, enantiomer, diastereomer, racemate, or tautomer" includes compounds in the form of salts, solvates, hydrates of the base compounds (including its enantiomers, diastereomers, racemates, or tautomers). But in certain embodiments as described above, the compound is not in the form of a salt, solvate or hydrate.

### Therapeutics Applications

Compounds of the present invention have an affinity for at least one cannabinoid receptor (e.g. CB1, CB2, 5HT1A, 5HT2A, GPR18, GPR55, GPR119, TRPV1, TPRV2, PPARs (such as PPARγ), or a µ-opioid receptor). Thus, the compounds bind and/or interact with a cannabinoid receptor.

In some embodiments, compounds of the present invention may have an affinity for one or both of the CB1 receptor and CB2 receptor. In a particular embodiment, compounds of the present invention may have an affinity for the CB1 receptor, but not the CB2 receptor. In a particular embodiment, compounds of the present invention may have an affinity for the CB2 receptor, but not the CB1 receptor.

In some embodiments, compounds of the present invention may exhibit a selectivity for one receptor over another. As used herein, by "selectivity" it is meant that a compound binds, interacts with, or modulates preferentially one receptor over another. In an embodiment, a compound is selective for one receptor over another if its affinity for one receptor is at least 1.25-fold, at least 1.5-fold, at least 2-fold, at least 5-fold, at least 10-fold, at least 25-fold, at least 50-fold, or at least 100-fold greater for the one receptor than another receptor. In an embodiment, the affinity of a compound as disclosed herein for a receptor may be determined by a radioligand competitive binding assay, such as for example described in the examples and reference examples herein.

In some embodiments, compounds of the present invention may have an affinity for the CB1 receptor, with selectivity for the CB1 receptor over the CB2 receptor.

In some embodiments, compounds of the present invention may have an affinity for the CB2 receptor, with selectivity for the CB2 receptor over the CB1 receptor.

In some embodiments, compounds of the present invention may function as an agonist, a partial agonist, an indirect agonist, an antagonist, an inverse agonist, a neutral agonist, or an allosteric modulator to at least one cannabinoid receptor (e.g. CB1, CB2, 5HT1A, 5HT2A, GPR18, GPR55, GPR119, TRPV1, TPRV2, PPARs (such as PPARγ), or a µ-opioid receptor). In some embodiments, compounds of the present invention may function as an agonist, a partial agonist, an indirect agonist, antagonist, inverse agonist, neutral agonist, or allosteric modulator to the CB1 and/or CB2 receptor. In some embodiments, compounds of the present invention may function as an agonist to the CB1 and/or CB2 receptor. In some embodiments, compounds of the present invention may function as an antagonist to the CB1 and/or CB2 receptor. In some embodiments, compounds of the present invention may function as an indirect agonist to the 5HT1A receptor.

As disclosed herein, but not forming part of the claimed invention, the compounds may act as a prodrug to a compound (e.g. a metabolite) that functions as an agonist, a partial agonist, an indirect agonist, an antagonist, an inverse agonist, or a neutral agonist to at least one of the cannabinoid receptors (e.g. CB1, CB2, 5HT1A, 5HT2A, GPR18, GPR55, GPR119, TRPV1, TPRV2, PPARs (e.g. PPARγ), or a µ-opioid receptor).

Also disclosed herein but not forming part of the claimed invention is a compound as disclosed herein for use as a therapeutically active substance or as a prodrug to a therapeutically active substance.

In an embodiment, the present invention relates to a compound as disclosed herein for use in treating or preventing a disease associated with a cannabinoid receptor (e.g. CB1, CB2, 5HT1A, 5HT2A, GPR18, GPR55, GPR119, TRPV1, TPRV2, PPARs (e.g. PPARγ), or a µ-opioid receptor). In the present invention, "disease associated with a cannabinoid" is meant to refer to a disease, disorder or condition that is treatable or preventable by acting on a cannabinoid receptor (e.g. CB1, CB2, 5HT1A, 5HT2A, GPR18, GPR55, GPR119, TRPV1, TPRV2, PPARs (e.g. PPARγ), or a µ-opioid receptor). In an embodiment, compounds of the present invention may treat or prevent the condition by acting as an agonist, partial agonist, antagonist, inverse agonist, neutral agonist, or an allosteric modulator to the cannabinoid receptor. In select embodiments, the cannabinoid receptor is CB1 or CB2.

Disclosed herein, but not forming part of the claimed invention is the use of a compound as disclosed herein for selectively modulating the activity of one receptor over another receptor. At least one of the receptors may be a cannabinoid receptor. Alternatively, both receptors may be a cannabinoid receptor. In some instances, the receptors are CB1 and CB2. Also disclosed herein is the use of a compound as disclosed herein for selectively modulating the activity of a CB1 or CB2 receptor.

As used herein, by "selectively modulating" it is intended to refer to the ability of the compounds to cause any change in activity of the receptor. In some instances, "selectively modulating" means to stimulate or inhibit the activity of the receptor, either directly or indirectly.

Disclosed herein, but not forming part of the claimed invention, are methods of treating or preventing a disease, such as a disease associated with a cannabinoid receptor (e.g. CB1 and/or CB2). These methods include administering to a subject in need of such treatment or prevention a therapeutically effective amount of one or more compounds as described herein (e.g., compounds of formula (I)) or a pharmaceutical composition as described herein.

The diseases to be treated can include, but are not limited to ADHD/ADD, alcohol use disorder, allergic asthma, ALS, Alzheimer's, anorexia (e.g. HIV-related cachexia), anxiety disorders (e.g., social anxiety disorder, specific phobia, test anxiety, generalized anxiety disorder), arthritis, atherosclerosis, autism, bipolar disorder, burns, cancer, cancer pain, Charcot-Marie-Tooth disease, chronic inflammatory demyelinating polyneuropathies, chronic allograft nephropathy, cocaine use disorder, complex regional pain syndrome, congestive heart failure, depression, fibromyalgia, fragile X syndrome/FXTAS, frontotemporal dementias (behavioural variant), gingivitis pyrexia, glaucoma, glioblastoma, glomerulonephropathy, Huntington's disease, hypertrophic scars, IBD/IBS, inflammation, Inflammatory myopathies, ischemia, kidney fibrosis, keloids, leukodystrophies, liver fibrosis, liver cirrhosis, lung fibrosis, migraine, multiple sclerosis, myocardial infarction, nausea (e.g., CINV, motion sickness), neuropathic pain (e.g., postherpetic neuralgia, painful diabetic neuropathy), nightmare disorder, non-alcoholic fatty liver disease, obesity, obsessive-compulsive disorder, opioid sparing, opioid use disorder, osteoarthritis, osteoporosis, pain (e.g. acute or chronic pain), Parkinson's, post-concussion syndrome/traumatic brain injury, psychosis/schizophrenia, PTSD, regulation of bone mass, REM sleep behaviour disorder, reperfusion injury, Rett syndrome, rheumatoid arthritis, skin conditions (e.g. acne, psoriatic arthritis), sleep disorders (e.g., insomnia, RLS), spinocerebellar ataxias, systemic fibrosis, systemic sclerosis, thermal injury, tobacco use disorder/nicotine dependence, Tourette's, tumors, and trigeminal neuralgia.

The compounds and compositions as described herein may also be administered in combination with one or more secondary therapeutic agents. Thus, in certain embodiments, the treatment also includes administering to a subject in need of such treatment or prevention a therapeutically effective amount of one or more compounds of the invention as described herein or a pharmaceutical composition of the invention as described herein and one or more secondary therapeutic agents. Examples of suitable secondary therapeutic agents include, but are not limited to, temozolomide, camptothecin, doxorubicin, daunorubicin, vincristine, paclitaxel, neocarzinostatin, calicheamicin, cisplatin, carboplatin, oxaliplatin, satraplatin, picoplatin, lurtotecan, annamycin, docetaxel, tamoxifen, epirubicin, methotrexate, vinblastin, vincristin, topotecan, prednisone, prednisolone, chloroquine, hydroxychloroquine, autophagy inhibitors, abt-737, leucovorin, psilocybin, psilocin, psiloacetin, netupitant, palonosetron, aprepitant, 3,4-methylenedioxymethamphetamine, nicotine, ketamine, lithium salts (e.g., lithium citrate), valproic acid, bevacizumab, bortezomib, fluorouracil, gemcitabine, irinotecan, oxaliplatin, adalimumab, azathioprine, infliximab, citalopram, mirtazapine, sertraline, esketamine, fluoxetine, paroxetine, venlafaxine, fenfluramine, vigabatrin, bupropion, atomoxetine, memantine, clobazam, stiripentol, cyclosporine, tacrolimus, methylprednisolone, megestrol acetate, biguanides (e.g., metformin), sulphonyl ureas (e.g., glipizide, tolbutamide), gabapentin, baclofen, clonazepam, dantrolene, diazepam, tizanidine, buprenorphine, naltrexone, opioids (e.g., codeine, oxycodone, morphine), amisulpride, aripiprazole, olanzapine, quetiapine, risperidone, clonidine, lomazenil, benzodiazepine, benzamide, dexamethasone, gemcitabine, and palmitoylethanolamide. When administered as a combination, the compounds and compositions as described herein and the secondary therapeutic agents can be formulated as separate compositions that are given simultaneously or sequentially, or the therapeutic agents can be given as a single composition. The secondary therapeutic agent may be administered in an amount below its established half maximal inhibitory concentration (IC₅₀). For example, the secondary therapeutic agent may be administered in an amount less than 1% of, e.g., less than 10%, or less than 25%, or less than 50%, or less than 75%, or even less than 90% of the inhibitory concentration (IC₅₀).

### Prodrugs

A compound as described herein may itself be a prodrug or may be further modified to provide a prodrug. Prodrugs are not part of the claimed invention.

Prodrugs may, for example, be produced by replacing appropriate functionalities present in the compounds disclosed herein, such as for example any of the compounds described with reference to formulae (I) and (Ia)-(If) and formulae 1-272 above, with certain moieties known to those skilled in the art as "pro-moieties" as described, for example, in Design of Prodrugs by H. Bundgaard (Elsevier, 1985) and Y.M. Choi-Sledeski and C.G. Wermuth, Designing Prodrugs and Bioprecursors in Practice of Medicinal Chemistry, (Fourth Edition), Chapter 28, 657-696 (Elsevier, 2015).

A prodrug in accordance with the present disclosure may be (a) an ester or amide derivative of a carboxylic acid in a compound disclosed herein; (b) an ester, carbonate, carbamate, acetal, aminal, phosphate, or ether derivative of a hydroxyl group in a compound disclosed herein; (c) an amide, imine, carbamate or amine derivative of an amino group in a compound disclosed herein; (d) an oxime or imine derivative of a carbonyl group in a compound disclosed herein; or (e) a methyl, primary alcohol or aldehyde group that can be metabolically oxidized to a carboxylic acid in a compound disclosed herein.

Certain compounds disclosed herein , such as for example any of the compounds described with reference to formulae (I) and (Ia)-(If) and formulae 1-272 above, may themselves act as prodrugs. In some instances, a compound disclosed herein, such as for example any of the compounds described with reference to formulae (I) and (Ia)-(If) and formulae 1-272 above, may act as a prodrug of a cannabinoid, such as for example a natural cannabinoid (e.g., CBD or CBDA).

References to compounds disclosed herein are taken to include the compounds themselves and prodrugs thereof. The present disclosure includes such prodrug compounds as well as any pharmaceutically acceptable salts of such prodrug compounds, and/or any solvates, hydrates, enantiomers, diastereomers, racemates, or tautomers of such prodrug compounds and their salts. Prodrugs are not part of the claimed invention

A prodrug compound as disclosed herein, but not forming part of the claimed invention, may exhibit improved pharmacokinetics (PK), improved biodistribution, and/or improved formulation capabilities (e.g. stability in formulation). For example, these characteristics may be improved over natural cannabinoids (e.g., CBD or CBDA).

A prodrug compound as disclosed herein, but not forming part of the claimed invention, may exhibit unique or improved biological activity. For example, these characteristics may be unique or improved over natural cannabinoids (e.g., CBD or CBDA).

### Pharmaceutical Compositions and Dosage Forms

A compound as described herein can usefully be provided in the form of a pharmaceutical composition. Such compositions include the compound as described herein, together with a pharmaceutically acceptable excipient, diluent, or carrier.

The compounds may be formulated in the pharmaceutical composition per se, or in the form of a hydrate, solvate, or pharmaceutically acceptable salt, as previously described. Typically, such salts are more soluble in aqueous solutions than the corresponding free acids and bases, but salts having lower solubility than the corresponding free acids and bases may also be formed.

The pharmaceutical composition can be, for example, in the form of a tablet, a capsule, or a parenteral formulation, but the person of ordinary skill in the art will appreciate that the compound can be provided in a wide variety of pharmaceutical compositions.

The compounds can be administered, for example, orally, topically, parenterally, by inhalation or spray or rectally in dosage unit formulations containing one or more pharmaceutically acceptable carriers, diluents or excipients. The term parenteral as used herein includes percutaneous, subcutaneous, intravascular (e.g., intravenous), intramuscular, or intrathecal injection or infusion techniques and the like. A medicament including a compound can be provided, for example, in any of the formulations and dosage forms as described herein.

Pharmaceutical compositions can be made using the presently disclosed compounds. A pharmaceutical composition includes a pharmaceutically acceptable carrier, diluent or excipient, and compound as described above with reference to any one of structural formulae.

In the pharmaceutical compositions disclosed herein, one or more compounds of the invention are present in association with one or more pharmaceutically acceptable carriers, diluents or excipients, and, if desired, other active ingredients. The pharmaceutical compositions containing compounds of the invention may be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to any suitable method for the manufacture of pharmaceutical compositions and such compositions may contain one or more agents selected from the group consisting of sweetening agents, flavoring agents, coloring agents and preservative agents in order to provide pharmaceutically elegant and palatable preparations. Tablets contain the active ingredient in admixture with non-toxic pharmaceutically acceptable excipients that are suitable for the manufacture of tablets. These excipients can be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets can be uncoated or they can be coated by known techniques. In some cases such coatings can be prepared by suitable techniques to delay disintegration and absorption in the gastrointestinal tract and thereby provide a sustained action over a longer period. For example, a time delay material such as glyceryl monostearate or glyceryl distearate can be employed.

Formulations for oral use can also be presented as hard gelatin capsules, wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Formulations for oral use can also be presented as lozenges.

Formulations for oral use can also be presented as beverages or edibles. For example, the compounds may be presented in water-soluble formulations such as disclosed in PCT/CA2019/051698, comprising an emulsifier and a glycerin-based carrier surfactant. In other instances, the compounds may be presented in compositions such as disclosed in PCT/CA2019/051704, comprising inulin and pectin.

Aqueous suspensions contain the active materials in admixture with excipients suitable for the manufacture of aqueous suspensions. Such excipients can be suspending agents, for example sodium carboxymethylcellulose, methylcellulose, hydropropylmethylcellulose, sodium alginate, polyvinylpyrrolidone, gum tragacanth and gum acacia; dispersing or wetting agents such as a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide palatable oral preparations. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavoring agents.

The pharmaceutically acceptable carrier, diluent, or excipient may be not water. Alternatively, the water may comprise less than 50% of the composition. Compositions comprising less than 50% water may have at least 1%, 2%, 3%, 4% or 5% water. In other instances, the water content may be present in the composition in a trace amount.

The pharmaceutically acceptable carrier, diluent, or excipient may be not alcohol. Alternatively, the alcohol may comprise less than 50% of the composition. Compositions comprising less than 50% alcohol may have at least 1%, 2%, 3%, 4% or 5% alcohol. In other instances, the alcohol content may be present in the composition in a trace amount.

Syrups and elixirs can be formulated with sweetening agents, for example glycerol, propylene glycol, sorbitol, glucose or sucrose. Such formulations can also contain a demulcent, a preservative, flavoring, and coloring agents. The pharmaceutical compositions can be in the form of a sterile injectable aqueous or oleaginous suspension. This suspension can be formulated according to the known art using those suitable dispersing or wetting agents and suspending agents that have been mentioned above. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils can be employed as a solvent or suspending medium. For this purpose any bland fixed oil can be employed including synthetic mono- or diglycerides. In addition, fatty acids such as oleic acid find use in the preparation of injectables.

The compounds can also be administered in the form of suppositories, e.g., for rectal administration of the drug. These compositions can be prepared by mixing the compound with a suitable non-irritating excipient that is solid at ordinary temperatures but liquid at the rectal temperature and will therefore melt in the rectum to release the drug. Such materials include cocoa butter and polyethylene glycols.

The compounds can also be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

The compositions can be formulated in a unit dosage form of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The compound can be effective over a wide dosage range and is generally administered in a therapeutically effective amount. It will be understood, however, that the amount of the compound actually administered will usually be determined by a physician, according to the relevant circumstances, including the condition to be treated or prevented, the chosen route of administration, the actual compound administered, the age, weight, and response of the individual patient, the severity of the patient's symptoms, and the like.

For preparing solid compositions such as tablets, the principal active ingredient is mixed with a pharmaceutical excipient to form a solid preformulation composition containing a homogeneous mixture of a compound described herein. When referring to these preformulation compositions as homogeneous, the active ingredient is typically dispersed evenly throughout the composition so that the composition can be readily subdivided into equally effective unit dosage forms such as tablets, pills and capsules. This solid preformulation is then subdivided into unit dosage forms of the type described above containing from, for example, 0.1 to about 500 mg of the active ingredient of a compound described herein.

The tablets or pills can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The amount of compound or composition administered to a patient will vary depending upon what is being administered, the purpose of the administration, such as prophylaxis or therapy, the state of the patient, the manner of administration, and the like. In therapeutic applications, compositions can be administered to a patient already suffering from a disease in an amount sufficient to cure or at least partially arrest the symptoms of the disease and its complications. Effective doses will depend on the disease condition being treated or prevented as well as by the judgment of the attending clinician depending upon factors such as the severity of the disease, the age, weight and general condition of the patient, and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers, or stabilizers will result in the formation of pharmaceutical salts.

The therapeutic dosage of the compounds can vary according to, for example, the particular use for which the treatment is made, the manner of administration of the compound, the health and condition of the patient, and the judgment of the prescribing physician. The proportion or concentration of a compound described herein in a pharmaceutical composition can vary depending upon a number of factors including dosage, chemical characteristics (e.g., hydrophobicity), and the route of administration. For example, the compounds described herein can be provided in an aqueous physiological buffer solution containing about 0.1 to about 10% w/v of the compound for parenteral administration. Some typical dose ranges are from about 1 µg/kg to about 1 g/kg of body weight per day. In some instances, the dose range may be from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from *in vitro* or animal model test systems.

The compounds described herein can also be formulated in combination with one or more additional active ingredients which can include any pharmaceutical agent such as anti-viral agents, vaccines, antibodies, immune enhancers, immune suppressants, anti-inflammatory agents and the like.

The person of ordinary skill in the art will formulate a compound as described into pharmaceutical formulations herein. For example, based on the physicochemical properties of the compound, the amount of the compound needed for a therapeutically effective amount, and the desired route of administration.

### Definitions

Terms used herein may be preceded and/or followed by a single dash, "-", or a double dash, "=", to indicate the bond order of the bond between the named substituent and its parent moiety; a single dash indicates a single bond and a double dash indicates a double bond or a pair of single bonds in the case of a spiro-substituent. In the absence of a single or double dash it is understood that a single bond is formed between the substituent and its parent moiety; further, substituents are intended to be read "left to right" with reference to the chemical structure referred to unless a dash indicates otherwise. For example, arylalkyl, arylalkyl-, and -alkylaryl indicate the same functionality.

For simplicity, chemical moieties are defined and referred to throughout primarily as univalent chemical moieties (e.g., alkyl, aryl, etc.). Nevertheless, such terms are also used to convey corresponding multivalent moieties under the appropriate structural circumstances clear to those skilled in the art. For example, while an "alkyl" moiety can refer to a monovalent radical (e.g., CH₃-CH₂-), in some circumstances a bivalent linking moiety can be "alkyl," in which case those skilled in the art will understand the alkyl to be a divalent radical (e.g., -CH₂-CH₂-), which is equivalent to the term "alkylene." (Similarly, in circumstances in which a divalent moiety is required and is stated as being "aryl," those skilled in the art will understand that the term "aryl" refers to the corresponding divalent moiety, arylene). All atoms are understood to have their normal number of valences for bond formation (i.e., 4 for carbon, 3 for N, 2 for O, and 2, 4, or 6 for S, depending on the oxidation state of the S). Nitrogens in the presently disclosed compounds can be hypervalent, e.g., an N-oxide or tetrasubstituted ammonium salt. On occasion a moiety may be defined, for example, as -B-(A)ₐ, wherein a is 0 or 1. In such instances, when a is 0 the moiety is -B and when a is 1 the moiety is -B-A.

As used herein, the term "alkyl" includes a saturated hydrocarbon having a designated number of carbon atoms, such as 1 to 10 carbons (i.e., inclusive of 1 and 10), 1 to 8 carbons, 1 to 6 carbons, 1 to 3 carbons, or 1, 2, 3, 4, 5 or 6. Alkyl group may be straight or branched and depending on context, may be a monovalent radical or a divalent radical (i.e., an alkylene group). For example, the moiety "-(C₁-C₆alkyl)-O-" signifies connection of an oxygen through an alkylene bridge having from 1 to 6 carbons and C₁-C₃alkyl represents methyl, ethyl, and propyl moieties. Examples of "alkyl" include, for example, methyl, ethyl, propyl, isopropyl, butyl, iso-, sec- and tert-butyl, pentyl, and hexyl.

The term "alkoxy" represents an alkyl group of indicated number of carbon atoms attached to the parent molecular moiety through an oxygen bridge, such as alkyl-O- in which the term "alkyl" has the previously given definition. Examples of "alkoxy" include, for example, methoxy, ethoxy, propoxy, and isopropoxy.

The term "alkenyl", as used herein, represents an unsaturated hydrocarbon having a designated number of carbon atoms, such as 2 to 10 carbons (i.e., inclusive of 2 and 10), 2 to 8 carbons, 2 to 6 carbons, or 2, 3, 4, 5 or 6, unless otherwise specified, and containing at least one carbon-carbon double bond. Alkenyl group may be straight or branched and depending on context, may be a monovalent radical or a divalent radical (i.e., an alkenylene group). For example, the moiety "-(C₂-C₆ alkenyl)-O-" signifies connection of an oxygen through an alkenylene bridge having from 2 to 6 carbons. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, 3-decenyl, and 3,7-dimethylocta-2,6-dienyl.

The term "alkynyl", as used herein, represents an unsaturated hydrocarbon having a designated number of carbon atoms, such as 2 to 10 carbons (i.e., inclusive of 2 and 10), 2 to 8 carbons, 2 to 6 carbons, or 2, 3, 4, 5 or 6, unless otherwise specified, and containing at least one carbon-carbon triple bond. Alkynyl group may be straight or branched and depending on context, may be a monovalent radical or a divalent radical (i.e., an alkynylene group). For example, the moiety "-(C₂-C₆ alkynyl)-O-" signifies connection of an oxygen through an alkynylene bridge having from 2 to 6 carbons. Representative examples of alkynyl include, but are not limited to, acetylenyl, 1-propynyl, 2-propynyl, 3-butynyl, 2-pentynyl, and 1-butynyl.

The term "aryl" represents an aromatic ring system having a single ring (e.g., phenyl) which is optionally fused to other aromatic hydrocarbon rings or non-aromatic hydrocarbon or heterocycle rings. "Aryl" includes ring systems having multiple condensed rings and in which at least one is carbocyclic and aromatic, (e.g., 1,2,3,4-tetrahydronaphthyl, naphthyl). Examples of aryl groups include phenyl, 1-naphthyl, 2-naphthyl, indanyl, indenyl, dihydronaphthyl, fluorenyl, tetralinyl, and 6,7,8,9-tetrahydro-5*H*-benzo[a]cycloheptenyl. "Aryl" also includes ring systems having a first carbocyclic, aromatic ring fused to a nonaromatic heterocycle, for example, 1H-2,3-dihydrobenzofuranyl and tetrahydroisoquinolinyl. The aryl groups herein are unsubstituted or, when specified as "optionally substituted", can unless stated otherwise be substituted in one or more substitutable positions with various groups as indicated.

The terms "halogen" or "halo" indicate fluorine, chlorine, bromine, and iodine. In certain embodiments of each and every embodiment as otherwise described herein, the term "halogen" or "halo" refers to fluorine or chlorine. In certain embodiments of each and every embodiment described herein, the term "halogen" or "halo" refers to fluorine. The term "fluoroalkyl" indicates an alkyl group (i.e., as otherwise described herein) that is substiuted with at least one fluorine. "Fluoroalkyl" includes alkyl groups substituted with multiple fluorines, such as perfluoroalkyl groups. Examples of fluoroalkyl groups include fluoromethyl, difluoromethyl, trifluoromethyl, pentafluoroethyl, 2,2,2-trifluoroethyl, 1,1,1,3,3,3-hexafluoroprop-2-yl and 2,2,3,3,3-pentafluoroprop-1-yl.

The term "heteroaryl" refers to an aromatic ring system containing at least one aromatic heteroatom selected from nitrogen, oxygen and sulfur in an aromatic ring. Most commonly, the heteroaryl groups will have 1, 2, 3, or 4 heteroatoms. The heteroaryl may be fused to one or more non-aromatic rings, for example, cycloalkyl or heterocycloalkyl rings, wherein the cycloalkyl and heterocycloalkyl rings are described herein. In one embodiment of the present compounds the heteroaryl group is bonded to the remainder of the structure through an atom in a heteroaryl group aromatic ring. In another embodiment, the heteroaryl group is bonded to the remainder of the structure through a non-aromatic ring atom. Examples of heteroaryl groups include, for example, pyridyl, pyrimidinyl, quinolinyl, benzothienyl, indolyl, indolinyl, pyridazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, benzo[1,4]oxazinyl, triazolyl, tetrazolyl, isothiazolyl, naphthyridinyl, isochromanyl, chromanyl, isoindolinyl, isobenzothienyl, benzoxazolyl, pyridopyridinyl, purinyl, benzodioxolyl, triazinyl, pteridinyl, benzothiazolyl, imidazopyridinyl, imidazothiazolyl, benzisoxazinyl, benzoxazinyl, benzopyranyl, benzothiopyranyl, chromonyl, chromanonyl, pyridinyl-*N*-oxide, isoindolinonyl, benzodioxanyl, benzoxazolinonyl, pyrrolyl *N*-oxide, pyrimidinyl *N*-oxide, pyridazinyl *N*-oxide, pyrazinyl *N*-oxide, quinolinyl *N*-oxide, indolyl *N*-oxide, indolinyl *N*-oxide, isoquinolyl *N*-oxide, quinazolinyl *N*-oxide, quinoxalinyl *N*-oxide, phthalazinyl *N*-oxide, imidazolyl *N*-oxide, isoxazolyl *N*-oxide, oxazolyl *N-*oxide, thiazolyl *N*-oxide, indolizinyl *N*-oxide, indazolyl *N*-oxide, benzothiazolyl *N*-oxide, benzimidazolyl *N*-oxide, pyrrolyl *N*-oxide, oxadiazolyl *N*-oxide, thiadiazolyl *N*-oxide, triazolyl *N-*oxide, tetrazolyl *N*-oxide, benzothiopyranyl S-oxide, benzothiopyranyl S,S-dioxide. Preferred heteroaryl groups include pyridyl, pyrimidyl, quinolinyl, indolyl, pyrrolyl, furanyl, thienyl and imidazolyl, pyrazolyl, indazolyl, thiazolyl and benzothiazolyl. In certain embodiments, each heteroaryl is selected from pyridyl, pyrimidinyl, pyridazinyl, pyrazinyl, imidazolyl, isoxazolyl, pyrazolyl, oxazolyl, thiazolyl, furanyl, thienyl, pyrrolyl, oxadiazolyl, thiadiazolyl, triazolyl, tetrazolyl, isothiazolyl, pyridinyl-*N*-oxide, pyrrolyl *N*-oxide, pyrimidinyl *N*-oxide, pyridazinyl *N-*oxide, pyrazinyl *N*-oxide, imidazolyl *N*-oxide, isoxazolyl *N*-oxide, oxazolyl *N*-oxide, thiazolyl *N-*oxide, pyrrolyl *N*-oxide, oxadiazolyl *N*-oxide, thiadiazolyl *N*-oxide, triazolyl N*-*oxide, and tetrazolyl *N*-oxide. Preferred heteroaryl groups include pyridyl, pyrimidyl, quinolinyl, indolyl, pyrrolyl, furanyl, thienyl, imidazolyl, pyrazolyl, indazolyl, thiazolyl and benzothiazolyl. The heteroaryl groups herein are unsubstituted or, when specified as "optionally substituted", can unless stated otherwise be substituted in one or more substitutable positions with various groups, as indicated.

The term "heterocycloalkyl" refers to a non-aromatic ring or ring system containing at least one heteroatom that is preferably selected from nitrogen, oxygen and sulfur, wherein said heteroatom is in a non-aromatic ring. The heterocycloalkyl may have 1, 2, 3 or 4 heteroatoms. The heterocycloalkyl may be saturated (i.e., a heterocycloalkyl) or partially unsaturated (i.e., a heterocycloalkenyl). Heterocycloalkyl includes monocyclic groups of three to eight annular atoms as well as bicyclic and polycyclic ring systems, including bridged and fused systems, wherein each ring includes three to eight annular atoms. The heterocycloalkyl ring is optionally fused to other heterocycloalkyl rings and/or non-aromatic hydrocarbon rings. In certain embodiments, the heterocycloalkyl groups have from 3 to 7 members in a single ring. In other embodiments, heterocycloalkyl groups have 5 or 6 members in a single ring. In some embodiments, the heterocycloalkyl groups have 3, 4, 5, 6 or 7 members in a single ring. Examples of heterocycloalkyl groups include, for example, azabicyclo[2.2.2]octyl (in each case also "quinuclidinyl" or a quinuclidine derivative), azabicyclo[3.2.1]octyl, 2,5-diazabicyclo[2.2.1]heptyl, morpholinyl, thiomorpholinyl, thiomorpholinyl S-oxide, thiomorpholinyl S,S-dioxide, 2-oxazolidonyl, piperazinyl, homopiperazinyl, piperazinonyl, pyrrolidinyl, azepanyl, azetidinyl, pyrrolinyl, tetrahydropyranyl, piperidinyl, tetrahydrofuranyl, tetrahydrothienyl, 3,4-dihydroisoquinolin-2(1*H*)-yl, isoindolindionyl, homopiperidinyl, homomorpholinyl, homothiomorpholinyl, homothiomorpholinyl S,S-dioxide, oxazolidinonyl, dihydropyrazolyl, dihydropyrrolyl, dihydropyrazinyl, dihydropyridinyl, dihydropyrimidinyl, dihydrofuryl, dihydropyranyl, imidazolidonyl, tetrahydrothienyl S-oxide, tetrahydrothienyl S,S-dioxide and homothiomorpholinyl S-oxide. Especially desirable heterocycloalkyl groups include morpholinyl, 3,4-dihydroisoquinolin-2(1*H*)-yl, tetrahydropyranyl, piperidinyl, aza-bicyclo[2.2.2]octyl, γ-butyrolactonyl (i.e., an oxo-substituted tetrahydrofuranyl), γ-butryolactamyl (i.e., an oxo-substituted pyrrolidine), pyrrolidinyl, piperazinyl, azepanyl, azetidinyl, thiomorpholinyl, thiomorpholinyl S,S-dioxide, 2-oxazolidonyl, imidazolidonyl, isoindolindionyl, piperazinonyl. The heterocycloalkyl groups herein are unsubstituted or, when specified as "optionally substituted", can unless stated otherwise be substituted in one or more substitutable positions with various groups, as indicated.

The term "cycloalkyl" refers to a non-aromatic carbocyclic ring or ring system, which may be saturated (i.e., a cycloalkyl) or partially unsaturated (i.e., a cycloalkenyl). The cycloalkyl ring may be optionally fused to or otherwise attached (e.g., bridged systems) to other cycloalkyl rings. Certain examples of cycloalkyl groups have from 3 to 7 members in a single ring, such as having 5 or 6 members in a single ring. In some embodiments, the cycloalkyl groups have 3, 4, 5, 6 or 7 members in a single ring. Examples of cycloalkyl groups include, for example, cyclohexyl, cyclopentyl, cyclobutyl, cyclopropyl, tetrahydronaphthyl and bicyclo[2.2.1]heptane. The cycloalkyl groups herein are unsubstituted or, when specified as "optionally substituted", may be substituted in one or more substitutable positions with various groups, as indicated.

The term "ring system" encompasses monocycles, as well as fused and/or bridged polycycles.

The term "amino" signifies the primary amino group, the secondary amino group, or the tertiary amino group, as context dictates.

The term "carbonyl" signifies the -C(O)- group.

The terms "hydroxy" and "hydroxyl" signify the -OH group.

The term "oxo" means a doubly bonded oxygen, sometimes designated as =O or for example in describing a carbonyl "C(O)" may be used to show an oxo substituted carbon.

The term "oxy" signifies the -O- group.

The term "sulfonyl" signifies the -SO₂- group.

The term "substituted," when used to modify a specified group or radical, means that one or more hydrogen atoms of the specified group or radical are each, independently of one another, replaced with the same or different substituent groups as defined below, unless specified otherwise.

As used herein, the term "metabolite" means a compound that results from the metabolism of a compound, including from any of the compounds described with reference to formulae (I) and (Ia)-(If) and formulae 1-272 above. The metabolite may be an active metabolite meaning that it is a physiologically active compound. Alternatively, the metabolite may be an active compound obtained from metabolism of a prodrug as described herein.

The term "pharmaceutically acceptable" is meant as reference to a compound, substance or composition that is generally safe, non-toxic and biologically acceptable.

As used herein, the phrase "pharmaceutically acceptable salt" refers to both pharmaceutically acceptable acid and base addition salts. Without limitation, such pharmaceutically acceptable salts include salts of acids such as hydrochloric, phosphoric, hydrobromic, sulfuric, sulfinic, formic, toluenesulfonic, methanesulfonic, nitric, benzoic, citric, tartaric, maleic, hydroiodic, alkanoic such as acetic, HOOC-(CH₂)ₙ-COOH where n is 0-4, and the like. Non-toxic pharmaceutical base addition salts include, without limitation, salts of bases such as sodium, potassium, calcium, ammonium, and the like. Those skilled in the art will recognize a wide variety of non-toxic pharmaceutically acceptable addition salts.

One of ordinary skill in the art of medicinal chemistry also will appreciate that the disclosed structures are intended to include isotopically enriched forms of the present compounds. As used herein "isotopes" includes those atoms having the same atomic number but different mass numbers. As is known to those of skill in the art, certain atoms, such as hydrogen occur in different isotopic forms. For example, hydrogen includes three isotopic forms, protium, deuterium and tritium. As will be apparent to those of skill in the art upon consideration of the present compounds, certain compounds can be enriched at a given position with a particular isotope of the atom at that position. For example, compounds having a fluorine atom, may be synthesized in a form enriched in the radioactive fluorine isotope ¹⁸F. Similarly, compounds may be enriched in the heavy isotopes of hydrogen: deuterium and tritium; and similarly can be enriched in a radioactive isotope of carbon, such as ¹³C. Such isotopic variant compounds undergo different metabolic pathways and can be useful, for example, in studying the ubiquitination pathway and its role in disease. Of course, in certain embodiments, the compound has substantially the same isotopic character as naturally-occurring materials.

The compounds may contain chiral centers, which may be either of the (R) or (S) configuration, or may comprise a mixture thereof. Accordingly, the present invention also includes stereoisomers of the compounds described herein, where applicable, either individually or admixed in any proportions. Stereoisomers may include, but are not limited to, enantiomers, diastereomers, racemic mixtures (racemates), and combinations thereof. Such stereoisomers can be prepared and separated using conventional techniques, either by reacting enantiomeric starting materials, or by separating isomers of compounds.

Isomers may further include tautomers and geometric isomers. Examples of geometric isomers include, but are not limited to, cis isomers or trans isomers across a double bond. Other isomers are contemplated among the compounds disclosed herein. The isomers may be used either in pure form or in admixture with other isomers of the compounds disclosed herein.

One of ordinary skill in the art of chemistry will also appreciate that the disclosed structures, unless otherwise indicated are intended to include all possible stereoisomers of the claimed molecule, including mixtures of certain or all stereoisomers. However, compounds drawn with certain stereochemistry at one or more stereocenters are indicative of the compounds in that particular embodiment having the indicated stereochemistry. Compounds and stereocenters drawn with ambiguous stereochemistry are meant to convey any stereoisomer or mixture thereof, e.g., a racemic mixture of compounds or a purified subset of stereoisomers. In some embodiments, the compounds may have a natural THC-type stereochemistry as indicated below:

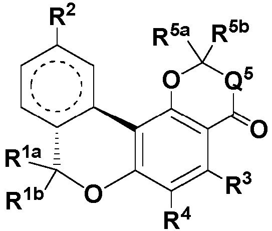

As used herein, the terms "individual," "patient," or "subject", used interchangeably, refer to any animal, including mammals, and preferably humans.

As used herein, the phrase "therapeutically effective amount" or "effective amount" refers to the amount of active compound or pharmaceutical agent that elicits the biological or medicinal response that is being sought in a cell, tissue, system, animal, individual or human by a researcher, veterinarian, medical doctor or other clinician.

An effective amount can be an amount suitable for:
(i) inhibiting the progression the disease;
(ii) prophylactic use for example, preventing or limiting development of a disease, condition or disorder in an individual who may be predisposed or otherwise at risk to the disease, condition or disorder but does not yet experience or display the pathology or symptomatology of the disease;
(iii) inhibiting the disease; for example, inhibiting a disease, condition or disorder in an individual who is experiencing or displaying the pathology or symptomatology of the disease, condition or disorder;
(iv) ameliorating the referenced disease state, for example, ameliorating a disease, condition or disorder in an individual who is experiencing or displaying the pathology or symptomatology of the disease, condition or disorder (i.e., reversing or improving the pathology and/or symptomatology) such as decreasing the severity of disease; or
(v) eliciting the referenced biological effect.

As used herein, the terms "treatment" and "treating" means (i) ameliorating the referenced disease state, condition, or disorder (or a symptom thereof), such as, for example, ameliorating a disease, condition or disorder in an individual who is experiencing or displaying the pathology or symptomatology of the disease, condition or disorder (i.e., reversing or improving the pathology and/or symptomatology) such as decreasing the severity of disease or symptom thereof, or inhibiting the progression of disease; or (ii) eliciting the referenced biological effect (e.g., inducing apoptosis, or inhibiting glutathione synthesis).

As used herein, the terms "preventing" or "prevent" means completely or partially preclude or delay the onset in a subject of a referenced disease state, condition, or disorder (or a symptom thereof).

### Methods of Preparation

Many general references providing commonly known chemical synthetic schemes and conditions useful for synthesizing the disclosed compounds are available (see, e.g., Smith and March, March's Advanced Organic Chemistry: Reactions, Mechanisms, and Structure, Fifth Edition, Wiley-Interscience, 2001; or Vogel, A Textbook of Practical Organic Chemistry, Including Qualitative Organic Analysis, Fourth Edition, New York: Longman, 1978).

Compounds as described herein can be purified by any of the means known in the art, including chromatographic means, such as HPLC, preparative thin layer chromatography, flash column chromatography and ion exchange chromatography. Any suitable stationary phase can be used, including normal and reversed phases as well as ionic resins. Most typically the disclosed compounds are purified via silica gel and/or alumina chromatography. See, e.g., Introduction to Modern Liquid Chromatography, 2nd Edition, ed. L. R. Snyder and J. J. Kirkland, John Wiley and Sons, 1979; and Thin Layer Chromatography, ed E. Stahl, Springer-Verlag, New York, 1969. In further embodiments, compounds may be purified by preparative HPLC.

During any of the processes for preparation of the subject compounds, it may be necessary and/or desirable to protect sensitive or reactive groups on any of the molecules concerned. This may be achieved by means of conventional protecting groups as described in standard works, such as J. F. W. McOmie, "Protective Groups in Organic Chemistry," Plenum Press, London and New York 1973, in T. W. Greene and P. G. M. Wuts, "Protective Groups in Organic Synthesis," Third edition, Wiley, New York 1999, in "The Peptides"; Volume 3 (editors: E. Gross and J. Meienhofer), Academic Press, London and New York 1981, in "Methoden der organischen Chemie," Houben-Weyl, 4.sup.th edition, Vol. 15/l, Georg Thieme Verlag, Stuttgart 1974, in H.-D. Jakubke and H. Jescheit, "Aminosauren, Peptide, Proteine," Verlag Chemie, Weinheim, Deerfield Beach, and Basel 1982, and/or in Jochen Lehmann, "Chemie der Kohlenhydrate: Monosaccharide and Derivate," Georg Thieme Verlag, Stuttgart 1974. The protecting groups may be removed at a convenient subsequent stage using methods known from the art.

A "leaving group" as used herein (e.g. suitable as LG) refers to a moiety of a reactant (e.g., alkylhalogenide ) that is displaced from the first reactant in the chemical reaction. A comprehensive and non-limiting list of suitable leaving groups can be found in J. March, Advanced Organic Chemistry, John Wiley and Sons, N.Y. (2013). Examples of suitable leaving groups include, but are not limited to, halogen (such as Cl or Br), acetoxy, and sulfonyloxy groups (such as methyl sulfonyloxy, trifluoromethylsulfonyloxy ("triflate"), p-toluenesulfonyloxy ("tosylate")).

The compounds disclosed herein can be made using procedures familiar to the person of ordinary skill in the art and as described herein, for example in Scheme 1, Schemes 2a-2c and/or Scheme 3a-3b. One of skill in the art can adapt the reaction sequences of schemes and examples as provided herein to fit the desired target molecule. Of course, in certain situations one of skill in the art will use different reagents to affect one or more of the individual steps or to use protected versions of certain of the substituents. Additionally, one skilled in the art would recognize that compounds disclosed herein can be synthesized using different routes altogether. For example, the person of ordinary skill in the art may adapt the procedures described herein and/or other procedures familiar to the person of ordinary skill in the art to make the compounds described herein.

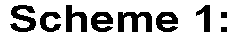

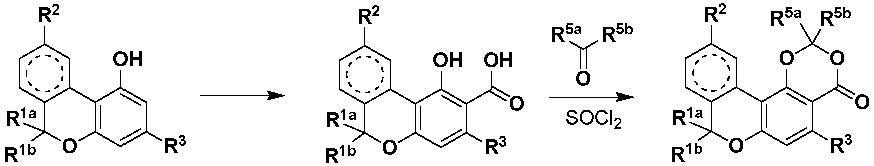

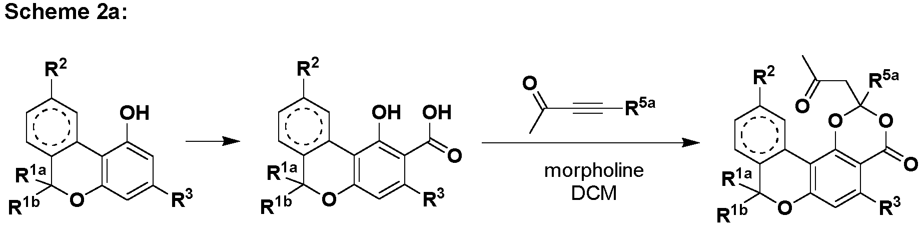

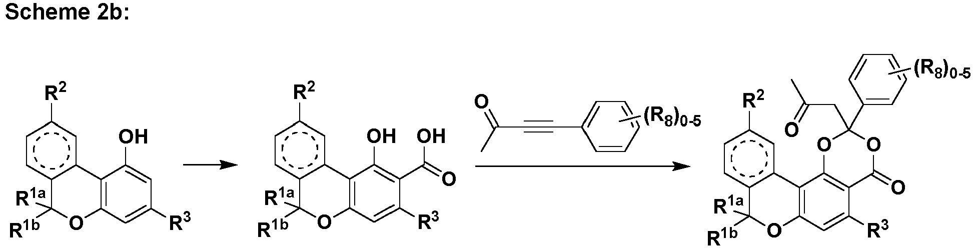

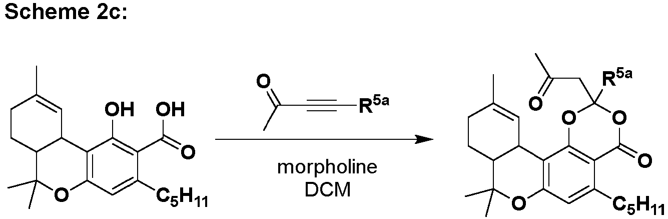

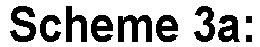

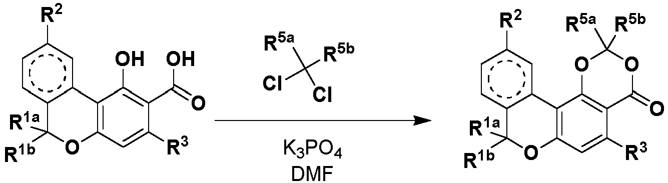

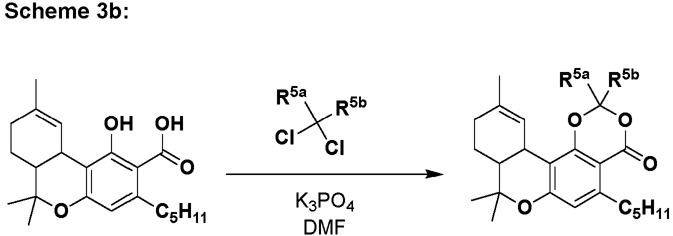

### EXAMPLES AND REFERENCE EXAMPLES

The preparation of the compounds of the invention and reference compounds also disclosed herein but not forming part of the claimed invention is illustrated further by the following examples and reference examples, which are not to be construed in any way as limiting the invention in scope to the specific procedures and compounds described in them. These examples and reference examples are offered to illustrate the invention.

### Compound Synthesis and Formulation

### Example 1: 8,8,11-trimethyl-5-pentyl-8a,9,10,12a-tetrahydro-4H,8H-benzo[c][1,3]dioxino[4,5-flchromen-4-one (1b)

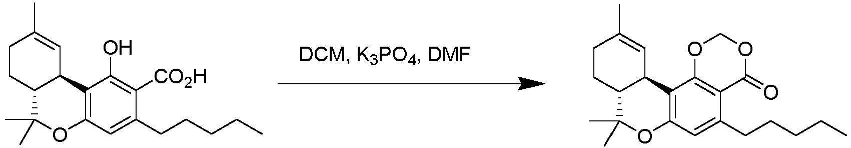

A mixture of tetrahydrocannabinolic acid (THCA; 50 mg, 0.14 mmol), K₃PO₄ (59 mg, 0.28 mmol), dichloromethane (DCM; 0.40 mL) in dimethylformamide (DMF; 1 mL) was stirred in a sealed vial at 100 °C for 1.5 h. The mixture was diluted with brine and extracted with ethyl acetate. The organic extract was dried over Na₂SO₄, concentrated, and purified by flash chromatography on silica gel (ethyl acetate-hexane) to give product (77% yield). (8aR,12aR)-8,8,11-trimethyl-5-pentyl-8a,9,10,12a-tetrahydroisochromeno[3,4-h][1,3]benzodioxin-4-one: ¹H NMR (400 MHz, CDCl₃): δ 6.46 (1H, s), 6.14-6.12 (1H, m), 5.65 (1H, d, *J =* 5.0 Hz), 5.50 (1H, d, *J= 5.0* Hz), 3.23-3.19 (1H, m), 3.04-2.89 (2H, m), 2.18-2.15 (2H, m), 1.95-1.90 (1H, m), 1.71-1.52 (7H), 1.45 (3H, s), 1.37-1.33 (4H, m), 1.11 (3H, s), 0.91-0.87 (3H, m). ESI-MS [M+H]⁺: 371.24.

### Example 2: 8,8,11-trimethyl-2-(2-oxopropyl)-5-pentyl-2-phenyl-8a,9,10,12a-tetrahydro-4H,8H-benzo[c][1,3]dioxino[4,5-f]chromen-4-one (12b)

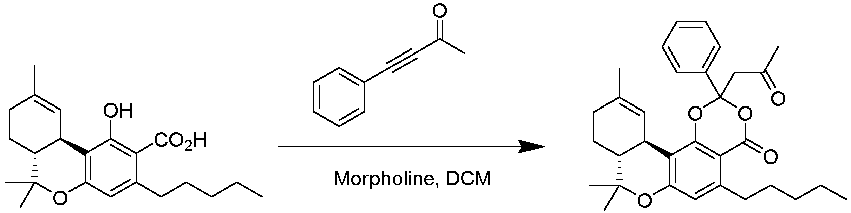

To a solution of THCA (50 mg, 0.14 mmol) and 4-phenylbut-3-yn-2-one (20 µL, 0.14 mmol) in DCM (1 mL) at room temperature was added morpholine (3 µL, 0.28 mmol). The mixture was stirred at room temperature for 24 h and concentrated. The crude mixture was was purified by flash chromatography on silica gel (ethyl acetate-hexane) to give product (29% yield). (8aR,12aR)-8,8,11-trimethyl-2-(2-oxopropyl)-5-pentyl-2-phenyl-8a,9,10,12a-tetrahydro-4H,8H-benzo[c][1,3]dioxino[4,5-f]chromen-4-one: ¹H NMR (400 MHz, CDCl₃): δ 7.63-7.61 (2H, m), 7.40-7.24 (8H, m), 6.61-6.60 (1H, m), 6.36 (1H, s), 6.32-6.31 (1H, m), 6.22 (1H, s), 3.43-3.39 (1H, m), 3.35-3.14 (4H, m), 3.13-3.06 (1H, m), 2.88-2.67 (3H, m), 2.41 (3H, s), 2.20-2.17 (4H, m), 2.04 (3H, s), 1.98-1.76 (2H, m), 1.77 (6H, s), 1.73-1.58 (5H, m), 1.51-1.37 (10H, m), 1.29-1.13 (11H, m), 1.04 (3H, m), 0.84-0.80 (6H, m). ESI-MS [M+Na]⁺: 525.44.

### Example 3: 2,8,8,11-tetramethyl-5-pentyl-8a,9,10,12a-tetrahydro-4H,8H-benzo[c][1,3]dioxino[4,5-f]chromen-4-one (16b)

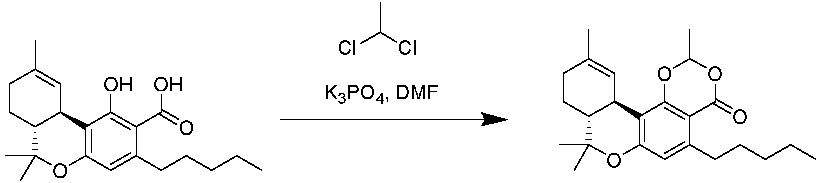

A mixture of THCA (100 mg, 0.28 mmol), K₃PO₄ (118 mg, 0.56 mmol), 1,1-dichloroethane (0.5 mL) in DMF (1.0 mL) was stirred in a sealed vial at 130 °C for 18 h. The mixture was diluted with brine and extracted with ethyl acetate. The organic extract was dried over Na₂SO₄, concentrated, and purified by flash chromatography on silica gel (ethyl acetate-hexane), followed by reverse phase C18 (CH₃CN-H₂O) to give product (23% yield). (8aR,12aR)-2,8,8,11-tetramethyl-5-pentyl-8a,9,10,12a-tetrahydro-4H,8H-benzo[c][1,3]dioxino[4,5-f]chromen-4-one ¹H NMR (400 MHz, CDCl₃): δ 6.46 (s, 1H), 6.42 (s, 1H), 6.20-6.18 (1H, m), 6.06-6.05 (1H, m), 5.73 (1H, q, *J =* 5.0 Hz), 5.56 (1H, q, *J =* 5.0 Hz), 3.27-2.72 (6H, m), 2.17-2.14 (4H, m), 1.95-1.89 (2H, m), 1.75 (3H, d, *J* = 5.0 Hz), 1.74 (3H, d, *J =* 5.0 Hz), 1.72-1.49 (14H, m), 1.44 (6H, s), 1.40-1.28 (8H, m), 1.11 (3H, s), 1.10 (3H, s), 0.90-0.87 (6H, m). ESI-MS [M+H]⁺: 384.90.

### Example 4: 8,8,11-trimethyl-2-(2-oxopropyl)-5-pentyl-8a,9,10,12a-tetrahydro-4H,8H-benzo[c][1,3]dioxino[4,5-f]chromen-4-one (14b)

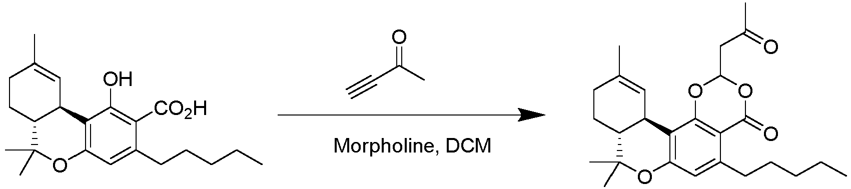

To a solution of THCA (100 mg, 0.28 mmol) and but-3-yn-2-one (22 µL, 0.28 mmol) in DCM (2 mL) at room temperature was added morpholine (5 µL, 0.056 mmol). The mixture was stirred at room temperature for 24 h and concentrated. The crude mixture was purified by flash chromatography on silica gel (ethyl acetate-hexane), followed by DCM-hexane to give product (9% yield). (8aR,12aR)-8,8,11-trimethyl-2-(2-oxopropyl)-5-pentyl-8a,9,10,12a-tetrahydro-4H,8H-benzo[c][1,3]dioxino[4,5-f]chromen-4-one: ¹H NMR (400 MHz, CDCl₃): δ 6.47 (s), 6.44 (1H, s), 6.09-6.08 (m), 6.02-6.01 (1H, m), 6.05 (dd, *J* = 7.0, 2.0 Hz), 5.85 (1H, t, *J =* 5.0 Hz), 3.31-2.72 (5H, m), 2.31 (3H, s), 2.15-2.13 (2H, m), 1.93-1.88 (1H, m), 1.70-1.50 (7H, m), 1.44 (3H, s), 1.38-1.31 (4H, m), 1.11 (s), 1.08 (3H, s), 0.90-0.87 (3H, m). ESI-MS [M+H]⁺: 426.95.

### Example 5: 8,8,11-trimethyl-5-pentyl-2-phenyl-8a,9,10,12a-tetrahydro-4H,8H-benzo[c][1,3]dioxino[4,5-f]chromen-4-one (3b)

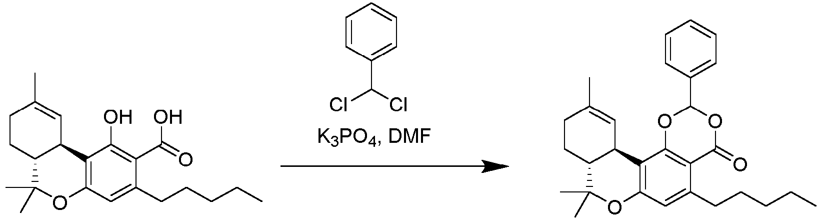

A mixture of THCA (100.00 mg, 0.28 mmol), K₃PO₄ (118 mg, 0.56 mmol) ,α,α-dichlorotoluene (0.5 mL) in DMF (1.0 mL) was stirred in a sealed vial at 120 °C for 18 h. The mixture was diluted with brine and extracted with ethyl acetate. The organic extract was dried over Na₂SO₄, concentrated and purified by flash chromatography on silica gel (ethyl acetate-hexane), followed by reverse phase C18 (CH₃CN-H₂O) to give product (26% yield). (8aR,12aR)-8,8,11-trimethyl-5-pentyl-2-phenyl-8a,9,10,12a-tetrahydro-4H,8H-benzo[c][1,3]dioxino[4,5-flchromen-4-one: ¹H NMR (400 MHz, CDCl₃): δ 7.67-7.65 (2H, m), 7.48-7.46 (3H, m), 6.52 (s), 6.50 (1H, s), 6.48 (s), 6.36 (1H, s), 5.42-5.40 (1H, br), 3.25-2.68 (4H, m), 2.17-2.13 (1H, m), 1.91-1.76 (3H, m), 1.69-1.56 (5H, m), 1.42 (s), 1.42 (3H, s), 1.40-1.36 (4H, m), 1.15 (s), 1.12 (3H, s), 0.93-0.89 (3H, m). ESI-MS [M+H]⁺: 446.79.

### Example 6: 2-ethyl-8,8,11-trimethyl-5-pentyl-8a,9,10,12a-tetrahydro-4H,8H-benzo[c][1,3]dioxino[4,5-f]chromen-4-one (17b)

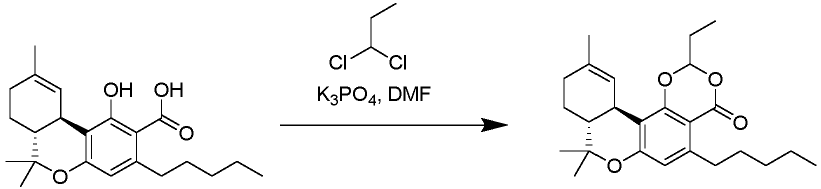

A mixture of THCA (50 mg, 0.14 mmol), K₃PO₄ (59 mg, 0.28 mmol), 1,1-dichloropropane (0.3 mL) in DMF (0.6 mL) was stirred in a sealed vial at 130 °C for 4 h. The mixture was diluted with brine and extracted with ethyl acetate. The organic extract was dried over Na₂SO₄, concentrated and purified by flash chromatography on silica gel (ethyl acetate-hexane), followed by reverse phase C18 (CH₃CN-H₂O) to give product (7% yield). (8aR,12aR)-2-ethyl-8,8,11-trimethyl-5-pentyl-8a,9,10,12a-tetrahydro-4H,8H-benzo[c][1,3]dioxino[4,5-f]chromen-4-one: ¹H NMR (400 MHz, CDCl₃): δ 6.46 (s), 6.42 (1H, s), 6.22-6.21 (m), 6.10-6.09 (1H, m), 5.55 (t, *J =* 5.0 Hz), 5.40 (1H, t, *J =* 5.0 Hz), 3.28-2.72 (3H, m), 2.18-2.16 (2H, m), 2.10-2.03 (2H, m), 1.95-1.89 (3H, m), 1.68-1.50 (5H, m), 1.45 (3H, s), 1.40-1.29 (4H, m), 1.21-1.10 (6H, m), 0.90-0.86 (3H, m). ESI-MS [M+H]⁺: 398.91.

### Reference Example 7: 2,2,8,8,11-pentamethyl-5-pentyl-8a,9,10,12a-tetrahydro-4H,8H-benzo[c][1,3]dioxino[4,5-f]chromen-4-one (Reference Compound 2b)

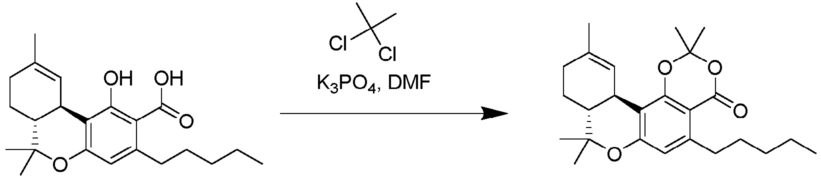

A mixture of THCA (100 mg, 0.28 mmol), K₃PO₄ (178 mg, 0.84 mmol), 2,2-dichloropropane (0.30 mL) in DMF (0.6 mL) was stirred in a sealed vial at 120 °C for 18 h. The mixture was diluted with brine and extracted with ethyl acetate. The organic extract was dried over Na₂SO₄, concentrated and purified by reverse phase C18 (CH₃CN-H₂O) to give product. (8aR,12aR)-2,2,8,8,11-pentamethyl-5-pentyl-8a,9,10,12a-tetrahydro-4H,8H-benzo[c][1,3]dioxino[4,5-f]chromen-4-one (1% yield): ¹H NMR (400 MHz, CDCl₃): δ 6.39 (1H, s), 6.14 (1H, s), 3.20-3.17 (1H, m), 3.12-3.05 (1H, m), 2.90-2.83 (1H, m), 2.17-2.15 (2H, m), 1.94-1.90 (1H, m), 1.77 (3H, s), 1.70 (3H, s), 1.68 (3H, s), 1.58-1.55 (4H, m), 1.44 (3H, s), 1.43-1.30 (4H, m), 1.10 (3H, s), 0.88 (3H, t, *J* = 7.0 Hz). ESI-MS [M+H]⁺: 398.99.

### Example 8: 2-(4-chlorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one (23b)

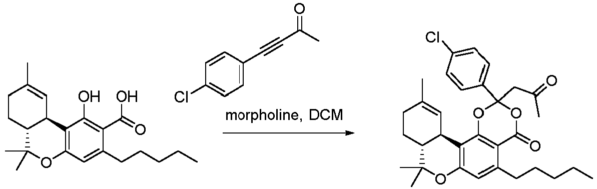

To a solution of THCA (50 mg, 0.14 mmol) and 4-(4-chlorophenyl)but-3-yn-2-one (20 µL, 0.17mmol) in DCM (1 mL) at room temperature was added morpholine (2.5 µL, 0.28 mmol). The mixture was stirred at room temperature for 24 h and concentrated. The crude mixture was purified by reverse phase C18 (CH₃CN-H₂O) to give product (2% yield). (8aR,12aR)-2-(4-chlorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one: ¹H NMR (400 MHz, CDCl₃): δ 7.55 (2H, d, *J* = 8.5 Hz), 7.33 (2H, d, *J =* 8.5 Hz), 6.57 (1H, s), 6.37 (1H, s), 3.41-3.23 (3H, m), 3.11-3.04 (1H, m), 2.73-2.66 (1H, m), 2.19-2.18 (2H, m), 2.08 (3H, s), 1.95-1.90 (1H, m), 1.77 (3H, s), 1.77-1.70 (1H, m), 1.47-1.38 (6H, m), 1.25-1.17 (4H, m), 1.14 (3H, s), 0.83 (3H, t, *J =* 7.0 Hz). ESI-MS [M+Na]⁺: 559.48.

### Example 9: 2-(4-methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-one (21b)

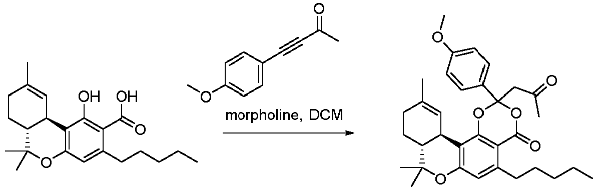

To a solution of THCA (50 mg, 0.14 mmol) and 4-(4-methoxyphenyl)but-3-yn-2-one (20 µL, 0.18 mmol) in DCM (1 mL) at room temperature was added morpholine (2.5 µL, 0.28 mmol). The mixture was stirred at room temperature for 24 h and concentrated. The crude mixture was purified by reverse phase C18 (CH₃CN-H₂O) to give product (2% yield). (8aR,12aR)-2-(4-methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-one: ¹H NMR (400 MHz, CDCl₃): δ 7.54-7.50 (2H, m), 6.88-6.84 (2H, m), 6.59 (1H, s), 6.35 (1H, s), 3.78 (3H, s), 3.40-3.25 (3H, m), 3.13-3.06 (1H, m), 2.74-2.67 (1H, m), 2.19-2.17 (2H, m), 2.06 (3H, s), 1.95-1.90 (1H, m), 1.77 (3H, s), 1.75-1.69 (1H, m), 1.47-1.38 (6H, m), 1.27-1.16 (4H, m), 1.15 (3H, s), 0.82 (3H, t, *J* = 7.0 Hz). ESI-MS [M+Na]⁺: 555.45.

### Example 10: 2-(4-fluorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one (13b)

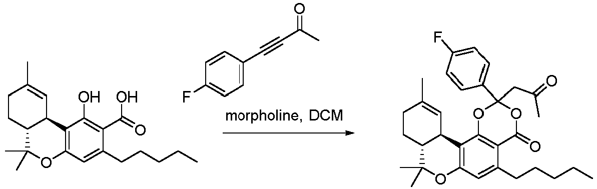

To a solution of THCA (100 mg, 0.28 mmol) and 4-(4-fluorophenyl)but-3-yn-2-one (54 mg, 0.33 mmol) in DCM (1 mL) at rt was added morpholine (5 µL, 0.56 mmol). The mixture was stirred at rt for 24 h and concentrated. The crude mixture was purified by reverse phase C18 (CH₃CN-H₂O) to give product (10% yield). (8aR,12aR)-2-(4-fluorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one: ¹H NMR (400 MHz, CDCl₃) δ 7.63-7.58 (2H, m), 7.07-6.94 (2H, m), 6.57 (1H, br), 6.37 (1H, s), 3.41-3.24 (3H, m), 3.12-3.05 (1H, m), 2.74-2.67 (1H, m), 2.19-2.18 (2H, m), 2.07 (3H, s), 1.96-1.91 (1H, m), 1.76 (3H, s), 1.76-1.70 (1H, m), 1.52-1.37 (6H, m), 1.29-1.17 (4H, m), 1.15 (3H, s), 0.83 (3H, t, *J =* 7.0 Hz). ESI-MS [M+Na]⁺: 543.42.

### Example 11

The following compounds of the invention and reference compounds disclosed herein but not forming part of the claimed invention may be prepared according to the procedures known to those of skill in the art in view of Scheme 1, Schemes 2a-2c and/or Scheme 3a-3b, Examples 1-6 and 8-10 and Reference Example 7:

| **Comp. No.** | **Name** | **Structure** |
|---|---|---|
| **1a** | 8,8,11-trimethyl-5-pentyl-4H,8H-benzo[c][1,3]dioxino[4,5-f]chromen-4-one | 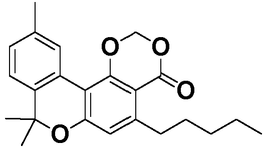 |
| **Reference Compound 2a** | 2,2,8,8,11-pentamethyl-5-pentyl-4H,8H-benzo[c][1,3]dioxino[4,5-f]chromen-4-one | 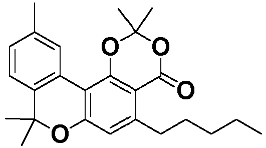 |
| **3a** | 8,8,11-trimethyl-5-pentyl-2-phenyl-4H,8H-benzo[c][1,3]dioxino[4,5-f]chromen-4-one | 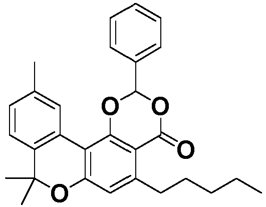 |
| **4a** | 2,8,8,11-tetramethyl-5-pentyl-2-(4-(trifluoromethyl)phenyl)-4H,8H-benzo[c][1,3]dioxino[4,5-f]chromen-4-one | 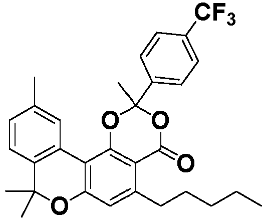 |
| **5a** | 2-acetyl-2,8,8,11-tetramethyl-5-pentyl-4H,8H-benzo[c][1,3]dioxino[4,5-f]chromen-4-one | 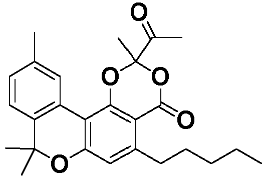 |
| **6a** | ethyl 2,8,8,11-tetramethyl-4-oxo-5-pentyl-4H,8H-benzo[c][1,3]dioxino[4,5-f]chromene-2-carboxylate | 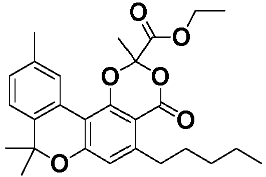 |
| **7a** | 2,8,8,11-tetramethyl-2-(2-oxopropyl)-5-pentyl-4H,8H-benzo[c][1,3]dioxino[4,5-f]chromen-4-one | 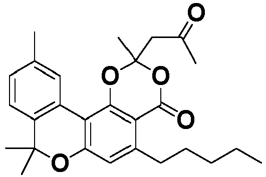 |
| **8a** | 2,8,8,11-tetramethyl-2-(2-methylprop-1-en-1-yl)-5-pentyl-4H,8H-benzo[c][1,3]dioxino[4,5-f]chromen-4-one | 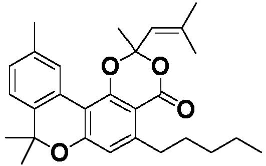 |
| **9a** | 8,8,11-trimethyl-5-pentyl-4H,8H-spiro[benzo[c][1,3]dioxino[4,5 -f]chromene-2,1'-cyclopentan]-4-one | 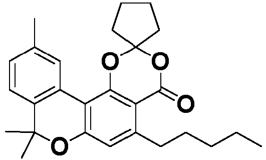 |
| **Reference Compound 10a** | 2-(3-chloropropyl)-2,8,8,11-tetramethyl-5-pentyl-4H, 8H-benzo[c][1,3]dioxino[4,5-f]chromen-4-one | 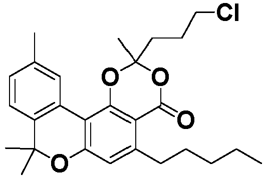 |
| **11a** | 2-(methoxymethyl)-2,8,8,11-tetramethyl-5-pentyl-4H,8H-benzo[c][1,3]dioxino[4,5-f]chromen-4-one | 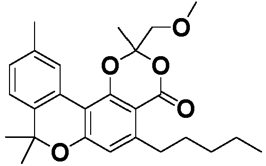 |
| **12a** | 8,8,11-trimethyl-2-(2-oxopropyl)-5-pentyl-2-phenyl-4H,8H-benzo[c][1,3]dioxino[4,5-f]chromen-4-one | 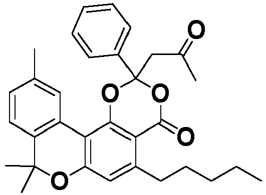 |
| **13a** | 2-(4-fluorophenyl)-8,8,11-trimethyl-2-(2-oxopropyl)-5-pentyl-4H,8H-benzo[c][1,3]dioxino[4,5-f]chromen-4-one | 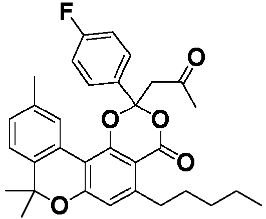 |
| **14a** | 8,8,11-trimethyl-2-(2-oxopropyl)-5-pentyl-4H,8H-benzo[c][1,3]dioxino[4,5-f]chromen-4-one | 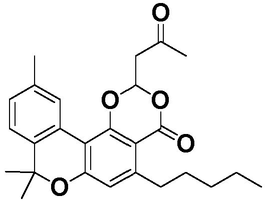 |
| **15a** | 2-butyl-8,8,11-trimethyl-2-(2-oxopropyl)-5-pentyl-4H,8H-benzo[c][1,3]dioxino[4,5-f]chromen-4-one | 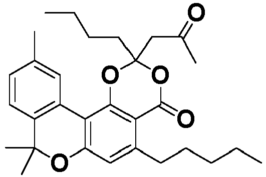 |
| **16a** | 2,8,8,11-tetramethyl-5-pentyl-4H,8H-benzo[c][1,3]dioxino[4,5-f]chromen-4-one | 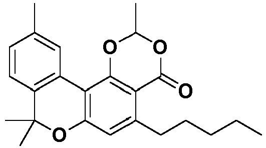 |
| **17a** | 2-ethyl-8,8,11-trimethyl-5-pentyl-4H,8H-benzo[c][1,3]dioxino[4,5-f]chromen-4-one | 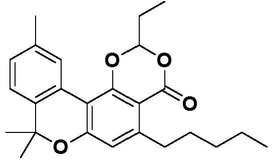 |
| **18a** | 8,8,11-trimethyl-5-pentyl-2,2-diphenyl-4H,8H-benzo[c][1,3]dioxino[4,5-f]chromen-4-one | 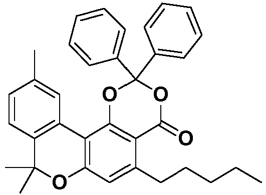 |
| **19a** | 8,8,11-trimethyl-2-(2-oxopropyl)-5-pentyl-2-(p-tolyl)-4H, 8H-benzo[c][1,3]dioxino[4,5-f]chromen-4-one | 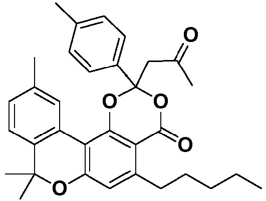 |
| **20a** | 8,8,11-trimethyl-2-(4-nitrophenyl)-2-(2-oxopropyl)-5-pentyl-4H,8H-benzo[c][1,3]dioxino[4,5-f]chromen-4-one | 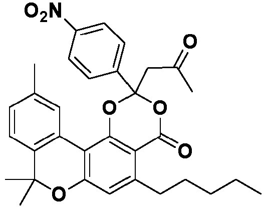 |
| **21a** | 2-(4-methoxyphenyl)-8,8,11-trimethyl-2-(2-oxopropyl)-5-pentyl-4H,8H-benzo[c][1,3]dioxino[4,5-f]chromen-4-one | 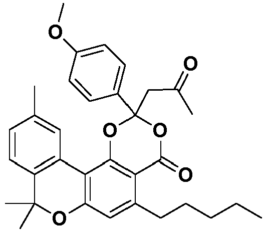 |
| **22a** | methyl 4-(8,8,11-trimethyl-4-oxo-2-(2-oxopropyl)-5-pentyl-4H,8H-benzo[c][1,3]dioxino[4,5-f]chromen-2-yl)benzoate | 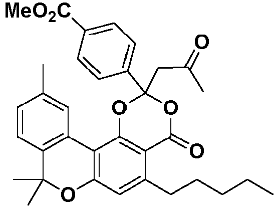 |
| **23a** | 2-(4-chlorophenyl)-8,8,11-trimethyl-2-(2-oxopropyl)-5-pentyl-4H,8H-benzo[c][1,3]dioxino[4,5-f]chromen-4-one | 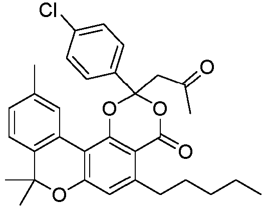 |
| **4b** | 2,8,8,11-tetramethyl-5-pentyl-2-(4-(trifluoromethyl)phenyl)-8a,9,10,12a-tetrahydro-4H,8H-benzo[c][1,3]dioxino[4,5-f]chromen-4-one | 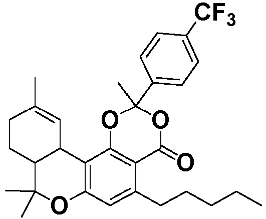 |
| **5b** | 2-acetyl-2,8,8,11-tetramethyl-5-pentyl-8a,9,10,12a-tetrahydro-4H,8H-benzo[c][1,3]dioxino[4,5-f]chromen-4-one | 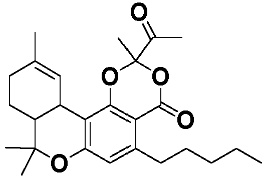 |
| **6b** | ethyl 2,8,8,11-tetramethyl-4-oxo-5-pentyl-8a,9,10,12a-tetrahydro-4H,8H-benzo[c][1,3]dioxino[4,5-f]chromene-2-carboxylate | 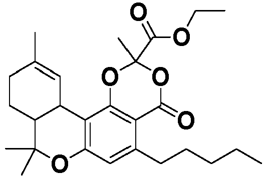 |
| **7b** | 2,8,8,11-tetramethyl-2-(2-oxopropyl)-5-pentyl-8a,9,10,12a-tetrahydro-4H,8H-benzo[c][1,3]dioxino[4,5-f]chromen-4-one | 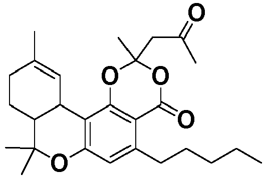 |
| **8b** | 2,8,8,11-tetramethyl-2-(2-methylprop-1-en-1-yl)-5-pentyl-8a,9,10,12a-tetrahydro-4H,8H-benzo[c][1,3]dioxino[4,5-f]chromen-4-one | 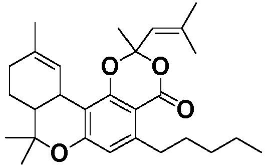 |
| **9b** | 8,8,11-trimethyl-5-pentyl-8a,9,10,12a-tetrahydro-4H,8H-spiro[benzo[c][1,3]dioxino[4,5 -f]chromene-2,1'-cyclopentan]-4-one | 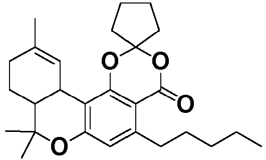 |
| **Reference Compound 10b** | 2-(3-chloropropyl)-2,8,8,11-tetramethyl-5-pentyl-8a,9,10,12a-tetrahydro-4H,8H-benzo[c][1,3]dioxino[4,5-f]chromen-4-one | 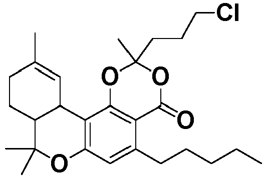 |
| **11b** | 2-(methoxymethyl)-2,8,8,11-tetramethyl-5-pentyl-8a,9,10,12a-tetrahydro-4H,8H-benzo[c][1,3]dioxino[4,5-f]chromen-4-one | 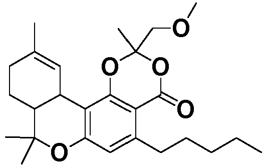 |
| **15b** | 2-butyl-8,8,11-trimethyl-2-(2-oxopropyl)-5-pentyl-8a,9,10,12a-tetrahydro-4H,8H-benzo[c][1,3]dioxino[4,5-f]chromen-4-one | 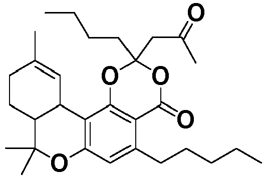 |
| **18b** | 8,8,11-trimethyl-5-pentyl-2,2-diphenyl-8a, 9,10, 12a-tetrahydro-4H,8H-benzo[c][1,3]dioxino[4,5-f]chromen-4-one | 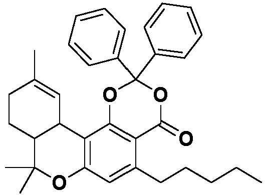 |
| **19b** | 8,8,11-trimethyl-2-(2-oxopropyl)-5-pentyl-2-(p-tolyl)-8a,9,10,12a-tetrahydro-4H,8H-benzo[c][1,3]dioxino[4,5-f]chromen-4-one | 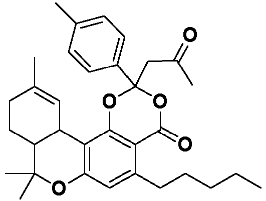 |
| **20b** | 8,8,11-trimethyl-2-(4-nitrophenyl)-2-(2-oxopropyl)-5-pentyl-8a,9,10,12a-tetrahydro-4H,8H-benzo[c][1,3]dioxino[4,5-f]chromen-4-one | 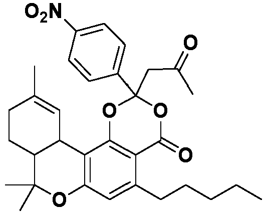 |
| **22b** | methyl 4-(8,8,11-trimethyl-4-oxo-2-(2-oxopropyl)-5-pentyl-8a,9,10,12a-tetrahydro-4H,8H-benzo[c][1,3]dioxino[4,5-f]chromen-2-yl)benzoate | 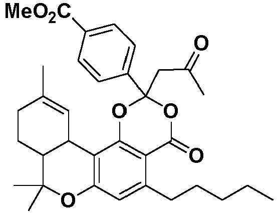 |

### Radioligand Competitive Binding Assays - CB1 and CB2

### Example 12:

Compounds of the present invention and reference compound disclosed herein but not forming part of the claimed invention were tested for CB1 receptor binding affinity and for CB2 receptor binding affinity. As used herein, "binding affinity" is represented by the IC₅₀ value, which was experimentally determined as described herein. The lower the IC₅₀ value the higher the binding affinity. A compound may be said to have "binding selectivity" if it has a higher binding affinity for one receptor compared to the other. For example, a compound that has an IC₅₀ of 1 µM for CB1 and 0.1 µM for CB2 is 10 times more selective for the CB2 receptor.

### CB1 Membrane Preparation

The membranes were prepared from CHO-K1 (Chinese hamster ovary) cells stably transfected with the human CB1 receptor (Cat.# ES-110-C; Perkin Elmer, Boston, MA). The cells were grown adherently and maintained in Ham's F12 medium containing 10% fetal bovine serum (FBS), penicillin, streptomycin and geneticin (G418) at 37°C in a humid atmosphere of 5% CO₂ following the manufacturer's instructions.

For membrane preparation the cells were washed with PBS and scraped off the plates in cold homogenization buffer (25 mM HEPES (pH 7.4), 2 mM EDTA) containing protease inhibitor cocktail (Sigma Cat.# P8340). The cell suspension was homogenized with a Pro-PK-01200D Polytron Homogenizer (Pro Scientific) and then centrifuged for 30 min at 120,000 × g. The supernatant was discarded and the pellet was re-suspended in the homogenization buffer and stored at -80°C until the time of use.

### CB1 Radioligand Binding Assay:

CP 55,940 is a synthetic cannabinoid that mimics the effects of naturally occurring THC. It acts as a full agonist at both cannabinoid CB1 and CB2 receptors. Radiolabeled ligands represent one of the most sensitive methods for probing receptor binding biology. In this experiment, 3H radiolabeled CP 55,940, i.e. [3H]CP 55,940 (Perkin Elmer, Boston, MA), was used as a radioligand for the CB1 receptor.

[3H]CP 55,940 displacement assays were used for the determination of the binding affinity of compounds for the CB1 receptor (Scheme 4; below).

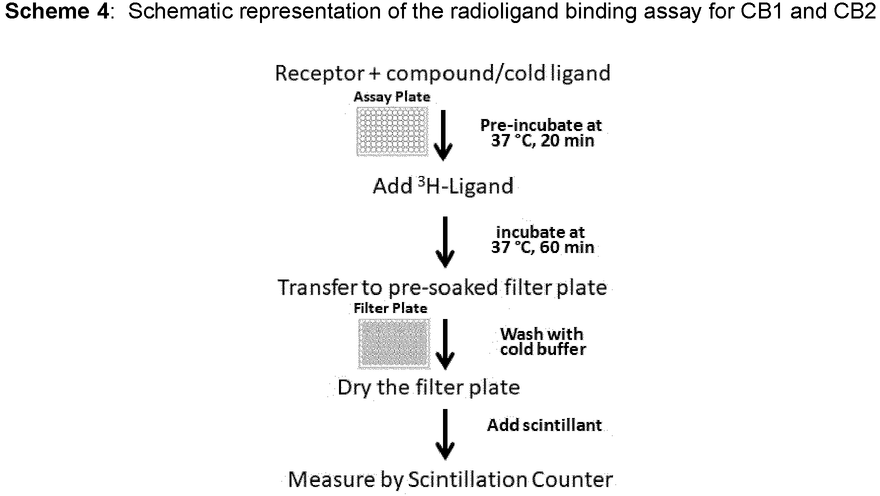

The competition binding experiments (single dose and dose-response) were performed by incubating 0.8 nM of [3H]CP 55,940 (specific activity 101 Ci/mmol, Perkin Elmer) and different concentrations of compounds disclosed herein with membranes prepared as above from CHO-K1 cells expressing human CB1 receptor (6 µg of protein/well), 50 mM Tris-HCl (pH 7.4), 5 mM MgCl₂, 1 mM CaCl₂, and 2 mg/mL BSA.

For the single dose experiments, the compound stocks (usually 10 mM in DMSO) were diluted to working stocks in the binding buffer to give the final compound concentrations of 10 µM and 1 µM. For the dose-response experiments, the test compounds were sequentially diluted to provide the desired concentration range for the IC₅₀ determination. In each case the compounds were pre-incubated with the membrane for 20 min, before adding the radioligand.

After incubation with the radioligand for 60 min at 37°C, the incubation was terminated by rapid filtration of the assay mixture through MultiScreen^{®}_{HTS}⁺ 96-well filter plates (Millipore, Cat.# MSFCNXB), pre-soaked for 60 min with 50 mM Tris-HCl (pH 7.4) containing 0.33% polyethylenimine (PEl). The nonspecific binding (NSB) was determined in the presence of 10 µM unlabelled CP 55,940. After drying the filter plate at 50°C for at least 60 min, the filter-bound radioactivity was determined by scintillation spectrometry using the 1450 MicroBeta Plate Counter (Perkin Elmer, Boston, MA). From the dose-response experiments, the IC₅₀ values were determined (see **Table 1,** CB1 IC₅₀).

Some compounds exhibited selectivity for the CB1 receptor over the CB2 receptor (see e.g. **Table 1).** In **Table 1** below, the fold difference in selectivity between CB1 and CB2 binding is indicated, with the fold difference presented in the column of the receptor that exhibited the higher binding affinity (i.e. CB1 or CB2). Where the test compound had about the same affinity for both receptors, this is represented by a value of 1.00 in the column for both receptors.

### CB2 Membrane Preparation

The membranes were prepared from CHO-K1 (Chinese hamster ovary) cells stably transfected with the human CB2 receptor (Cat.# ES-111-C; Perkin Elmer, Boston, MA). The cells were grown adherently and maintained in Ham's F12 medium containing 10% fetal bovine serum (FBS), penicillin, streptomycin and geneticin (G418) at 37°C in a humid atmosphere of 5% CO₂ following the manufacturer's instructions.

For membrane preparation the cells were washed with PBS and scraped off the plates in cold homogenization buffer (25 mM HEPES (pH 7.4), 2 mM EDTA) containing protease inhibitor cocktail (Sigma Cat.# P8340). The cell suspension was homogenized with a Pro-PK-01200D Polytron Homogenizer (Pro Scientific) and then centrifuged for 30 min at 120,000 × g. The supernatant was discarded and the pellet was re-suspended in the homogenization buffer and stored at -80°C until the time of use.

### CB2 Radioligand Binding Assay

In this experiment, 3H radiolabeled CP 55,940, i.e. [3H]CP 55,940 (Perkin Elmer, Boston, MA), was used as a radioligand for the CB2 receptor to determine the binding affinity of compounds for the CB2 receptor (Scheme 4; above).

The competition binding experiments (single dose and dose-response) were performed by incubating 0.6 nM of [3H]CP55,940 (specific activity 101 Ci/mmol, Perkin Elmer) and different concentrations of compounds disclosed herein with membranes prepared as above from CHO-K1 cells expressing human CB2 receptor (1 µg of protein/well), 50 mM Tris-HCl (pH 7.4), 5 mM MgCl₂, 1 mM CaCl₂, and 2 mg/mL BSA.

For the single dose experiments, the compound stocks (usually 10 mM in DMSO) were diluted to working stocks in the binding buffer to give the final compound concentrations of 10 µM and 1 µM. For the dose-response experiments the test compounds were sequentially diluted to provide the desired concentration range for the IC50 determination. In each case the compounds were pre-incubated with the membrane for 20 min, before adding the radioligand.

After incubation with the radioligand for 60 min at 37°C, the incubation was terminated by rapid filtration of assay mixture through MultiScreen^{®}_{HTS}⁺ 96-well filter plates (Millipore, Cat.# MSFCNXB), pre-soaked for 60 min with 50 mM Tris-HCl (pH 7.4) containing 0.33 % polyethylenimine (PEl). The nonspecific binding (NSB) was determined in the presence of 10 µM unlabelled CP55,940. After drying the filter plate at 50°C for at least 60 min, the filter-bound radioactivity was determined by scintillation spectrometry using the 1450 MicroBeta Plate Counter (Perkin Elmer, Boston, MA). From the dose-response experiments, the IC₅₀ values were determined (see **Table 1,** CB2 IC₅₀).

Some compounds exhibited selectivity for the CB2 receptor over the CB1 receptor (see e.g. **Table 1).** Again, in **Table 1** below, the fold difference in selectivity between CB1 and CB2 binding is indicated, with the fold difference presented in the column of the receptor that exhibited the higher binding affinity (i.e. CB1 or CB2). Where the test compound had about the same affinity for both receptors, this is represented by a value of 1.00 in the column for both receptors.

**Table 1: Data from Radioligand Competitive Binding Assays**

| **Compound** | | | **Selectivity** | |
|---|---|---|---|---|
| | **CB1 IC₅₀** | **CB2 IC₅₀** | **CB1** | **CB2** |
| 1b | B | A | - | 1.80 |
| 12b | D | D | - | 1.33 |
| 16b | A | A | 1.50 | - |
| 14b | C | C | 2.69 | - |
| 3b | C | C | 1.62 | - |
| 17b | C | A | - | 12.73 |
| Reference Compound 2b | A | A | - | 1.03 |
| 23b | C | C | 1.00 | 1.00 |
| 21b | C | C | 1.00 | 1.00 |
| 13b | C | C | 1.00 | 1.00 |

| | | | | |
|---|---|---|---|---|
| A = <0.50 µM B = 0.50 µM to 0.99 µM C = 1.00 µM to 4.99 µM D = ≥5.00 µM | | | | |

### Pathhunter^{®} β-Arrestin Functional Assay

### Example 13:

In the PathHunter^{®} β-Arrestin system from DiscoveRx (Eurofins, Fremont, CA), a small peptide, the ProLinkTM (PK), is fused to the intracellular sequence of a GPCR target, and a complementing peptide, the enzyme acceptor (EA) fragment, is fused to β-arrestin. After binding to its specific ligand, the GPCR target recruits β-arrestin, forcing the complementation of the two β-galactosidase enzyme fragments (EA and PK) to produce a functional β-galactosidase enzyme. The enzyme activity, and thus the amount of ligand bound to the GPCR, is detected with a single addition of a reagent cocktail to lyse the cells and produce a chemiluminescent signal before being analyzed using a traditional plate reader.

### CB1 Receptor Response to Ligand using DiscovRx Cells

PathHunter^{®} CHO-K1 CNR1 β-Arrestin cells (DiscoverRx, cat. Number 93-0959C2) stably expressing CB1 receptor (CB1R) were grown in Ham's F12 medium containing 10% fetal bovine serum (FBS), 1% penicillin/streptomycin, 300 µg/mL Hygromycin B and 800 µg/mL geneticin (G418) and maintained in 37°C incubators at 5% CO₂.

For CB1R dose-response assays, cells were plated in 96-well white flat bottom plates at a density of 40,000 cells/well and incubated overnight at 37°C in 5 % CO₂. The following day, the compound stocks (10 mM in DMSO) were sequentially diluted (11 point 3-fold dilutions) in DMSO to provide the desired concentration range for the EC₅₀ (agonist mode) or IC₅₀ (antagonist mode) determination. In agonist mode, CP 55,940 (CAS number 83002-04-4) was used, whereas in antagonist mode the synthetic cannabinoid rimonabant (CAS number 158681-13-1) was used as a reference compound.

Media was aspirated from the plate wells and cells washed with PBS, followed by adding 49.5 µL (agonist mode) or 49 µL (antagonist mode) of PBS to each well and 0.5 µL of test compound or CP55,940. Cells were then incubated for 90 minutes at 37°C in the agonist mode. Alternatively, in the antagonist mode cells were incubated with test compound or rimonabant for 30 minutes (37°C), followed by an additional incubation with 0.5 µL of 2-AG (CAS number 53847-30-6) at an EC₈₀ concentration (determined as 4 µM final concentration) for 90 min (37°C).

The incubation was terminated by adding the detection reagent (19 parts of 1% CHAPS, 5 parts Emerald-II and 1 part Galacton-Star), equivalent to 50% of the assay volume to each wells. Plates were incubated for 1 hour in the dark at room temperature, followed by chemiluminescence detection using a microplate reader.

Data were analysed by generating nonlinear regression curves. The response of agonists was normalized to the effect of CP55,940 reference agonist and the response of antagonists was normalized to the effect of rimonabant.

Compounds were tested in agonist and antagonist mode. For example, in agonist mode compound 16b was found to exhibit an EC₅₀ towards the human CB1 receptor of about 252 nM with a relative efficacy in comparison to CP55,940 of about 7%, and in antagonist mode compound 16b was found to exhibit an IC₅₀ towards human CB1 receptor of about 3.0 µM with a relative inhibition in comparison to rimonabant of about 35%.

### Further Biological Examples

### Example 14: Simulated Gastric Fluid (SGF) Assay

In a microcentrifuge tube, 626.5 µL of a solution containing 1.1x assay buffer (37.6 mM NaCl, pH 1.2-1.5) is diluted with 70 µL of a 10x pepsin solution (Sigma-Aldrich Co. Cat#: P7012, 80,000 U/mL in milliQ water). The resulting solution is incubated at 37°C and 1,200 rpm in an orbital mixer for 5 minutes, prior to the addition of 3.5 µL of the test compound (2 mM, DMSO). The sample is incubated in the same conditions for as long as required.

At the specified time points, 150 µL aliquots of the sample are transferred to microcentrifuge tubes containing 24 µL of acid quenching solution (0.5 M NaHCO₃). After vortexing the tube for 5 seconds, 348 µL of the protein precipitation solution containing an internal standard (25 µM Glyburide, ACN) are added. The tube is vortexed again for 20 seconds, and stored in ice.

Finally, the tubes are centrifuged at 5,000 x g and 4°C for 15 minutes. The percentage remaining of the test compound, compared to time zero, is quantified in the supernatant by HPLC / LC-MS or LC-MS/MS and the half-life is determined.

### Example 15: TRPs activation: measurement of cation flux through intracellular calcium detection

The transient receptor potential ion channels (TRPs) are non-selective ligand-gated cation channels that integrate a variety of physical and chemical stimuli. When activated, these channels lead to the gating of cations, including Ca²⁺, thus generating changes in intracellular calcium concentration. The single wavelength fluorescent indicator Fluo-4 acetoxymethyl (AM) is used to measure intracellular calcium flux and concentration in cells expressing TRPs and stimulated with cannabinoids. The Fluo-4 Direct^{™} calcium assay kit (Molecular Probes, Invitrogen, Carlsbad, CA, USA) allows the direct addition of the reagent into microplate wells containing cultured cells, without the requirement of media removal or a wash step, therefore facilitating the process of target screening.

As adapted from Moriello et al. ("Assay of TRPV1 Receptor Signaling" Methods In Molecular Biology (2016) 1412:65-76), human embryonic kidney (HEK-293) cells are used to express different TRPs, including TRPV1 and TRPV2. Cells are grown in Eagle's Minimum Essential Medium supplemented with 10% fetal bovine serum (FBS), 1% penicillin and streptomycin(pen/strep), and maintained in incubators at 37 °C in 5% CO₂. Cells are seeded in 96-well plates and polyethylenimine (PEI) used to transiently transfect HEK-293 cells with expression vectors containing the open reading frame (ORF) of the TRPs of interest. Transfection using the empty expression vector is performed as a negative control.

24-48 hours after transfection, cells are treated with Fluo-4 Direct^{™} (Molecular Probes) for 30-60 minutes and subsequently exposed to cannabinoids and benchmark compounds for different periods of time. Following incubations, fluorescence is measured using a microplate reader (excitation at 494 nm and emission at 516 nm).

Analysis of data is done by generating nonlinear regression curves and all data points corrected for background fluorescence and negative control. The response of agonists is normalized to the effect of a reference agonist and the response of antagonists normalized to the EC₈₀ of a reference agonist.

### Example 16: PPARy activation: nuclear hormone receptor activation assay

Peroxisome proliferator activated receptors (PPARs) are ligand-activated transcription factors of nuclear hormone receptors (NHRs). The PathHunter^{®} PPARγ protein interaction assay (DiscoverX, Fremont, CA, USA) reports the activation of NHRs based on enzyme complementation of β-galactosidase, rendering a chemiluminescent signal.

CHO-K1 PPARy cell lines (DiscoverX) stably expressing the target receptor is used. Cells are grown using reagents provided by the manufacturer (DiscoverX) and maintained in incubators at 37 °C in 5% CO₂. Cells are harvested and plated in black skirt, clear bottom 96-well plates and allowed to attach and recover overnight. Subsequently, cells are incubated with cannabinoids and/or benchmark compounds, such as troglitazone and rosiglitazone, for 30-90 minutes at 37 °C in 5 % CO₂. The detection reagent provided by the manufacturer (DiscoverX) is then added to the wells, and plates are incubated for 1 hour in the dark, followed by chemiluminescence detection using a microplate reader. Analysis of data is done by generating nonlinear regression curves and all data points are corrected for background luminescence and negative control. The response of agonists is normalized to the effect of a reference agonist and the response of antagonists normalized to the EC₈₀ of a reference agonist. Basal activity of the cells is set at 0 %.

## Claims

1. A compound having structural formula: 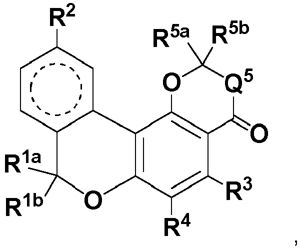 or an enantiomer, diastereomer, racemate, or tautomer thereof, or a pharmaceutically acceptable salt, solvate or hydrate of the compound, enantiomer, diastereomer, racemate, or tautomer, wherein the 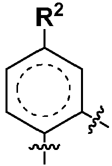 moiety is 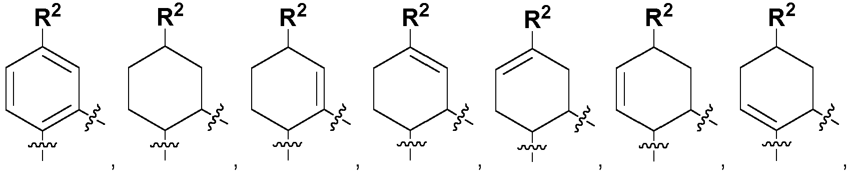 , or 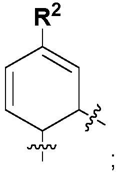
R^{1a} and R^{1b} are independently
hydrogen, C₁-C₈ alkyl, C₂-C₈ alkenyl, C₂-C₈ alkynyl, hydroxy, halo, -O(C₁-C₄ alkyl), -C(O)(C₁-C₄ alkyl), -CO₂H, -CO₂(C₁-C₄ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{1c}R^{1d}, wherein
R^{1c} and R^{1d} are independently hydrogen, C₁-C₄ alkyl, or -C(O)(C₁-C₄ alkyl),
or R^{1a} and R^{1b} together with a carbon atom to which they are attached form a cycloalkyl or heterocycloalkyl ring;
R² is hydrogen, C₁-C₈ alkyl, hydroxy, -CO₂H, -CO₂(C₁-C₈ alkyl), or oxo when attached to a ring of appropriate saturation;
R³ is hydrogen, C₁-C₁₂ alkyl, C₂-C₁₂ alkenyl, C₂-C₁₂ alkynyl, -(OCH₂CH₂)₀₋₆O(C₁-C₈ alkyl), -(C₀-C₄ alkyl)-NR^{3a}R^{3b}, -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl, wherein R^{3a} and R^{3b} are each independently hydrogen or C₁-C₆ alkyl;
R⁴ is hydrogen, -CO₂H or -CO₂(C₁-C₆ alkyl); and
R^{5a} is:
hydrogen, C₁-C₈ alkyl, C₂-C₈ alkenyl, C₂-C₈ alkynyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₈ alkyl), -CO₂H, -CO₂(C₁-C₈ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, and
R^{5b} is:
hydrogen, C₂-C₈ alkenyl, C₂-C₈ alkynyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₈ alkyl), -CO₂H, -CO₂(C₁-C₈ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, wherein
R^{5c} and R^{5d} are independently hydrogen, C₁-C₆ alkyl, C₂-C₆ alkenyl, C₂-C₆ alkynyl or -(C₀-C₄ alkyl)C(O)₁₋₂(C₁-C₆ alkyl);
or R^{5a} and R^{5b} together with a carbon atom to which they are attached form a cycloalkyl or heterocycloalkyl ring; and
Q⁵ is O, S, or NR^{5e}, wherein
R^{5e} is hydrogen, C₁-C₈ alkyl, C₂-C₈ alkenyl, C₂-C₈ alkynyl, -(CH₂CH₂O)₁₋₈(C₁-C₄ alkyl), -C(O)R^{5f}, -CO₂R^{5f}, -(C₁-C₄ alkyl)-aryl, -(C₁-C₄ alkyl)-heteroaryl, -(C₁-C₄ alkyl)-cycloalkyl or -(C₁-C₄ alkyl)-heterocycloalkyl, wherein each R^{5f} is hydrogen, C₁-C₈ alkyl, C₂-C₈ alkenyl, C₂-C₈ alkynyl, or -(CH₂CH₂O)₁₋₆(C₁-C₄ alkyl);
wherein
each alkyl, alkenyl and alkynyl is unsubstituted, fluorinated, substituted with one or two hydroxyl or C₁-C₆ alkoxy groups, or substituted with one or two oxo groups;
each cycloalkyl has 3-10 ring carbons and is saturated or partially unsaturated, and optionally includes one or two fused cycloalkyl rings, each fused ring having 3-8 ring members, and is substituted with 0-6 R⁷;
each heterocycloalkyl has 3-10 ring members and 1-3 heteroatoms where each is independently nitrogen, oxygen or sulfur and is saturated or partially unsaturated, and optionally includes one or two fused cycloalkyl rings, each having 3-8 ring members, and is substituted with 0-6 R⁷;
each aryl is a phenyl or a naphthyl, and optionally includes one or two fused cycloalkyl or heterocycloalkyl rings, each fused cycloalkyl or heterocycloalkyl ring having 4-8 ring members, and is substituted with 0-5 R⁸;
each heteroaryl is a 5-6 membered monocyclic heteroaryl ring having 1-4 heteroatoms, where each is independently nitrogen, oxygen or sulfur or a 8-10 membered bicyclic heteroaryl having 1-5 heteroatoms where each is independently nitrogen, oxygen or sulfur, and optionally includes one or two fused cycloalkyl or heterocycloalkyl rings, each fused cycloalkyl or heterocycloalkyl ring having 4-8 ring members, and is substituted with 0-5 R⁸,
in which
each R⁷ is independently oxo, C₁-C₄ alkyl, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} or -NR^{B}S(O)₁₋₂NR^{B}R^{A}; and
each R⁸ is independently optionally-substituted C₁-C₄ alkyl, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} or -NR^{B}S(O)₁₋₂NR^{B}R^{A};
wherein each R^{A} is independently H or C₁-C₃ alkyl, and each R^{B} is independently H, C₁-C₃ alkyl, C₁-C₃ fluoroalkyl, C₁-C₃ hydroxyalkyl, -S(O)₁-₂(C₁-C₃ alkyl), -C(O)(C₁-C₃ alkyl) or -CO₂(C₁-C₃ alkyl), or R^{A} and R^{B} together with the nitrogen atom to which they are attached come together to form an unsubstituted heterocycloalkyl ring comprised of 3-6 ring members.

2. The compound according to claim 1, wherein the moiety 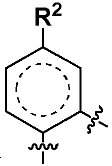 is

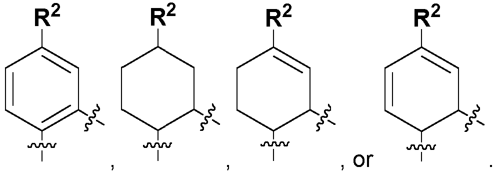

3. The compound according to any one of claims 1-2, wherein R^{1a} and R^{1b} are each independently hydrogen, C₁-C₄ alkyl, C₂-C₄ alkenyl, hydroxy, halo, -O(C₁-C₄ alkyl), -C(O)(C₁-C₄ alkyl), -CO₂(C₁-C₄ alkyl), -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl.

4. The compound according to any one of claims 1-3, wherein R² is hydrogen, C₁-C₄ alkyl, hydroxy, -CO₂H, -CO₂(C₁-C₄ alkyl), or oxo.

5. The compound according to any one of claims 1-4, wherein R³ is hydrogen, C₁-C₁₂ alkyl, C₂-C₁₂ alkenyl, C₂-C₁₂ alkynyl, -(OCH₂CH₂)₀₋₆OCH₃, -(C₀-C₄ alkyl)-aryl, -(C₀-C₄ alkyl)-heteroaryl, -(C₀-C₄ alkyl)-cycloalkyl or -(C₀-C₄ alkyl)-heterocycloalkyl.

6. The compound according to any one of claims 1-5, wherein R⁴ is hydrogen, -CO₂H, or -CO₂(C₁-C₆ alkyl).

7. The compound according to claim 1, wherein the compound is of formula: 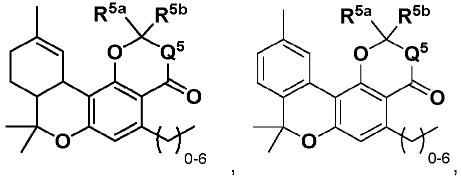 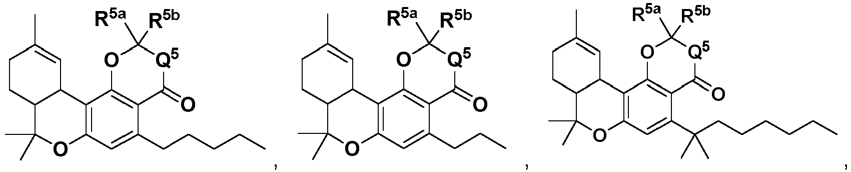 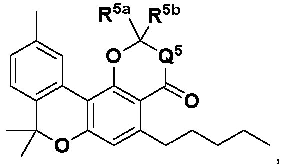 or

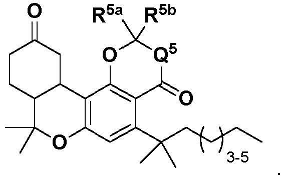

8. The compound according to any one of claims 1-7, wherein R^{5a} is:
hydrogen, C₁-C₆ alkyl, C₂-C₆ alkenyl, C₂-C₈ alkynyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₆ alkyl), -CO₂H, -CO₂(C₁-C₆ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, and
R^{5b} is:
hydrogen, C₂-C₆ alkenyl, C₂-C₆ alkynyl, hydroxy, halo, -(C₀-C₄ alkyl)O(C₁-C₆ alkyl), -(CH₂CH₂O)₁₋₈(C₁-C₄ alkyl), -(C₀-C₄ alkyl)C(O)(C₁-C₆ alkyl), -CO₂H, -CO₂(C₁-C₆ alkyl), -(C₀-C₄ alkyl)-aryl, -(C₀-C₄ alkyl)-heteroaryl, -(C₀-C₄ alkyl)-cycloalkyl, -(C₀-C₄ alkyl)-heterocycloalkyl, or -NR^{5c}R^{5d}, or
R^{5a} and R^{5b} together with a carbon atom form a cycloalkyl or heterocycloalkyl ring.

9. The compound according to any one of claims 1-8, wherein Q⁵ is oxygen or sulfur, or -NR^{5e}, wherein R^{5e} is hydrogen, C₁-C₈ alkyl, -C(O)R^{5f}, or -CO₂R^{5f}, wherein each R^{5f} is hydrogen or C₁-C₆ alkyl.

10. The compound according to any one of claims 1-9, wherein each R⁷ is independently oxo, C₁-C₄ alkyl, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, or -NR^{B}S(O)₁₋₂R^{A}.

11. The compound according to any one of claims 1-10, wherein each R⁸ is independently C₁-C₄ alkyl, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, or -NR^{B}S(O)₁₋₂R^{A}.

12. The compound of claim 1, which is:
8,8,11-trimethyl-5-pentyl-4H,8H-benzo[c][1,3]dioxino[4,5-f]chromen-4-one;
8,8,11-trimethyl-5-pentyl-2-phenyl-4H,8H-benzo[c][1,3]dioxino[4,5-f]chromen-4-one;
2,8,8,11-tetramethyl-5-pentyl-2-(4-(trifluoromethyl)phenyl)-4H,8H-benzo[c][1,3]dioxino[4,5-f]chromen-4-one;
2-acetyl-2,8,8,11-tetramethyl-5-pentyl-4H,8H-benzo[c][1,3]dioxino[4,5-f]chromen-4-one;
ethyl 2,8,8,11-tetramethyl-4-oxo-5-pentyl-4H,8H-benzo[c][1,3]dioxino[4,5-f]chromene-2-carboxylate;
2,8,8,11-tetramethyl-2-(2-oxopropyl)-5-pentyl-4H,8H-benzo[c][1,3]dioxino[4,5-flchromen-4-one;
2,8,8,11-tetramethyl-2-(2-methylprop-1-en-1-yl)-5-pentyl-4H,8H-benzo[c][1,3]dioxino[4,5-f]chromen-4-one;
8,8,11-trimethyl-5-pentyl-4H,8H-spiro[benzo[c][1,3]dioxino[4,5-f]chromene-2,1'-cyclopentan]-4-one;
2-(methoxymethyl)-2,8,8,11-tetramethyl-5-pentyl-4H,8H-benzo[c][1,3]dioxino[4,5-flchromen-4-one;
8,8,11-trimethyl-2-(2-oxopropyl)-5-pentyl-2-phenyl-4H,8H-benzo[c][1,3]dioxino[4,5-f]chromen-4-one;
2-(4-fluorophenyl)-8,8,11-trimethyl-2-(2-oxopropyl)-5-pentyl-4H,8H-benzo[c][1,3]dioxino[4,5-f]chromen-4-one;
8,8,11-trimethyl-2-(2-oxopropyl)-5-pentyl-4H,8H-benzo[c][1,3]dioxino[4,5-f]chromen-4-one;
2-butyl-8,8,11-trimethyl-2-(2-oxopropyl)-5-pentyl-4H,8H-benzo[c][1,3]dioxino[4,5-flchromen-4-one;
2,8,8,11-tetramethyl-5-pentyl-4H,8H-benzo[c][1,3]dioxino[4,5-f]chromen-4-one;
2-ethyl-8,8,11-trimethyl-5-pentyl-4H,8H-benzo[c][1,3]dioxino[4,5-f]chromen-4-one;
8,8,11-trimethyl-5-pentyl-2,2-diphenyl-4H,8H-benzo[c][1,3]dioxino[4,5-f]chromen-4-one;
8,8,11-trimethyl-2-(2-oxopropyl)-5-pentyl-2-(p-tolyl)-4H,8H-benzo[c][1,3]dioxino[4,5-flchromen-4-one;
8,8,11-trimethyl-2-(4-nitrophenyl)-2-(2-oxopropyl)-5-pentyl-4H,8H-benzo[c][1,3]dioxino[4,5-f]chromen-4-one;
2-(4-methoxyphenyl)-8,8,11-trimethyl-2-(2-oxopropyl)-5-pentyl-4H,8H-benzo[c][1,3]dioxino[4,5-f]chromen-4-one;
methyl 4-(8,8,11-trimethyl-4-oxo-2-(2-oxopropyl)-5-pentyl-4H,8H-benzo[c][1,3]dioxino[4,5-f]chromen-2-yl)benzoate;
2-(4-chlorophenyl)-8,8,11-trimethyl-2-(2-oxopropyl)-5-pentyl-4H,8H-benzo[c][1,3]dioxino[4,5-f]chromen-4-one;
8,8,11-trimethyl-5-pentyl-8a,9,10,12a-tetrahydro-4H,8H-benzo[c][1,3]dioxino[4,5-flchromen-4-one;
8,8,11-trimethyl-5-pentyl-2-phenyl-8a,9,10,12a-tetrahydro-4H,8H-benzo[c][1,3]dioxino[4,5-f]chromen-4-one;
2,8,8,11-tetramethyl-5-pentyl-2-(4-(trifluoromethyl)phenyl)-8a,9,10,12a-tetrahydro-4H,8H-benzo[c][1,3]dioxino[4,5-f]chromen-4-one;
2-acetyl-2,8,8,11-tetramethyl-5-pentyl-8a,9,10,12a-tetrahydro-4H,8H-benzo[c][1,3]dioxino[4,5-f]chromen-4-one;
ethyl 2,8,8,11-tetramethyl-4-oxo-5-pentyl-8a,9,10,12a-tetrahydro-4H,8H-benzo[c][1,3]dioxino[4,5-f]chromene-2-carboxylate;
2,8,8,11-tetramethyl-2-(2-oxopropyl)-5-pentyl-8a,9,10,12a-tetrahydro-4H,8H-benzo[c][1,3]dioxino[4,5-f]chromen-4-one;
2,8,8,11-tetramethyl-2-(2-methylprop-1-en-1-yl)-5-pentyl-8a,9,10,12a-tetrahydro-4H,8H-benzo[c][1,3]dioxino[4,5-f]chromen-4-one;
8,8,11-trimethyl-5-pentyl-8a,9,10,12a-tetrahydro-4H,8H-spiro[benzo[c][1,3]dioxino[4,5-f]chromene-2,1'-cyclopentan]-4-one;
2-(methoxymethyl)-2,8,8,11-tetramethyl-5-pentyl-8a,9,10,12a-tetrahydro-4H,8H-benzo[c][1,3]dioxino[4,5-f]chromen-4-one;
8,8, 11-trimethyl-2-(2-oxopropyl)-5-pentyl-2-phenyl-8a, 9,10, 12a-tetrahydro-4H ,8H-benzo[c][1,3]dioxino[4,5-f]chromen-4-one;
2-(4-fluorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one;
8,8, 11-trimethyl-2-(2-oxopropyl)-5-pentyl-8a, 9,10, 12a-tetrahydro-4H ,8H-benzo[c][1,3]dioxino[4,5-f]chromen-4-one;
2-butyl-8,8,11-trimethyl-2-(2-oxopropyl)-5-pentyl-8a,9,10,12a-tetrahydro-4H,8H-benzo[c][1,3]dioxino[4,5-f]chromen-4-one;
2,8,8,11-tetramethyl-5-pentyl-8a,9,10,12a-tetrahydro-4H,8H-benzo[c][1,3]dioxino[4,5-flchromen-4-one;
2-ethyl-8,8,11-trimethyl-5-pentyl-8a,9,10,12a-tetrahydro-4H,8H-benzo[c][1,3]dioxino[4,5-f]chromen-4-one;
8,8, 11-trimethyl-5-pentyl-2,2-diphenyl-8a,9, 10, 12a-tetrahydro-4H,8H-benzo[c][1,3]dioxino[4,5-f]chromen-4-one;
8,8,11-trimethyl-2-(2-oxopropyl)-5-pentyl-2-(p-tolyl)-8a,9,10,12a-tetrahydro-4H,8H-benzo[c][1,3]dioxino[4,5-f]chromen-4-one;
8,8,11-trimethyl-2-(4-nitrophenyl)-2-(2-oxopropyl)-5-pentyl-8a,9,10,12a-tetrahydro-4H,8H-benzo[c][1,3]dioxino[4,5-f]chromen-4-one;
2-(4-methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-one;
4-(8,8,11-trimethyl-4-oxo-2-(2-oxopropyl)-5-pentyl-8a,9,10,12a-tetrahydro-4H,8H-benzo[c][1,3]dioxino[4,5-f]chromen-2-yl)benzoate; or
2-(4-chlorophenyl)-8,8,11-trimethyl-2-(2-oxopropyl)-5-pentyl-8a,9,10,12a-tetrahydro-4H,8H-benzo[c][1,3]dioxino[4,5-f]chromen-4-one.

13. A pharmaceutical composition comprising a compound of any one of claims 1 to 12, or an enantiomer, diastereomer, racemate, or tautomer thereof, or a pharmaceutically acceptable salt, solvate or hydrate of the compound, enantiomer, diastereomer, racemate, or tautomer together with a pharmaceutically acceptable excipient, diluent, or carrier.

14. A compound according to any one of claims 1 to 12 or a composition according to claim 13 for use for treating or preventing a disease associated with cannabinoid receptor, preferably, the cannabinoid receptor is one or more of CB1, CB2, 5HT1A, 5HT2A, GPR18, GPR55, GPR119, TRPV1, TPRV2, PPARy or a µ-opioid receptor, more preferably CB1 or CB2.

15. A compound for use according to claim 14 or a composition for use according to claim 14 wherein the disease is chosen among acute pain, ADHD/ADD, alcohol use disorder, allergic asthma, ALS, Alzheimer's, anorexia (e.g. HIV-related cachexia), anxiety disorders (e.g., social anxiety disorder, specific phobia, test anxiety, generalized anxiety disorder), arthritis, atherosclerosis, autism, bipolar disorder, burns, cancer, cancer pain, Charcot-Marie-Tooth disease, chronic inflammatory demyelinating polyneuropathies, chronic pain, chronic allograft nephropathy, cocaine use disorder, complex regional pain syndrome, congestive heart failure, depression, fibromyalgia, fragile X syndrome/FXTAS, frontotemporal dementias (behavioural variant), gingivitis pyrexia, glaucoma, glioblastoma, glomerulonephropathy, Huntington's disease, hypertrophic scars, IBD/IBS, inflammation, Inflammatory myopathies, ischemia, kidney fibrosis, keloids, leukodystrophies, liver fibrosis, liver cirrhosis, lung fibrosis, migraine, multiple sclerosis, myocardial infarction, nausea (e.g. CINV, motion sickness), neuropathic pain (e.g., postherpetic neuralgia, painful diabetic neuropathy), nightmare disorder, non-alcoholic fatty liver disease, obesity, obsessive-compulsive disorder, opioid sparing, opioid use disorder, osteoarthritis, osteoporosis, Parkinson's, post-concussion syndrome/traumatic brain injury, psychosis/schizophrenia, PTSD, regulation of bone mass, REM sleep behaviour disorder, reperfusion injury, Rett syndrome, rheumatoid arthritis, skin conditions (e.g. acne, psoriatic arthritis), sleep disorders (e.g., insomnia, RLS), spinocerebellar ataxias, systemic fibrosis, systemic sclerosis, thermal injury, tobacco use disorder/nicotine dependence, Tourette's, tumors, or trigeminal neuralgia.

## Patentansprüche

1. Verbindung mit der Strukturformel: 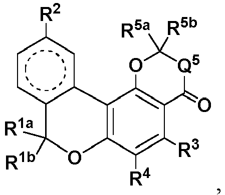
oder Enantiomer, Diastereomer, Racemat oder Tautomer davon, oder pharmazeutisch verträgliches Salz, Solvat oder Hydrat der Verbindung, des Enantiomers, Diastereomers, Racemats oder Tautomers, wobei
die 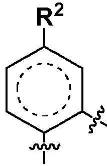 -Einheit 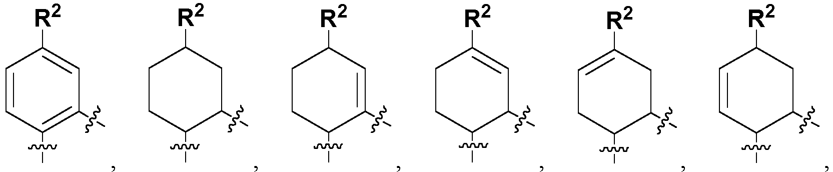 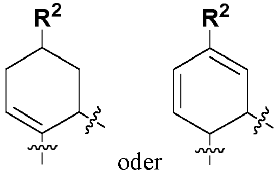 ist,
R^{1a} und R^{1b} unabhängig
Wasserstoff, C₁-C₈-Alkyl, C₂-C₈-Alkenyl, C₂-C₈-Alkinyl, Hydroxy, Halogen, -O(C₁-C₄-Alkyl), -C(O)(C₁-C₄-Alkyl), -CO₂H, -CO₂(C₁-C₄-Alkyl), -(C₀-C₄-Alkyl)aryl, -(C₀-C₄-Alkyl)heteroaryl, -(C₀-C₄-Alkyl)cycloalkyl, -(C₀-C₄-Alkyl)heterocycloalkyl oder -NR^{1c}R^{1d} sind, wobei
R^{1c} und R^{1d} unabhängig Wasserstoff, C₁-C₄-Alkyl oder -C(O)(C₁-C₄-Alkyl) sind, oder R^{1a} und R^{1b} zusammen mit einem Kohlenstoffatom, an das sie gebunden sind, einen Cycloalkyl- oder Heterocycloalkylring bilden;
R² Wasserstoff, C₁-C₈-Alkyl, Hydroxy, -CO₂H, -CO₂(C₁-C₈-Alkyl) oder Oxo ist, wenn es an einen Ring mit angemessener Sättigung gebunden ist;
R³ Wasserstoff, C₁-C₁₂-Alkyl, C₂-C₁₂-Alkenyl, C₂-C₁₂-Alkinyl, -(OCH₂CH₂)₀₋₆O(C₁-C₈-Alkyl), -(C₀-C₄-Alkyl)-NR^{3a}R^{3b}, -(C₀-C₄-Alkyl)aryl, -(C₀-C₄-Alkyl)heteroaryl, -(C₀-C₄-Alkyl)cycloalkyl oder -(C₀-C₄-Alkyl)heterocycloalkyl ist, wobei R^{3a} und R^{3b} jeweils unabhängig Wasserstoff oder C₁-C₆-Alkyl sind;
R⁴ Wasserstoff, -CO₂H oder -CO₂(C₁-C₆-Alkyl) ist; und
R^{5a} ist:
Wasserstoff, C₁-C₈-Alkyl, C₂-C₈-Alkenyl, C₂-C₈-Alkinyl, Hydroxy, Halogen, -(C₀-C₄-Alkyl)O(C₁-C₆-alkyl), -(CH₂CH₂O)₁₋₈(C₁-C₄-Alkyl), -(C₀-C₄-Alkyl)C(O)(C₁-C₈-alkyl), -CO₂H, -CO₂(C₁-C₈-Alkyl), -(C₀-C₄-Alkyl)aryl, -(C₀-C₄-Alkyl)heteroaryl, -(C₀-C₄-Alkyl)cycloalkyl, (C₀-C₄-Alkyl)heterocycloalkyl oder -NR^{5c}R^{5d}, und
R^{5b} ist:
Wasserstoff, C₂-C₈-Alkenyl, C₂-C₈-Alkinyl, Hydroxy, Halogen, -(C₀-C₄-Alkyl)O(C₁-C₆-alkyl), -(CH₂CH₂O)₁₋₈(C₁-C₄-Alkyl), -(C₀-C₄-Alkyl)C(O)(C₁-C₈-alkyl), -CO₂H, -CO₂(C₁-C₈-Alkyl), -(C₀-C₄-Alkyl)aryl, -(C₀-C₄-Alkyl)heteroaryl, -(C₀-C₄-Alkyl)cycloalkyl, -(C₀-C₄-Alkyl)heterocycloalkyl oder -NR^{5c}R^{5d}, wobei
R^{5c} und R^{5d} unabhängig Wasserstoff, C₁-C₆-Alkyl, C₂-C₆-Alkenyl, C₂-C₆-Alkinyl oder -(C₀-C₄-Alkyl)C(O)₁₋₂(C₁-C₆-alkyl) sind;
oder R^{5a} und R^{5b} zusammen mit einem Kohlenstoffatom, an das sie gebunden sind, einen Cycloalkyl- oder Heterocycloalkylring bilden; und
Q⁵ O, S oder NR^{5e} ist, wobei
R^{5e} Wasserstoff, C₁-C₈-Alkyl, C₂-C₈-Alkenyl, C₂-C₈-Alkinyl, -(CH₂CH₂O)₁₋₈(C₁-C₄-Alkyl), -C(O)R^{5f}, -CO₂R^{5f}, -(C₁-C₄-Alkyl)aryl, -(C₁-C₄-Alkyl)heteroaryl, -(C₁-C₄-Alkyl)cycloalkyl oder -(C₁-C₄-Alkyl)heterocycloalkyl ist, wobei jedes R^{5f} Wasserstoff, C₁-C₈-Alkyl, C₂-C₈-Alkenyl, C₂-C₈-Alkinyl oder -(CH₂CH₂O)₁₋₆(C₁-C₄-Alkyl) ist;
wobei
jedes Alkyl, Alkenyl und Alkinyl unsubstituiert, fluoriert, mit einer oder zwei Hydroxyl- oder C₁-C₆-Alkoxygruppen substituiert, oder mit einer oder zwei Oxogruppen substituiert ist;
jedes Cycloalkyl 3-10 Ringkohlenstoffe aufweist und gesättigt oder teilweise ungesättigt ist und optional einen oder zwei kondensierte Cycloalkylringe einschließt, wobei jeder kondensierte Ring 3-8 Ringglieder aufweist und mit 0-6 R⁷ substituiert ist;
jedes Heterocycloalkyl 3-10 Ringglieder und 1-3 Heteroatome aufweist, worin jedes unabhängig Stickstoff, Sauerstoff oder Schwefel ist, und gesättigt oder teilweise ungesättigt ist, und optional einen oder zwei kondensierte Cycloalkylringe einschließt, wobei jedes 3-8 Ringglieder aufweist und mit 0-6 R⁷ substituiert ist;
jedes Aryl ein Phenyl oder ein Naphthyl ist und optional einen oder zwei kondensierte Cycloalkyl- oder Heterocycloalkylringe einschließt, wobei jeder kondensierte Cycloalkyl- oder Heterocycloalkylring 4-8 Ringglieder aufweist, und mit 0-5 R⁸ substituiert ist;
jedes Heteroaryl ein 5- bis 6-gliedriger monocyclischer Heteroarylring mit 1-4 Heteroatomen ist, worin jedes unabhängig Stickstoff, Sauerstoff oder Schwefel ist, oder ein 8- bis 10-gliedriges bicyclisches Heteroaryl mit 1-5 Heteroatomen, worin jedes unabhängig Stickstoff, Sauerstoff oder Schwefel ist, und optional einen oder zwei kondensierte Cycloalkyl- oder Heterocycloalkylringe einschließt, wobei jeder kondensierte Cycloalkyl- oder Heterocycloalkylring 4-8 Ringglieder aufweist und mit 0-5 R⁸ substituiert ist,
bei der
jedes R⁷ unabhängig Oxo, C₁-C₄-Alkyl, -Cl, -F, -Br, -CN, -SF₅, -N₃, Nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} oder -NR^{B}S(O)₁₋₂NR^{B}R^{A} ist; und
jedes R⁸ unabhängig optional substituiertes C₁-C₄-Alkyl, -Cl, -F, -Br, -CN, -SF₅, -N₃, Nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} oder -NR^{B}S(O)₁₋₂NR^{B}R^{A} ist;
wobei jedes R^{A} unabhängig H oder C₁-C₃-Alkyl ist, und jedes R^{B} unabhängig H, C₁-C₃-Alkyl, C₁-C₃-Fluoralkyl, C₁-C₃-Hydroxyalkyl, -S(O)₁-₂(C₁-C₃-Alkyl) ), -C(O)(C₁-C₃-Alkyl) oder -CO₂(C₁-C₃-Alkyl) ist, oder R^{A} und R^{B} zusammen mit dem Stickstoffatom, an das sie gebunden sind, zusammenkommen, um einen unsubstituierten Heterocycloalkylring, der aus 3 bis 6 Ringglieder besteht, zu bilden.

2. Verbindung nach Anspruch 1, wobei die Einheit 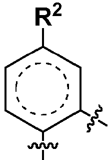 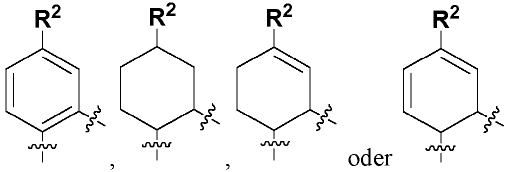 ist.

3. Verbindung nach einem der Ansprüche 1-2, wobei R^{1a} und R^{1b} jeweils unabhängig Wasserstoff, C₁-C₄-Alkyl, C₂-C₄-Alkenyl, Hydroxy, Halogen, -O(C₁-C₄-Alkyl), -C(O)(C₁-C₄-Alkyl), -CO₂(C₁-C₄-Alkyl), -(C₀-C₄-Alkyl)aryl, -(C₀-C₄-Alkyl)heteroaryl, -(C₀-C₄-Alkyl)cycloalkyl oder -(C₀-C₄-Alkyl)heterocycloalkyl sind.

4. Verbindung nach einem der Ansprüche 1-3, wobei R² Wasserstoff, C₁-C₄-Alkyl, Hydroxy, -CO₂H, -CO₂(C₁-C₄-Alkyl) oder Oxo ist.

5. Verbindung nach einem der Ansprüche 1-4, wobei R³ Wasserstoff, C₁-C₁₂-Alkyl, C₂-C₁₂-Alkenyl, C₂-C₁₂-Alkinyl, -(OCH₂CH₂)₀₋₆OCH₃, -(C₀-C₄-Alkyl)aryl, -(C₀-C₄-Alkyl)heteroaryl, -(C₀-C₄-Alkyl)cycloalkyl oder -(C₀-C₄-Alkyl)heterocycloalkyl ist.

6. Verbindung nach einem der Ansprüche 1-5, wobei R⁴ Wasserstoff, -CO₂H oder - CO₂(C₁-C₆-Alkyl) ist.

7. Verbindung nach Anspruch 1, wobei die Verbindung die Formel: 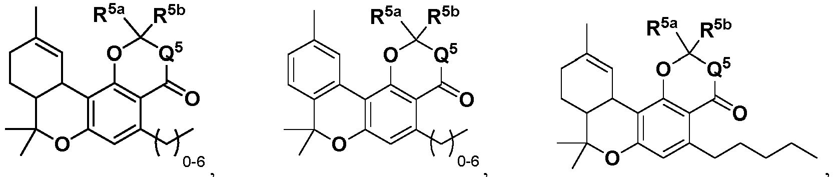 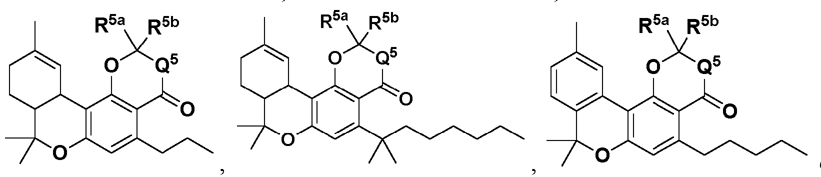 oder 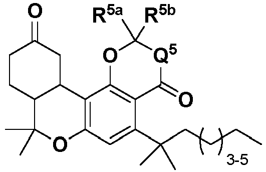 aufweist.

8. Verbindung nach einem der Ansprüche 1-7, wobei R^{5a} ist:
Wasserstoff, C₁-C₆-Alkyl, C₂-C₆-Alkenyl, C₂-C₈-Alkinyl, Hydroxy, Halogen, -(C₀-C₄-Alkyl)O(C₁-C₆-alkyl), -(CH₂CH₂O)₁₋₈(C₁-C₄-Alkyl), -(C₀-C₄-Alkyl)C(O)(C₁-C₆-alkyl), -CO₂H, -CO₂(C₁-C₆-Alkyl), -(C₀-C₄-Alkyl)-aryl, -(C₀-C₄-Alkyl)heteroaryl, -(C₀-C₄-Alkyl)cycloalkyl, -(C₀-C₄-Alkyl)heterocycloalkyl oder -NR^{5c}R^{5d}, und
R^{5b} ist:
Wasserstoff, C₂-C₆-Alkenyl, C₂-C₆-Alkinyl, Hydroxy, Halogen, -(C₀-C₄-Alkyl)O(C₁-C₆-alkyl), -(CH₂CH₂O)₁₋₈(C₁-C₄-Alkyl), -(C₀-C₄-Alkyl)C(O)(C₁-C₆-alkyl), -CO₂H, -CO₂(C₁-C₆-Alkyl), -(C₀-C₄-Alkyl)aryl, -(C₀-C₄-Alkyl)heteroaryl, -(C₀-C₄-Alkyl)cycloalkyl, -(C₀-C₄-Alkyl)heterocycloalkyl oder -NR^{5c}R^{5d}, oder
R^{5a} und R^{5b} zusammen mit einem Kohlenstoffatom einen Cycloalkyl- oder Heterocycloalkylring bilden.

9. Verbindung nach einem der Ansprüche 1-8, wobei Q⁵ Sauerstoff oder Schwefel oder -NR^{5e} ist, wobei R^{5e} Wasserstoff, C₁-C₈-Alkyl, -C(O)R^{5f} oder -CO₂R^{5f} ist, wobei jedes R^{5f} Wasserstoff oder C₁-C₆-Alkyl ist.

10. Verbindung nach einem der Ansprüche 1-9, wobei jedes R⁷ unabhängig Oxo, C₁-C₄-Alkyl, -Cl, -F, -Br, -CN, -SF₅, -N₃, Nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A} oder -NR^{B}S(O)₁₋₂R^{A} ist.

11. Verbindung nach einem der Ansprüche 1-10, wobei jedes R⁸ unabhängig C₁-C₄-Alkyl, -Cl, -F, -Br, -CN, -SF₅, -N₃, Nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A} oder -NR^{B}S(O)₁₋₂R^{A} ist.

12. Verbindung nach Anspruch 1, die
8,8,11-Trimethyl-5-pentyl-4H,8H-benzo[c][1,3]dioxino[4,5-f]chromen-4-on;
8,8,11-Trimethyl-5-pentyl-2-phenyl-4H,8H-benzo[c][1,3]dioxino[4,5-f]chromen-4-on;
2,8,8,11-Tetramethyl-5-pentyl-2-(4-(trifluormethyl)phenyl)-4H,8H-benzo[c][1,3]dioxino[4,5-f]chromen-4-on;
2-Acetyl-2,8,8,11-tetramethyl-5-pentyl-4H,8H-benzo[c][1,3]dioxino[4,5-f]chromen-4-on;
Ethyl-2,8,8,11-tetramethyl-4-oxo-5-pentyl-4H,8H-benzo[c][1,3]dioxino[4,5-f]chromen-2-carboxylat;
2,8,8,11-Tetramethyl-2-(2-oxopropyl)-5-pentyl-4H,8H-benzo[c][1,3]dioxino[4,5-f]chromen-4-on;
2,8,8,11-Tetramethyl-2-(2-methylprop-1-en-1-yl)-5-pentyl-4H,8H-benzo[c][1,3]dioxino[4,5-f]chromen-4-on;
8,8,11-Trimethyl-5-pentyl-4H,8H-spiro[benzo[c][1,3]dioxino[4,5-f]chromen-2,1'-cyclopentan]-4-on;
2-(Methoxymethyl)-2,8,8,11-tetramethyl-5-pentyl-4H,8H-benzo[c][1,3]dioxino[4,5-f]chromen-4-on;
8,8,11-Trimethyl-2-(2-oxopropyl)-5-pentyl-2-phenyl-4H,8H-benzo[c][1,3]dioxino[4,5-f]chromen-4-on;
2-(4-Fluorphenyl)-8,8,11-trimethyl-2-(2-oxopropyl)-5-pentyl-4H,8H-benzo[c][1,3]dioxino[4,5-f]chromen-4-on;
8,8,11-Trimethyl-2-(2-oxopropyl)-5-pentyl-4H,8H-benzo[c][1,3]dioxino[4,5-f]chromen-4-on;
2-Butyl-8,8,11-trimethyl-2-(2-oxopropyl)-5-pentyl-4H,8H-benzo[c][1,3]dioxino[4,5-f]chromen-4-on,
2,8,8,11-Tetramethyl-5-pentyl-4H,8H-benzo[c][1,3]dioxino[4,5-f]chromen-4-on,
2-Ethyl-8,8,11-trimethyl-5-pentyl-4H,8H-benzo[c][1,3]dioxino[4,5-f]chromen-4-on;
8,8,11-Trimethyl-5-pentyl-2,2-diphenyl-4H,8H-benzo[c][1,3]dioxino[4,5-f]chromen-4-on;
8,8,11-Trimethyl-2-(2-oxopropyl)-5-pentyl-2-(p-tolyl)-4H,8H-benzo[c][1,3]dioxino[4,5-f]chromen-4-on,
8,8,11-Trimethyl-2-(4-nitrophenyl)-2-(2-oxopropyl)-5-pentyl-4H,8H-benzo[c][1,3]dioxino[4,5-f]chromen-4-on,
2-(4-Methoxyphenyl)-8,8,11-trimethyl-2-(2-oxopropyl)-5-pentyl-4H,8H-benzo[c][1,3]dioxino[4,5-f]chromen-4-on,
Methyl-4-(8,8,11-trimethyl-4-oxo-2-(2-oxopropyl)-5-pentyl-4H,8H-benzo[c][1,3]dioxino[4,5-f]chromen-2-yl)benzoat;
2-(4-Chlorphenyl)-8,8,11-trimethyl-2-(2-oxopropyl)-5-pentyl-4H,8H-benzo[c][1,3]dioxino[4,5-f]chromen-4-on;
8,8,11-Trimethyl-5-pentyl-8a,9,10,12a-tetrahydro-4H,8H-benzo[c][1,3]dioxino[4,5-f]chromen-4-on,
8,8,11-Trimethyl-5-pentyl-2-phenyl-8a,9,10,12a-tetrahydro-4H,8H-benzo[c][1,3]dioxino[4,5-f]chromen-4-on,
2,8,8,11-Tetramethyl-5-pentyl-2-(4-(trifluormethyl)phenyl)-8a,9,10,12a-tetrahydro-4H,8H-benzo[c][1,3]dioxino[4,5-f]chromen-4-on,
2-Acetyl-2,8,8,11-tetramethyl-5-pentyl-8a,9,10,12a-tetrahydro-4H,8H-benzo[c][1,3]dioxino[4,5-f]chromen-4-on,
Ethyl-2,8,8,11-tetramethyl-4-oxo-5-pentyl-8a,9,10,12a-tetrahydro-4H,8H-benzo[c][1,3]dioxino[4,5-f]chromen-2-carboxylat;
2,8,8,11-Tetramethyl-2-(2-oxopropyl)-5-pentyl-8a,9,10,12a-tetrahydro-4H,8H-benzo[c][1,3]dioxino[4,5-f]chromen-4-on,
2,8,8,11-Tetramethyl-2-(2-methylprop-1-en-1-yl)-5-pentyl-8a,9,10,12a-tetrahydro-4H,8H-benzo[c][1,3]dioxino[4,5-f]chromen-4-on;
8,8,11-Trimethyl-5-pentyl-8a,9,10,12a-tetrahydro-4H,8H-spiro[benzo[c][1,3]dioxino[4,5-f]chromen-2,1'-cyclopentan]-4-on;
2-(Methoxymethyl)-2,8,8,11-tetramethyl-5-pentyl-8a,9,10,12a-tetrahydro-4H,8H-benzo[c][1,3]dioxino[4,5-f]chromen-4-on;
8,8,11-Trimethyl-2-(2-oxopropyl)-5-pentyl-2-phenyl-8a,9,10,12a-tetrahydro-4H,8H-benzo[c][1,3]dioxino[4,5-f]chromen-4-on;
2-(4-Fluorphenyl)-8,8,11-trimethyl-2-(2-oxopropyl)-5-pentyl-8a,9,10,12a-tetrahydro-4H,8H-benzo[c][1,3]dioxino[4,5-f]chromen-4-on;
8,8,11-Trimethyl-2-(2-oxopropyl)-5-pentyl-8a,9,10,12a-tetrahydro-4H,8H-benzo[c][1,3]dioxino[4,5-f]chromen-4-on,
2-Butyl-8,8,11-trimethyl-2-(2-oxopropyl)-5-pentyl-8a,9,10,12a-tetrahydro-4H,8H-benzo[c][1,3]dioxino[4,5-f]chromen-4-on,
2,8,8,11-Tetramethyl-5-pentyl-8a,9,10,12a-tetrahydro-4H,8H-benzo[c][1,3]dioxino[4,5-f]chromen-4-on,
2-Ethyl-8,8,11-trimethyl-5-pentyl-8a,9,10,12a-tetrahydro-4H,8H-benzo[c][1,3]dioxino[4,5-f]chromen-4-on;
8,8,11-Trimethyl-5-pentyl-2,2-diphenyl-8a,9,10,12a-tetrahydro-4H,8H-benzo[c][1,3]dioxino[4,5-f]chromen-4-on;
8,8,11-Trimethyl-2-(2-oxopropyl)-5-pentyl-2-(p-tolyl)-8a,9,10,12a-tetrahydro-4H,8H-benzo[c][1,3]dioxino[4,5-f]chromen-4-on;
8,8,11-Trimethyl-2-(4-nitrophenyl)-2-(2-oxopropyl)-5-pentyl-8a,9,10,12a-tetrahydro-4H,8H-benzo[c][1,3]dioxino[4,5-f]chromen-4-on;
2-(4-Methoxyphenyl)-8,8,11-trimethyl-2-(2-oxopropyl)-5-pentyl-8a,9,10,12a-tetrahydro-4H,8H-benzo[c][1,3]dioxino[4,5-f]chromen-4-on;
4-(8,8,11-Trimethyl-4-oxo-2-(2-oxopropyl)-5-pentyl-8a,9,10,12a-tetrahydro-4H,8H-benzo[c][1,3]dioxino[4,5-f]chromen-2-yl)benzoat; oder
2-(4-Chlorphenyl)-8,8,11-trimethyl-2-(2-oxopropyl)-5-pentyl-8a,9,10,12a-tetrahydro-4H,8H-benzo[c][1,3]dioxino[4,5-f]chromen-4-on ist.

13. Pharmazeutische Zusammensetzung, umfassend eine Verbindung nach einem der Ansprüche 1 bis 12 oder ein Enantiomer, Diastereomer, Racemat oder Tautomer davon oder ein pharmazeutisch verträgliches Salz, Solvat oder Hydrat der Verbindung, des Enantiomers, Diastereomers, Racemats oder Tautomers zusammen mit einem pharmazeutisch verträglichen Exzipienten, Verdünnungsmittel oder Träger.

14. Verbindung nach einem der Ansprüche 1 bis 12 oder Zusammensetzung nach Anspruch 13 zur Verwendung für die Behandlung oder Prävention einer mit einem Cannabinoid-Rezeptor assoziierten Krankheit, wobei der Cannabinoid-Rezeptor vorzugsweise einer oder mehrere von CB1, CB2, 5HT1A, 5HT2A, GPR18, GPR55, GPR119, TRPV1, TPRV2, PPARγ oder ein µ-Opioidrezeptor, bevorzugter CB1 oder CB2, ist.

15. Verbindung zur Verwendung nach Anspruch 14 oder eine Zusammensetzung zur Verwendung nach Anspruch 14, wobei die Krankheit, ausgewählt ist aus akutem Schmerz, ADHS/ADS, Alkoholkonsumstörung, allergischem Asthma, ALS, Alzheimer, Anorexie (z.B. HIV-bedingte Kachexie), Angststörungen (z.B. soziale Angststörung, spezifische Phobie, Prüfungsangst, generalisierte Angststörung), Arthritis, Atherosklerose, Autismus, bipolarer Störung, Verbrennungen, Krebs, Krebsschmerzen, Charcot-Marie-Tooth-Krankheit, chronisch-entzündlichen demyelinisierenden Polyneuropathien, chronischen Schmerzen, chronischer Allograft-Nephropathie, Kokainabhängigkeit, komplexem regionalem Schmerzsyndrom, Herzinsuffizienz, Depression, Fibromyalgie, Fragilem-X-Syndrom/FXTAS, frontotemporalen Demenzen (Verhaltensvariante), Gingivitis, Pyrexie, Glaukom, Glioblastom, Glomerulonephropathie, Huntington-Krankheit, hypertrophen Narben, IBD/IBS, Entzündung, entzündlichen Myopathien, Ischämie, Nierenfibrose, Keloiden, Leukodystrophien, Leberfibrose, Leberzirrhose, Lungenfibrose, Migräne, Multipler Sklerose, Myokardinfarkt, Übelkeit (z.B. CINV, Reisekrankheit), neuropathischer Schmerz (z.B. postherpetischer Neuralgie, schmerzhafter diabetischer Neuropathie), Albtraumstörung, nichtalkoholischer Fettlebererkrankung, Adipositas, Zwangsstörung, Opioideinsparung, Opioidkonsumstörung, Osteoarthritis, Osteoporose, Parkinson, Postkommotionellem Syndrom/traumatischer Hirnverletzung, Psychose/Schizophrenie, PTBS, Regulierung der Knochenmasse, REM-Schlafverhaltensstörung, Reperfusionsschaden, Rett-Syndrom, rheumatoider Arthritis, Hautkrankheiten (z.B. Akne, Psoriasis-Arthritis), Schlafstörungen (z.B. Schlaflosigkeit, RLS), spinozerebellären Ataxien, systemischer Fibrose, systemischer Sklerose, thermischer Verletzung, Tabakkonsumstörung/Nikotinabhängigkeit, Tourette-Syndrom, Tumoren oder Trigeminusneuralgie.

## Revendications

1. Composé présentant la formule structurale : 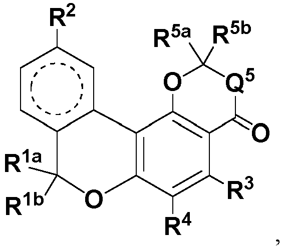
ou énantiomère, diastéréoisomère, racémate ou tautomère de celui-ci, ou sel, solvate ou hydrate pharmaceutiquement acceptables du composé, de l'énantiomère, du diastéréoisomère, du racémate ou du tautomère, dans lequel :
la fraction 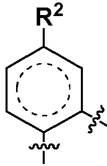
est : 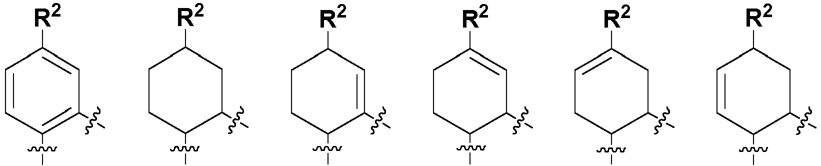 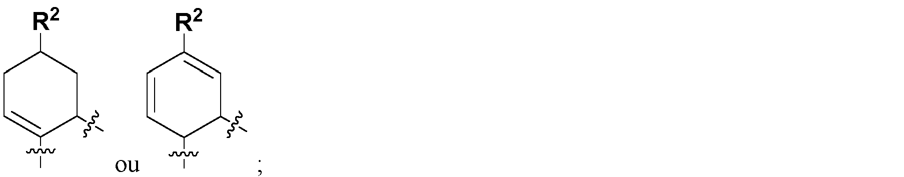
R^{1a} et R^{1b} sont indépendamment :
hydrogène, C₁-C₈ alkyle, C₂-C₈ alcényle, C₂-C₈ alcynyle, hydroxy, halo, -O(C₁-C₄ alkyle), -C(O)(C₁-C₄ alkyle), -CO₂H, -CO₂(C₁-C₄ alkyle), -(C₀-C₄ alkyl)-aryle, -(C₀-C₄ alkyl)-hétéroaryle, -(C₀-C₄ alkyl)-cycloalkyle, -(C₀-C₄ alkyl)-hétérocycloalkyle ou -NR^{1c}R^{1d}, dans lequel
R^{1c} et R^{1d} sont indépendamment hydrogène, C₁-C₄ alkyle ou -C(O)(C₁-C₄ alkyle),
ou R^{1a} et R^{1b} ensemble avec un atome de carbone auquel ils sont attachés forment un cycle cycloalkyle ou hétérocycloalkyle ;
R² est hydrogène, C₁-C₈ alkyle, hydroxy, -CO₂H, -CO₂(C₁-C₈ alkyle) ou oxo lorsqu'il est attaché à un cycle d'une saturation appropriée ;
R³ est hydrogène, C₁-C₁₂ alkyle, C₂-C₁₂ alcényle, C₂-C₁₂ alcynyle, -(OCH₂CH₂)₀₋₆O(C₁-C₈ alkyle), -(C₀-C₄ alkyl)-NR^{3a}R^{3b}, -(C₀-C₄ alkyl)-aryle, -(C₀-C₄ alkyl)-hétéroaryle, -(C₀-C₄ alkyl)-cycloalkyle ou -(C₀-C₄ alkyl)-hétérocycloalkyle, dans lequel R^{3a} et R^{3b} sont chacun indépendamment hydrogène ou C₁-C₆ alkyle ;
R⁴ est hydrogène, -CO₂H ou -CO₂(C₁-C₆ alkyle) ; et
R^{5a} est :
hydrogène, C₁-C₈ alkyle, C₂-C₈ alcényle, C₂-C₈ alcynyle, hydroxy, halo, -(C₀-C₄ alkyl)O(C₁-C₆ alkyle), -(CH₂CH₂O)₁₋₈(C₁-C₄ alkyle), -(C₀-C₄ alkyl)C(O)(C₁-C₈ alkyle), -CO₂H, -CO₂(C₁-C₈ alkyle), -(C₀-C₄ alkyl)-aryle, -(C₀-C₄ alkyl)-hétéroaryle, -(C₀-C₄ alkyl)-cycloalkyle, -(C₀-C₄ alkyl)-hétérocycloalkyle ou -NR^{5c}R^{5d}, et
R^{5b} est :
hydrogène, C₂-C₈ alcényle, C₂-C₈ alcynyle, hydroxy, halo, -(C₀-C₄ alkyl)O(C₁-C₆ alkyl), -(CH₂CH₂O)₁₋₈(C₁-C₄ alkyle), -(C₀-C₄ alkyl)C(O)(C₁-C₈ alkyle), -CO₂H, -CO₂(C₁-C₈ alkyle), -(C₀-C₄ alkyl)-aryle, -(C₀-C₄ alkyl)-hétéroaryle, -(C₀-C₄ alkyl)-cycloalkyle, -(C₀-C₄ alkyl)-hétérocycloalkyle ou -NR^{5c}R^{5d}, dans lequel
R^{5c} et R^{5d} sont indépendamment hydrogène, C₁-C₆ alkyle, C₂-C₆ alcényle, C₂-C₆ alcynyle ou -(C₀-C₄ alkyl)C(O)₁₋₂(C₁-C₆ alkyle) ;
ou R^{5a} et R^{5b} ensemble avec un atome de carbone auquel ils sont attachés forment un cycle cycloalkyle ou hétérocycloalkyle ; et
Q⁵ est : O, S ou NR^{5e}, dans lequel
R^{5e} est hydrogène, C₁-C₈ alkyle, C₂-C₈ alcényle, C₂-C₈ alcynyle, -(CH₂CH₂O)₁₋₈(C₁-C₄ alkyle), -C(O)R^{5f}, -CO₂R^{5f}, -(C₁-C₄ alkyl)-aryle, -(C₁-C₄ alkyl)-hétéroaryle, -(C₁-C₄ alkyl)-cycloalkyle ou -(C₁-C₄ alkyl)-hétérocycloalkyle, dans lequel chaque R^{5f} est hydrogène, C₁-C₈ alkyle, C₂-C₈ alcényle, C₂-C₈ alcynyle ou -(CH₂CH₂O)₁₋₆(C₁-C₄ alkyle) ;
dans lequel
chaque alkyle, alcényle et alcynyle est non substitué, fluoré, substitué par un ou deux groupes hydroxyles ou C₁-C₆ alcoxy ou substitué par un ou deux groupes oxo ;
chaque cycloalkyle comporte 3-10 carbones de cycle et est saturé ou partiellement insaturé et inclut optionnellement un ou deux cycles cycloalkyles condensés, chaque cycle condensé comportant 3-8 chaînons de cycle et est substitué par 0-6 R⁷ ;
chaque hétérocycloalkyle comporte 3-10 chaînons de cycle et 1-3 hétéroatomes où chacun est indépendamment azote, oxygène ou soufre et est saturé ou partiellement insaturé et inclut optionnellement un ou deux cycles cycloalkyles condensés, chacun comportant 3-8 chaînons de cycle et est substitué par 0-6 R⁷ ;
chaque aryle est un phényle ou un naphtyle et inclut optionnellement un ou deux cycles cycloalkyles ou hétérocycloalkyles condensés, chaque cycle cycloalkyle ou hétérocycloalkyle condensé comportant 4-8 chaînons de cycle et est substitué par 0-5 R⁸ ;
chaque hétéroaryle est un cycle hétéroaryle monocyclique à 5-6 chaînons comportant 1-4 hétéroatomes, où chacun est indépendamment azote, oxygène ou soufre, ou un hétéroaryle bicyclique à 8-10 chaînons comportant 1-5 hétéroatomes où chacun est indépendamment azote, oxygène or soufre et inclut optionnellement un ou deux cycles cycloalkyles ou hétérocycloalkyles condensés, chaque cycle cycloalkyle ou hétérocycloalkyle condensé comportant 4-8 chaînons de cycle et est substitué par 0-5 R⁸,
dans lequel
chaque R⁷ est indépendamment oxo, C₁-C₄ alkyle, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A} -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} ou -NR^{B}S(O)₁₋₂NR^{B}R^{A} ; et
chaque R⁸ est indépendamment C₁-C₄ alkyle optionnellement substitué, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A}, -NR^{B}S(O)₁₋₂R^{A}, -OCO₂R^{A}, -OC(O)NR^{B}R^{A}, -NR^{B}CO₂R^{A}, -NR^{B}C(O)NR^{B}R^{A}, -SCO₂R^{A}, -OC(O)SR^{A}, -SC(O)SR^{A}, -SC(O)NR^{B}R^{A}, -NR^{B}C(O)SR^{A}, -OC(S)OR^{A}, -OC(S)NR^{B}R^{A}, -NR^{B}C(S)OR^{A}, -NR^{B}C(S)NR^{B}R^{A}, -SC(S)OR^{A}, -OC(S)SR^{A}, -SC(S)SR^{A}, -SC(S)NR^{B}R^{A}, -NR^{B}C(S)SR^{A}, -NR^{B}C(NR^{B})NR^{B}R^{A} ou -NR^{B}S(O)₁₋₂NR^{B}R^{A} ;
dans lequel chaque R^{A} est indépendamment H ou C₁-C₃ alkyle, et chaque R^{B} est indépendamment H, C₁-C₃ alkyle, C₁-C₃ fluoroalkyle, C₁-C₃ hydroxyalkyle, -S(O)₁₋₂(C₁-C₃ alkyle), -C(O)(C₁-C₃ alkyle) ou -CO₂(C₁-C₃ alkyle), ou R^{A} et R^{B} ensemble avec l'atome d'azote auquel ils sont attachés se rejoignent pour former un cycle hétérocycloalkyle non substitué constitué par 3-6 chaînons de cycle.

2. Composé selon la revendication 1, dans lequel la fraction 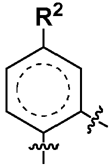 est

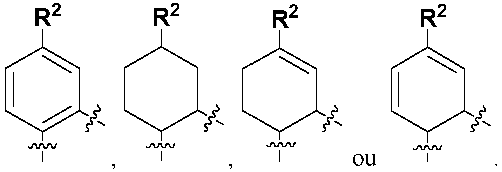

3. Composé selon l'une quelconque des revendications 1-2, dans lequel R^{1a} et R^{1b} sont chacun indépendamment hydrogène, C₁-C₄ alkyle, C₂-C₄ alcényle, hydroxy, halo, -O(C₁-C₄ alkyle), -C(O)(C₁-C₄ alkyle), -CO₂(C₁-C₄ alkyle), -(C₀-C₄ alkyl)-aryle, -(C₀-C₄ alkyl)-hétéroaryle, -(C₀-C₄ alkyl)-cycloalkyle ou -(C₀-C₄ alkyl)-hétérocycloalkyle.

4. Composé selon l'une quelconque des revendications 1-3, dans lequel R² est hydrogène, C₁-C₄ alkyle, hydroxy, -CO₂H, -CO₂(C₁-C₄ alkyle) ou oxo.

5. Composé selon l'une quelconque des revendications 1-4, dans lequel R³ est hydrogène, C₁-C₁₂ alkyle, C₂-C₁₂ alcényle, C₂-C₁₂ alcynyle, -(OCH₂CH₂)₀₋₆OCH₃, -(C₀-C₄ alkyl)-aryle, -(C₀-C₄ alkyl)-hétéroaryle, -(C₀-C₄ alkyl)-cycloalkyle ou -(C₀-C₄ alkyl)-hétérocycloalkyle.

6. Composé selon l'une quelconque des revendications 1-5, dans lequel R⁴ est hydrogène, -CO₂H ou -CO₂(C₁-C₆ alkyle).

7. Composé selon la revendication 1, où le composé est de la formule : 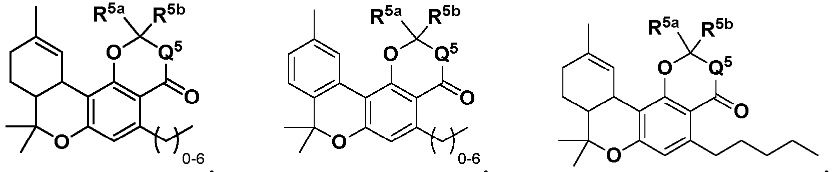 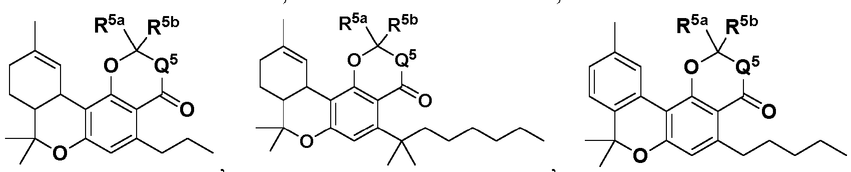 ou

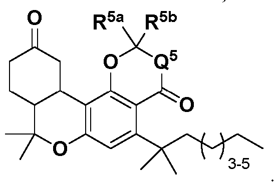

8. Composé selon l'une quelconque des revendications 1-7, dans lequel R^{5a} est :
hydrogène, C₁-C₆ alkyle, C₂-C₆ alcényle, C₂-C₈ alcynyle, hydroxy, halo, -(C₀-C₄ alkyl)O(C₁-C₆ alkyle), -(CH₂CH₂O)₁₋₈(C₁-C₄ alkyle), -(C₀-C₄ alkyl)C(O)(C₁-C₆ alkyle), -CO₂H, -CO₂(C₁-C₆ alkyle), -(C₀-C₄ alkyl)-aryle, -(C₀-C₄ alkyl)-hétéroaryle, -(C₀-C₄ alkyl)-cycloalkyle, -(C₀-C₄ alkyl)-hétérocycloalkyle ou -NR^{5c}R^{5d}, et
R^{5b} est :
hydrogène, C₂-C₆ alcényle, C₂-C₆ alcynyle, hydroxy, halo, -(C₀-C₄ alkyl)O(C₁-C₆ alkyle), -(CH₂CH₂O)₁₋₈(C₁-C₄ alkyle), -(C₀-C₄ alkyl)C(O)(C₁-C₆ alkyle), -CO₂H, -CO₂(C₁-C₆ alkyle), -(C₀-C₄ alkyl)-aryle, -(C₀-C₄ alkyl)-hétéroaryle, -(C₀-C₄ alkyl)-cycloalkyle, -(C₀-C₄ alkyl)-hétérocycloalkyle ou -NR^{5c}R^{5d}, ou
R^{5a} et R^{5b} ensemble avec un atome de carbone forment un cycle cycloalkyle ou hétérocycloalkyle.

9. Composé selon l'une quelconque des revendications 1-8, dans lequel Q⁵ est oxygène ou soufre ou -NR^{5e}, dans lequel R^{5e} est hydrogène, C₁-C₈ alkyle, -C(O)R^{5f} ou -CO₂R^{5f}, dans lequel chaque R^{5f} est hydrogène ou C₁-C₆ alkyle.

10. Composé selon l'une quelconque des revendications 1-9, dans lequel chaque R⁷ est indépendamment oxo, C₁-C₄ alkyle, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A} ou -NR^{B}S(O)₁₋₂R^{A}.

11. Composé selon l'une quelconque des revendications 1-10, dans lequel chaque R⁸ est indépendamment C₁-C₄ alkyle, -Cl, -F, -Br, -CN, -SF₅, -N₃, nitro, -SR^{A}, -S(O)₁₋₂R^{A}, -OR^{A}, -NR^{B}R^{A}, -C(O)R^{A}, -C(O)NR^{B}R^{A}, -NR^{B}C(O)R^{A}, -C(S)NR^{B}R^{A}, -NR^{B}C(S)R^{A}, -CO₂R^{A}, -OC(O)R^{A}, -C(O)SR^{A}, -SC(O)R^{A}, -C(S)OR^{A}, -OC(S)R^{A}, -C(S)SR^{A}, -SC(S)R^{A}, -S(O)₁₋₂OR^{A}, -OS(O)₁₋₂R^{A}, -S(O)₁₋₂NR^{B}R^{A} ou -NR^{B}S(O)₁₋₂R^{A}.

12. Composé selon la revendication 1, qui est :
8,8,11-triméthyl-5-pentyl-4H,8H-benzo[c][1,3]dioxino[4,5-f]chromén-4-one ;
8,8,11-triméthyl-5-pentyl-2-phényl-4H,8H-benzo[c][1,3]dioxino[4,5-f]chromén-4-one ;
2,8,8,11-tétraméthyl-5-pentyl-2-(4-(trifluorométhyl)phényl)-4H,8H-benzo[c][1,3]dioxino[4,5-f]chromén-4-one ;
2-acétyl-2,8,8,11-tétraméthyl-5-pentyl-4H,8H-benzo[c][1,3]dioxino[4,5-f]chromén-4-one ;
2,8,8,11-tétraméthyl-4-oxo-5-pentyl-4H,8H-benzo[c][1,3]dioxino[4,5-f]chromène-2-carboxylate d'éthyle ;
2,8,8,11-tétraméthyl-2-(2-oxopropyl)-5-pentyl-4H,8H-benzo[c][1,3]dioxino[4,5-f]chromén-4-one ;
2,8,8,11-tétraméthyl-2-(2-méthylprop-1-én-1-yl)-5-pentyl-4H,8H-benzo[c][1,3]dioxino[4,5-f]chromén-4-one ;
8,8,11-triméthyl-5-pentyl-4H,8H-spiro[benzo[c][1,3]dioxino[4,5-f]chromène-2,1'-cyclopentan]-4-one ;
2-(méthoxyméthyl)-2,8,8,11-tétraméthyl-5-pentyl-4H,8H-benzo[c][1,3]dioxino[4,5-f]chromén-4-one ;
8,8,11-triméthyl-2-(2-oxopropyl)-5-pentyl-2-phényl-4H,8H-benzo[c][1,3]dioxino[4,5-f]chromén-4-one ;
2-(4-fluorophényl)-8,8,11-triméthyl-2-(2-oxopropyl)-5-pentyl-4H,8H-benzo[c][1,3]dioxino[4,5-f]chromén-4-one ;
8,8,11-triméthyl-2-(2-oxopropyl)-5-pentyl-4H,8H-benzo[c][1,3]dioxino[4,5-f]chromén-4-one ;
2-butyl-8,8,11-triméthyl-2-(2-oxopropyl)-5-pentyl-4H,8H-benzo[c][1,3]dioxino[4,5-f]chromén-4-one ;
2,8,8,11-tétraméthyl-5-pentyl-4H,8H-benzo[c][1,3]dioxino[4,5-f]chromén-4-one ;
2-éthyl-8,8,11-triméthyl-5-pentyl-4H,8H-benzo[c][1,3]dioxino[4,5-f]chromén-4-one ;
8,8,11-triméthyl-5-pentyl-2,2-diphényl-4H,8H-benzo[c][1,3]dioxino[4,5-f]chromén-4-one ;
8,8,11-triméthyl-2-(2-oxopropyl)-5-pentyl-2-(p-tolyl)-4H,8H-benzo[c][1,3]dioxino[4,5-f]chromén-4-one ;
8,8,11-triméthyl-2-(4-nitrophényl)-2-(2-oxopropyl)-5-pentyl-4H,8H-benzo[c][1,3]dioxino[4,5-f]chromén-4-one ;
2-(4-méthoxyphényl)-8,8,11-triméthyl-2-(2-oxopropyl)-5-pentyl-4H,8H-benzo[c][1,3]dioxino[4,5-f]chromén-4-one ;
4-(8,8,11-triméthyl-4-oxo-2-(2-oxopropyl)-5-pentyl-4H,8H-benzo[c][1,3]dioxino[4,5-f]chromén-2-yl)benzoate de méthyle ;
2-(4-chlorophényl)-8,8,11-triméthyl-2-(2-oxopropyl)-5-pentyl-4H,8H-benzo[c][1,3]dioxino[4,5-f]chromén-4-one ;
8,8,11-triméthyl-5-pentyl-8a,9,10,12a-tétrahydro-4H,8H-benzo[c][1,3]dioxino[4,5-f]chromén-4-one ;
8,8,11-triméthyl-5-pentyl-2-phényl-8a,9,10,12a-tétrahydro-4H,8H-benzo[c][1,3]dioxino[4,5-f]chromén-4-one ;
2,8,8,11-tétraméthyl-5-pentyl-2-(4-(trifluorométhyl)phényl)-8a,9,10,12a-tétrahydro-4H,8H-benzo[c][1,3]dioxino[4,5-f]chromén-4-one ;
2-acétyl-2,8,8,11-tétraméthyl-5-pentyl-8a,9,10,12a-tétrahydro-4H,8H-benzo[c][1,3]dioxino[4,5-f]chromén-4-one ;
2,8,8,11-tétraméthyl-4-oxo-5-pentyl-8a,9,10,12a-tétrahydro-4H,8H-benzo[c][1,3]dioxino[4,5-f]chromène-2-carboxylate d'éthyle ;
2,8,8,11-tétraméthyl-2-(2-oxopropyl)-5-pentyl-8a,9,10,12a-tétrahydro-4H,8H-benzo[c][1,3]dioxino[4,5-f]chromén-4-one ;
2,8,8,11-tétraméthyl-2-(2-méthylprop-1-én-1-yl)-5-pentyl-8a,9,10,12a-tétrahydro-4H,8H-benzo[c][1,3]dioxino[4,5-f]chromén-4-one ;
8,8,11-triméthyl-5-pentyl-8a,9,10,12a-tétrahydro-4H,8H-spiro[benzo[c][1,3]dioxino[4,5-f]chromène-2,1'-cyclopentan]-4-one ;
2-(méthoxyméthyl)-2,8,8,11-tétraméthyl-5-pentyl-8a,9,10,12a-tétrahydro-4H,8H-benzo[c][1,3]dioxino[4,5-f]chromén-4-one ;
8,8,11-triméthyl-2-(2-oxopropyl)-5-pentyl-2-phényl-8a,9,10,12a-tétrahydro-4H,8H-benzo[c][1,3]dioxino[4,5-f]chromén-4-one ;
2-(4-fluorophényl)-8,8,11-triméthyl-2-(2-oxopropyl)-5-pentyl-8a,9,10,12a-tétrahydro-4H,8H-benzo[c][1,3]dioxino[4,5-f]chromén-4-one ;
8,8,11-triméthyl-2-(2-oxopropyl)-5-pentyl-8a,9,10,12a-tétrahydro-4H,8H-benzo[c][1,3]dioxino[4,5-f]chromén-4-one ;
2-butyl-8,8,11-triméthyl-2-(2-oxopropyl)-5-pentyl-8a,9,10,12a-tétrahydro-4H,8H-benzo[c][1,3]dioxino[4,5-f]chromén-4-one ;
2,8,8,11-tétraméthyl-5-pentyl-8a,9,10,12a-tétrahydro-4H,8H-benzo[c][1,3]dioxino[4,5-f]chromén-4-one ;
2-éthyl-8,8,11-triméthyl-5-pentyl-8a,9,10,12a-tétrahydro-4H,8H-benzo[c][1,3]dioxino[4,5-f]chromén-4-one ;
8,8,11-triméthyl-5-pentyl-2,2-diphényl-8a,9,10,12a-tétrahydro-4H,8H-benzo[c][1,3]dioxino[4,5-f]chromén-4-one ;
8,8,11-triméthyl-2-(2-oxopropyl)-5-pentyl-2-(p-tolyl)-8a,9,10,12a-tétrahydro-4H,8H-benzo[c][1,3]dioxino[4,5-f]chromén-4-one ;
8,8,11-triméthyl-2-(4-nitrophényl)-2-(2-oxopropyl)-5-pentyl-8a,9,10,12a-tétrahydro-4H,8H-benzo[c][1,3]dioxino[4,5-f]chromén-4-one ;
2-(4-méthoxyphényl)-8,8,11-triméthyl-2-(2-oxopropyl)-5-pentyl-8a,9,10,12a-tétrahydro-4H,8H-benzo[c][1,3]dioxino[4,5-f]chromén-4-one ;
4-(8,8,11-triméthyl-4-oxo-2-(2-oxopropyl)-5-pentyl-8a,9,10,12a-tétrahydro-4H,8H-benzo[c][1,3]dioxino[4,5-flchromén-2-yl)benzoate ; ou
2-(4-chlorophényl)-8,8,11-triméthyl-2-(2-oxopropyl)-5-pentyl-8a,9,10,12a-tétrahydro-4H,8H-benzo[c][1,3]dioxino[4,5-f]chromén-4-one.

13. Composition pharmaceutique comprenant un composé selon l'une quelconque des revendications 1 à 12, ou un énantiomère, diastéréoisomère, racémate ou tautomère de celui-ci, ou un sel, solvate ou hydrate pharmaceutiquement acceptables du composé, de l'énantiomère, du diastéréoisomère, du racémate ou du tautomère accompagné d'un excipient, diluant ou véhicule pharmaceutiquement acceptables.

14. Composé selon l'une quelconque des revendications 1 à 12 ou composition selon la revendication 13 pour utilisation pour le traitement ou la prévention d'une maladie associée à un récepteur cannabinoïde, de préférence, le récepteur cannabinoïde est un ou plusieurs parmi CB1, CB2, 5HT1A, 5HT2A, GPR18, GPR55, GPR119, TRPV1, TPRV2, PPARγ ou un récepteur µ-opioïde, plus préférablement CB1 ou CB2.

15. Composé pour utilisation selon la revendication 14 ou composition pour utilisation selon la revendication 14 où la maladie est choisie parmi : douleur aiguë, ADHD/ADD, trouble lié à la consommation d'alcool, asthme allergique, ALS, maladie d'Alzheimer, anorexie (par ex. cachexie liée au VIH), troubles de l'anxiété (par ex., trouble d'anxiété sociale, phobie spécifique, anxiété test, trouble d'anxiété généralisée), arthrite, athérosclérose, autisme, trouble bipolaire, brûlures, cancer, douleur cancéreuse, maladie de Charcot-Marie- Tooth, polyneuropathies démyélinisantes inflammatoires chroniques, douleur chronique, néphropathie d'allogreffe chronique, trouble lié à la consommation de cocaïne, syndrome de douleur régionale complexe, insuffisance cardiaque congestive, dépression, fibromyalgie, syndrome de l'X fragile/FXTAS, démences fronto-temporales (variante comportementale), gingivite, pyrexie, glaucome, glioblastome, glomérulonéphropathie, maladie de Huntington, cicatrices hypertrophiées, IBD/IBS, inflammation, myopathies inflammatoires, ischémie, fibrose du rein, chéloïdes, leucodystrophies, fibrose du foie, cirrhose du foie, fibrose du poumon, migraine, sclérose en plaques, infarctus du myocarde, nausée (par ex. CINV, mal des transports), douleur neuropathique (par ex., névralgie post-herpétique, neuropathie diabétique douloureuse), cauchemars, maladie du foie gras non alcoolique, obésité, trouble obsessionnel compulsif, substitution d'opioïde, trouble lié à la consommation d'opioïde, ostéoarthrite, ostéoporose, maladie de Parkinson, syndrome post-commotion cérébrale/lésion cérébrale traumatique, psychose/schizophrénie, PTSD, régulation de la masse osseuse, trouble comportemental en sommeil paradoxal, lésion de reperfusion, syndrome de Rett, polyarthrite rhumatoïde, affections de la peau (par ex. acné, polyarthrite psoriasique), troubles du sommeil (par ex., insomnie, RLS), ataxies spinocérébelleuses, fibrose systémique, sclérose systémique, lésion thermique, trouble lié à la consommation de tabac/dépendance à la nicotine, syndrome de la Tourette, tumeurs ou névralgie du trijumeau.
